(19) Europäisches Patentamt
European Patent Office
Office européen des brevets

(11) EP 0 395 312 B1

(12) EUROPEAN PATENT SPECIFICATION

(45) Date of publication and mention of the grant of the patent: **12.05.1999 Bulletin 1999/19**

(21) Application number: **90304250.5**

(22) Date of filing: **20.04.1990**

(51) Int Cl.[6]: **C07D 295/14**, A61K 31/495, C07D 239/42, C07D 295/18, C07D 295/12, C07D 295/08, C07D 205/04, C07D 211/88, C07D 319/20

(54) **Piperazine derivatives**

Piperazin-Derivate

Dérivés de pipérazine

(84) Designated Contracting States:
**AT BE CH DE DK ES FR GR IT LI LU NL SE**

(30) Priority: **22.04.1989 GB 8909209**
**28.10.1989 GB 8924323**

(43) Date of publication of application:
**31.10.1990 Bulletin 1990/44**

(73) Proprietor: **JOHN WYETH & BROTHER LIMITED**
**Maidenhead Berkshire, SL6 0PH (GB)**

(72) Inventor: **Cliffe, Ian Anthony**
**Cippenham, Slough, Berkshire (GB)**

(74) Representative: **Brown, Keith John Symons et al**
**c/o Wyeth Laboratories**
**Huntercombe Lane South**
**Taplow Maidenhead Berkshire SL6 0PH (GB)**

(56) References cited:

| | |
|---|---|
| **EP-A- 15 615** | **EP-A- 48 045** |
| **DE-A- 2 348 973** | **FR-A- 1 361 523** |
| **FR-A- 2 201 084** | **FR-A- 2 567 886** |
| **US-A- 2 928 834** | **US-A- 4 397 855** |
| **US-A- 4 668 687** | |

- **COLLECTION OF CZECHOSLOVAK CHEMICAL COMMUNICATIONS, vol. 50, July 1985, pages 1498-1506; Z. VEJDELEK et al.: "Synthesis of several compounds related to 1-(4-methylsulfonylphenacyl)-4-phenylpiperazine and their pharmacological screening"**
- **JOURNAL OF CHROMATOGRAPHY, vol. 252, 3rd December 1982, pages 310-314; S. CACCIA et al.: "Identification and quantitation of 1-(2-pyrimidinyl)piperazine, an active metabolite of the anxiolytic agent buspirone, in rat plasma and brain"**
- **Ind.Chim.Belg. 28(1963), 123-134**
- **Trends Pharmocol. Sci. 8(11),1987, 432-437**
- **J. Med. Chem. 30(1), 1987, 1-12**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 395 312 B1

Printed by Jouve, 75001 PARIS (FR)

## Description

This invention relates to piperazine derivatives, to processes for their preparation, to their use and to pharmaceutical compositions containing them. The novel compounds act on the central nervous system by binding to 5-HT receptors (as more fully explained below) and hence can be used as medicaments for treating humans and other mammals.

EP-A-395312, the joint applicants of which are American Home Products Corporation and John Wyeth & Brother Limited, describes certain tertiary alkyl functionalized piperazine derivatives. EP-A-395312 and the present application are co-pending applications, both being published on 31 October 1990.

H. Morren et al, Ind. Chim. Belg., 1963, 28, 123-134 describes the synthesis of 1-(ω-phenylalkyl)-4-phenylpiperazine derivatives, some of which are stated to be tranquilizers and others of which are stated to be psycholeptics.

US-A-4668687 relates to 1-(2-pyrimidinyl)piperazine derivatives, particularly derivatives of 1-pyrrolin-2-ones. The compounds are stated to have cognition enhancing activity and/or mild CNS stimulatory activity.

Z. Vejdelek et al, Coll. Czech. Chem. Comm., 1985, 50, 1498-1506 describes the synthesis of certain compounds related to 1-(4-methylsulphonylphenacyl)-4-phenylpiperazine, including the acetate, propionate, benzoate and 4-nitrobenzoate esters.

S. Caccia et al, J. Chrom., 1982, 252, 310-314 describes the identification of 1-(2-pyrimidyl)piperazine as an active metabolite of the anxiolytic agent, buspirone.

EP-A-015615 and EP-A-048045 describe phenylpiperazine derivatives stated to have strong and selective antiagressive activity.

FR-A-2201084 and DE-A-2348973 disclose derivatives of 1-(m-trifluoromethylphenyl)-4-propylpiperazine which are stated to have analgesic and/or anti-inflammatory and/or anorexigenic properties.

FR-A-2567886 discloses 1-(2-pyrimidinyl)piperazinyl derivatives of 1-pyrrolidin-2-ones.

US-A-2928834 describes 2-(4-methyl-1-piperazinyl)-1-phenylethylbenzoates stated to be hypotensive agents.

US-A-4397855 describes 4-(substituted)-α,α-dimethyl-1-piperazine pentanoic acids and derivatives as antiarteriosclerotic agents.

J. Traber et al, Trends Pharmacol. Sci. 1987, 8, 432-437 reviews known 5-$HT_{1A}$ receptor related anxiolytics.

R. A. Glennon, J. Med. Chem., 1987, 30, 1-12 reviews central serotonin receptor sites and the agents known to interact with these sites.

The novel compounds of the invention are those of the general formula

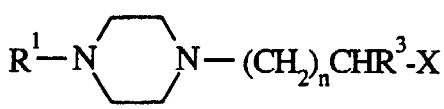

(I)

and the pharmaceutically acceptable acid addition salts thereof.

In formula (I)

n is one of the integers 1 or 2,

$R^1$ is Ar or Het (where "Ar" and "Het" are defined below),

$R^3$ is Ar or an Ar($C_{1-6}$)alkyl radical,

X is $-OCOR^{10}$, $-CO_2R^6$, $-CONR^5R^9$ $-OCO_2R^6$ $-NR^4COR^6$, $OCONHR^{11}$, $-NHCO_2R^6$, $-NR^4CONHR^6$, $-CONHNHR^6$, $-CONHOR^6$,

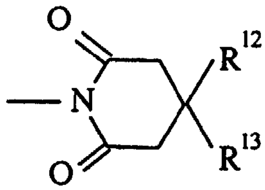 or 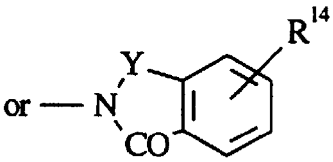

$R^4$ and $R^5$ are each hydrogen or $C_{1-6}$ alkyl

$R^6$ is $-CHR^7R^8$, cycloalkyl of 3 to 12 carbon atoms or Ar($C_{1-6}$)alkyl (where $R^7$ and $R^8$ are each hydrogen or $C_{1-6}$ alkyl),

$R^9$ is hydrogen, an alkyl group of 1 to 8 carbon atoms, other than a tertiary alkyl group, cycloalkyl of 3 to 12 carbon atoms, cycloalkyl($C_{1-6}$)alkyl, Ar($C_{1-6}$)alkyl or 8-azaspiro[4.5]deca-7,9-dione-8-yl-($C_{1-6}$)alkyl [with the proviso that when $R^9$ is hydrogen, alkyl or Ar($C_{1-6}$)alkyl $R^5$ is other than a tertiary alkyl group],

or $R^5$ and $R^9$ together with the nitrogen atom to which they are attached represent an azetidino, pyrrolidino, piperidino, hexahydroazepino, morpholino or piperazino ring which may be optionally substituted by $C_{1-6}$ alkyl, Ar or Ar($C_{1-6}$)alkyl,

$R^{10}$ is cycloalkyl of 3 to 12 carbon atoms, or 2,3-dihydro[1,4]benzodioxinyl optionally substituted by $C_{1-6}$ alkyl, $C_{1-6}$ alkoxy or halogen,

$R^{11}$ is cycloalkyl of 3 to 12 carbon atoms, Ar or Ar($C_{1-6}$)alkyl,

$R^{12}$ and $R^{13}$ are each $C_{1-6}$ alkyl or together with the carbon atom to which they are both attached represent $C_{4-6}$ cycloalkyl,

$R^{14}$ represents hydrogen, halogen, $C_{1-6}$ alkyl or $C_{1-6}$ alkoxy
and

Y is CO or $SO_2$

with the further provisos that

(a) when $R^1$ is phenyl optionally substituted by $CH_3$, $OCH_3$, Cl or $CF_3$, $R^3$ is phenyl optionally substituted by $C_{1-6}$ alkyl, methoxy or halogen and X is $-CONH_2$, $-CON(CH_3)_2$ or $-COOC_2H_5$ then n is 1

and (b) when $R^1$ is 2-pyrimidyl, $R^3$ is phenyl optionally substituted at one or two ring positions with $C_{1-6}$ alkyl, halogen, trifluoromethyl, cyano, nitro, ($C_{1-6}$)alkylamino, di($C_{1-6}$)alkylamino, $C_{1-6}$-alkyloxy and X is

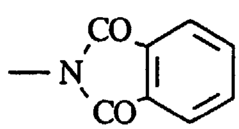

or $-NR^4COR^6$ where $R^4$ is hydrogen or $C_{1-6}$ alkyl and $R^6$ is $-CHR^7R^8$ (where $R^7$ and $R^8$ are each hydrogen or $C_{1-6}$ alkyl and $-CHR^7R^8$ contains a total of 1 to 6 carbon atoms) then n is 2;

and wherein the term "Ar" means an aromatic radical having 6 to 12 carbon atoms which optionally may be substituted by one or more $C_{1-6}$ alkoxy, halogen, trifluoromethyl, nitro, carbalkoxy, carboxamido, cyano, amino, ($C_{1-6}$)alkylamino or di($C_{1-6}$)alkylamino substituents and the term "Het" means an aromatic radical containing one or more nitrogen atoms as heteroatoms and which may be optionally substituted by one or more $C_{1-6}$ alkyl, $C_{1-6}$ alkoxy, halogen, trifluoromethyl, amino, ($C_{1-6}$)alkylamino or di($C_{1-6}$)alkylamino substituents.

Radicals referred to as "$C_{1-6}$" radicals preferably contain 1 to 4 carbon atoms. Examples of "$C_{1-6}$ alkyl" are

methyl, ethyl, propyl, isopropyl, butyl, isobutyl, tert.-butyl, pentyl and isopentyl.

Preferably $R^3$ is an Ar radical.

Examples of Ar($C_{1-6}$)alkyl and Ar($C_{1-6}$)alkoxy include, for example, benzyl and benzyloxy in which the phenyl group may be substituted as defined above.

Examples of "Het" radicals are pyridinyl, pyrimidinyl or pyrazinyl which may optionally be substituted by one or more $C_{1-6}$ alkyl, $C_{1-6}$ alkoxy, halogen, trifluoromethyl, amino, ($C_{1-6}$)alkylamino or di($C_{1-6}$)alkylamino substituents. Preferably the Het radical is monocyclic.

Preferred compounds are:-

those in which n is 1;

those in which $R^1$ is Ar particularly an optionally substituted phenyl such as o-methoxphenyl;

those in which $R^3$ is Ar particularly optionally substituted phenyl;

those in which X is an ester grouping of formula $-CO_2R^6$ or an amide grouping of formula $-CONR^5R^9$ particularly where $-NR^5R^9$ represents a cyclic grouping e.g. piperidino or hexahydroazepino.

The compounds of the invention may be prepared by a number of methods known in the art from known starting materials or starting materials that may be prepared by conventional methods. In one method for preparing an amide of formula (I), where X represents $-CONR^5R^9$ an amine of formula

$$NHR^5R^9 \qquad (II)$$

where $R^5$ and $R^9$ are as defined above is acylated with an acid of formula

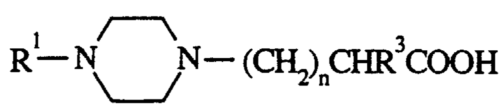

(III)

(where $R^1$ and $R^3$ are as defined above) or with an acylating derivative thereof. Examples of acylating derivatives include the acid halides (eg acid chlorides), azides, anhydrides, imidazolides (eg obtained from carbonyldiimidazole), activated esters or O-acyl ureas obtained from a carbodiimide such as a dialkylcarbodiimide particularly dicyclohexylcarbodiimide. Preferably the amine is acylated with the acid in presence of a coupling agent such as 1,1'-carbonyldiimidazole, iso-butylchloroformate or diphenylphosphinyl chloride.

The acids of formula III are novel compounds and are also provided by this invention.

The reverse amides, i.e. the compounds of the invention in which X is $-NR^4COR^6$ may be prepared in an analogous manner to the amides mentioned above by acylating the piperazine alkylamine

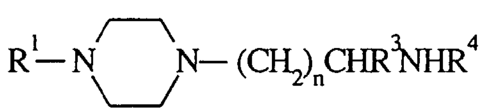

with an acid of formula

$$R^6COOH$$

or with an acylating derivative thereof.

Similarly, the compounds of the invention in which X is

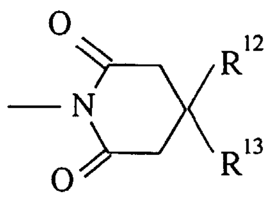 or 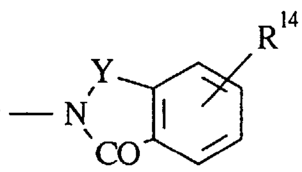

may be prepared by reacting an amine of formula

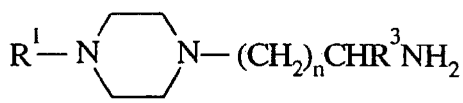

with an anhydride of formula

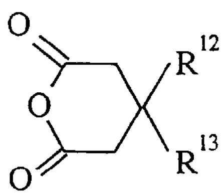

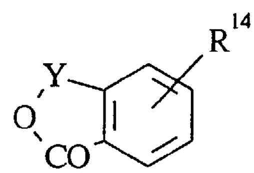

or with an acid of formula

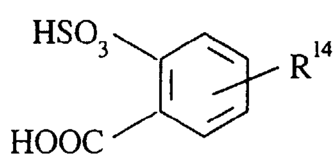

An ester of the invention in which -X is $-CO_2R^6$ may be prepared by esterification of the acid of formula (III) above with an alcohol of formula $R^6OH$.

The reverse esters, i.e. the compounds in which X is $-OCOR^{10}$, may be prepared by esterifying a piperazine alcohol of formula

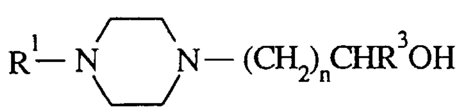

with an acid of formula $R^{10}COOH$.

Both types of esterification may be carried out by methods known in the art. For example an acid halide may be reacted with the appropriate alcohol.

The ureas, (compounds in which X is $-NR^4CONHR^6$) and the carbamates (compounds in which X is -O.CO. $NHR^{11}$) may be prepared by reacting the piperazinyl-alkanol or -alkylamine of formula

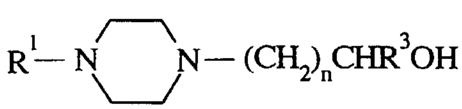

or

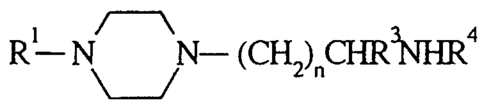

with the appropriate isocyanate. The reverse carbamates (the compounds in which X is $-NHCO_2R^6$) may be prepared in a similar manner from the piperazine isocyanate derivative

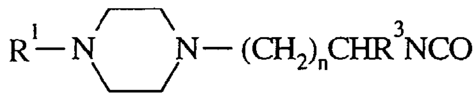

and the alcohol $R^6OH$, or by reacting an amine of formula

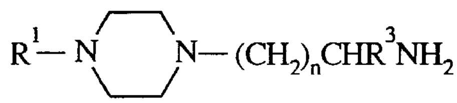

with a compound of formula $R^6OCOHal$ (where Hal is halogen, e.g. chlorine).

The hydroxylamine compounds of the invention (compounds in which X is $CONHOR^6$) may be prepared by reacting the acid of formula (III) above with a hydroxylamine of formula $NH_2OR^6$.

The hydrazide compounds of the invention (compounds in which X is $-CONHNHR^6$) may be prepared by reacting the acid of formula (III) with a hydrazide of formula $NH_2NHR^6$.

An alternative method of preparing the compounds of the invention comprises alkylation of a piperazine of formula

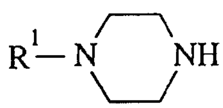

(IV)

(where $R^1$ is as defined above) with an alkylating agent providing the group

$$-(CH_2)_nCHR^3X \qquad (V)$$

(where n, $R^3$ and X are as defined above).

The alkylating agent may be, for example, a compound of formula

$$Z\text{-}CH_2CHR^3X \qquad (VI)$$

where $R^3$ and X are as defined above and Z is a leaving group such as halogen or an alkyl- or aryl-sulphonyloxy group. Alternatively the alkylating agent may be an unsaturated compound of formula

$$CH_2{=}CR^3X \qquad (VII)$$

(where $R^3$ and X are as defined above) and the compound of formula (VII) is reacted with the piperazine of formula (IV) by means of a Michael reaction. The reaction may be carried out at elevated temperature in the presence of an alcohol. A small quantity of an acid catalyst may be employed in the reaction when X represents $-CONR^5R^9$.

The starting materials for the processes described above may be prepared by methods known in the art. For

example certain acids of formula (III) may be prepared by Michael reaction of an acid of formula

$$CH_2{=}CR^3COOH \quad \text{(VIII)}$$

and a piperazine of formula

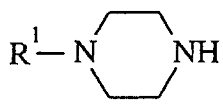

(IV)

in a method similar to the Michael reaction described above. The unsaturated compound of formula (VII) may be prepared from the acid of formula (VIII) by the known methods of obtaining amides and esters from acids. In a preferred method the acid is reacted with an amine in the presence of a condensing agent such as isobutylchloroformate or the acid is esterified, eg by reaction with an alcohol in presence of 2-chloro-1-methylpyridinium iodide. The acids of formula (VIII) are known or may be prepared by methods known in the art.

The amides of formula (I) in which X is $-CONR^5R^9$ where $R^5$ is hydrogen and $R^9$ is a secondary $(C_{1-6})$alkyl group may be prepared by an alternative method comprising reacting a nitrile of formula

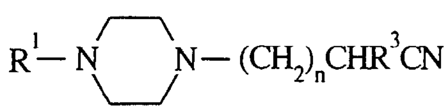

(IX)

with a secondary alcohol under acidic conditions as in the Ritter reaction. The nitrile (IX) may also be subjected to acid hydrolysis to give an amide of formula (I) in which X is $CONH_2$. Furthermore the nitrile may be hydrolysed to the acid (III) which may then be converted to compounds of formula (I) by the methods given above. The nitrile of formula (IX) may be prepared by known methods such as reacting an unsaturated nitrile of formula $CH_2{=}CR_3CN$ with a piperazine of formula (IV) under Michael conditions or reacting a ketone of formula

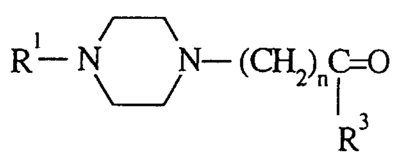

(X)

with p-toluenesulphonylisocyanide.

A further method of preparing the amides of formula (I) in which X is $-CONHR^9$ comprises the desulphurisation of a sulphur containing compound of formula

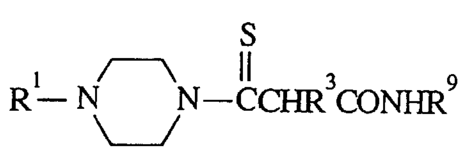

(XI)

where $R^1$ and $R^9$ are as defined above and $R^3$ is aryl. The desulphurisation may be carried out in presence of a nickel

catalyst. The compound of formula (XI) may be prepared by a Willgerodt reaction, eg an aryl alkyl ketone of formula $CH_3CO.R^3$ is reacted with sulphur and a piperazine of formula (IV) and the resulting thioamide is treated with a base and with a isocyanate of formula $R^9NCO$.

The processes described above may be carried out to give a compound of the invention in the form of a free base or as an acid addition salt. If the compound of the invention is obtained as an acid addition salt, the free base can be obtained by basifying a solution of the acid addition salt. Conversely, if the product of the process is a free base an acid addition salt, particularly a pharmaceutically acceptable acid addition salt, may be obtained by dissolving the free base in a suitable organic solvent and treating the solution with an acid, in accordance with conventional procedures for preparing acid addition salts from base compounds.

Examples of acid addition salts are those formed from inorganic and organic acids, such as sulphuric, hydrochloric, hydrobromic, phosphoric, tartaric, fumaric, maleic, citric, acetic, formic, methanesulphonic, p-toluenesulphonic, oxalic and succinic acids.

The compounds of the invention may contain one or more asymmetric carbon atoms, so that the compounds can exist in different steroisomeric forms. The compounds can be for example, racemates or optically active forms. The optically active forms can be obtained by resolution of the racemates or by asymmetric synthesis.

The compounds of the present invention possess pharmacological activity. In particular, they act on the central nervous system by binding to 5-HT receptors. In pharmacological testing it has been shown that the compounds particularly bind to receptors of the $5\text{-}HT_{1A}$ type. In general, the compounds selectively bind to receptors of the $5\text{-}HT_{1A}$ type to a much greater extent than they bind to other receptors such as $\alpha_1$ and $D_2$ receptors. Many exhibit activity as $5\text{-}HT_{1A}$ antagonists in pharmacological testing. The pharmacological testing of the compounds indicates that they can be used for the treatment of CNS disorders, such as anxiety in mammals, particularly humans. They may also be useful as antidepressants, hypotensives and as agents for regulating the sleep/wake cycle, feeding behaviour and/or sexual function.

The compounds of the invention were tested for $5\text{-}HT_{1A}$ receptor binding activity in rat hippocampal membrane homogenate by the method of B S Alexander and M D Wood, J Pharm Pharmarol, 1988, 40, 888-891. The results for representative compounds of the invention are given below.

| Compounds of Example | $IC_{50}$(nM) |
|---|---|
| 6 | 127 |
| 8 | 45 |
| 19 | 24 |
| 21 | 49 |
| 22 | 45 |
| 23 | 59 |
| 24 | 75 |
| 26 | 46 |
| 29 | 25 |
| 31 | 28 |
| 32 | 21 |
| 33 | 8 |
| 34 | 9 |
| 35 | 16.5 |
| 37 | 45 |
| 39 | 22 |
| 40 | 78 |
| 41 | 88 |
| 42 | 37 |

The affinity for $D_2$ receptor sites (as measured by the procedure of A A Hancock et al, Mol Pharmacol, 1984, 26, 439) and for $\alpha_1$ sites (as measured by the procedure of A L Morrow et al, Mol Pharmacol, 1986, 29, 321) for various compounds is given below:

| Compound of Example | Affinity for $D_2$ site $IC_{50}$ (nM) | Affinity for $\alpha_1$ site $IC_{50}$ (nM) |
|---|---|---|
| 19 | 6290 | 976 |

(continued)

| Compound of Example | Affinity for $D_2$ site $IC_{50}$ (nM) | Affinity for $\alpha_1$ site $IC_{50}$ (nM) |
|---|---|---|
| 21 | | 1200 |
| 22 | | 1230 |
| 24 | | $> 10^4$ |
| 26 | | 1090 |
| 29 | | 1140 |
| 32 | >10000 | 851 |
| 37 | | 7310 |
| 41 | | 2850 |
| 42 | | 988 |

The compounds are tested for $5\text{-HT}_{1A}$ receptor antagonism activity in a test involving the antagonism of 5-carboxamidotryptamine in the guine-pig ileum in in vitro (based upon the procedure of Fozard et al, Br J Pharmac 1985, 86, 601P). The results for compounds of the invention are given below.

| Compound of Example | $pA_2$ |
|---|---|
| 19 | 6.9 |
| 21 | 6.9 |
| 23 | 7.0 |
| 26 | 6.8 |
| 31 | 7.4 |
| 32 | 6.8 |
| 37 | 7.6 |
| 42 | 6.9 |

The invention also provides a pharmaceutical composition comprising a compound or a pharmaceutically acceptable acid addition salt thereof in association with a pharmaceutically acceptable carrier. Any suitable carrier known in the art can be used to prepare the pharmaceutical composition. In such a composition, the carrier is generally a solid or liquid or a mixture of a solid or liquid.

Solid form compositions include powders, granules, tablets, capsules (eg hard and soft gelatine capsules), suppositories and pessaries. A solid carrier can be, for example, one or more substances which may also act as flavouring agents, lubricants, solubilisers, suspending agents, fillers, glidants, compression aides, binders or tablet-disintegrating agents; it can also be an encapsulating material. In powders the carrier is a finely divided solid which is in admixture with the finely divided active ingredient. In tablets the active ingredient is mixed with a carrier having the necessary compression properties in suitable proportions and compacted in the shape and size desired. The powders and tablets preferably contain up to 99%, e.g. from 0.03 to 99%, preferably 1 to 80% of the active ingredient. Suitable solid carriers include, for example, calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

The term "composition" is intended to include the formulation of an active ingredient with encapsulating material as carrier to give a capsule in which the active ingredient (with or without other carriers) is surrounded by the carrier, which is thus in association with it. Similarly cachets are included.

Liquid form compositions include, for example, solutions, suspensions, emulsions, syrups, elixirs and pressurised compositions. The active ingredient, for example, can be dissolved or suspended in a pharmaceutically acceptable liquid carrier such as water, an organic solvent, a mixture of both or pharmaceutically acceptable oils or fats. The liquid carrier can contain other suitable pharmaceutical additives such as solubilisers, emulsifiers, buffers, preservatives, sweeteners, flavouring agents, suspending agents, thickening agents, colours, viscosity regulators, stabilisers or osmo-regulators. Suitable examples of liquid carriers for oral and parenteral administration include water (particularly containing additives as above, e.g. cellulose derivatives, preferably sodium carboxymethyl cellulose solution), alcohols, e.g. glycerol and glycols) and their derivatives, and oils (e.g. fractionated coconut oil and arachis oil). For parenteral administration the carrier can also be an oily ester such as ethyl oleate and isopropyl myristate. Sterile liquid carriers are used in sterile liquid form compositions for parenteral administration.

Liquid pharmaceutical compositions which are sterile solutions or suspensions can be utilized by, for example,

intramuscular, intraperitoneal or subcutaneous injection. Sterile solutions can also be administered intravenously. When the compound is orally active it can be administered orally either in liquid or solid composition form.

Preferably the pharmaceutical composition is in unit dosage form, eg as tablets or capsules. In such form, the composition is sub-divided in unit dose containing appropriate quantities of the active ingredient; the unit dosage forms can be packaged composition, for example packeted powders, vials, ampoules, prefilled syringes or sachets containing liquid. The unit dosage form can be, for example, a capsule or tablet itself, or it can be the appropriate number of any such compositions in package form. The quantity of the active ingredient in unit dose of composition may be varied or adjusted from 0.5 mg or less to 750 mg or more, according to the particular need and the activity of the active ingredient.

The following Examples illustrate the invention:

EXAMPLE 1

α-{1-[4-(2-Methoxyphenyl)piperazinyl]methyl}benzeneacetic acid

1-(2-Methoxyphenyl)piperazine (22.6 g, 0.118 mol) and atropic acid (174 g, 0.118 mol) in ethanol (300 ml) were heated under reflux for 18 hours, cooled to room temperature, and evaporated in vacuo. The solid was triturated with acetone (3 x 100 ml) to give a first crop of product (13.8 g) as white crystals. The filtrate was evaporated in vacuo to give an oil which slowly crystallised over 1 month. The solid was triturated with acetone (200 ml) to give a second crop of the hemihydrate of the product (9.01 g) as white crystals, m.p. 160-163°.
(Found: C, 68.4; H, 7.2; N, 7.9.
$C_{20}H_{24}N_2O_3.0.5H_2O$ requires C, 68.8; H, 7.2; N, 8.0%.)

EXAMPLE 2

2-{1-[4-(2-Methoxyphenyl)piperazinyl]methyl}-3-benzenepropanoic acid

2-(Phenylmethyl)propenoic acid (Mannich and Ganz, Chem. Ber., 1922, 55, 3486) (2.00 g, 12.35 mmol) and 1-(2-methoxyphenyl)piperazine (2.37 g, 12.35 mmol) in propanol (25 ml) were heated under reflux for 18 hours, cooled to room temperature, and evaporated in vacuo. The residue was triturated with acetone and ether to give the product (0.80 g) as a colourless powder, m.p. 155-158°.
(Found: C, 71.6; H, 7.4; N, 7.6.
$C_{21}H_{26}N_2O_3$ requires C, 71.2; H, 7.3; N, 7.9%.)

EXAMPLE 3

2-Phenyl-N-(phenylmethyl)propenamide

A stirred solution of atropic acid (10.3 g, 69.5 mmol) in dry tetrahydrofuran (100 ml) was treated under nitrogen with N-methylmorpholine (7.7 ml, 70.0 mmol), cooled to -10°, treated dropwise with iso-butylchloroformate (9 ml, 69.4 mmol), treated dropwise with benzylamine (7.6 ml, 69.6 mmol), warmed to room temperature over 1 hour, filtered and evaporated in vacuo to give a yellow oil which was dissolved in ether (100 ml). The solution was washed with 0.1N-HCl (200 ml), brine (100 ml), 0.1N-NaOH (100 ml) and brine (100 ml), dried ($MgSO_4$), and evaporated in vacuo to give a yellow liquid. Purification by chromatography (silica; di-iso-propyl ether) gave the product as white crystals (7.3g), m.p. 84-86° (from di-iso-propyl ether).
(Found: C, 80.8; H, 6.3; N, 5.7.
$C_{16}H_{15}NO$ requires C, 81.0; H, 6.4; N, 5.9%.)

EXAMPLE 4

N-Cyclohexyl-2-phenylpropenamide

This compound was made from atropic acid (10.48 g, 70.8 mmol), N-methylmorpholine (7.8 ml, 70.9 mmol), iso-butyl chloroformate (9.2 ml, 70.9 mmol), and cyclohexylamine (8.1 ml, 70.7 mmol) using the procedure described in Example 3. The crude product was purified by recrystallisation from cyclohexane to give the product (4.69 g), as white crystals, m.p. 131-133°.
(Found: C, 78.7; H, 8.8; N, 5.95. $C_{15}H_{19}NO$ requires C, 78.6; H, 8.35; N, 6.1%.)

EXAMPLE 5

Propyl 3-{1-[4-(2-methoxyphenyl)piperazinyl]}-2-phenylpropanoate

A stirred solution of atropic acid (2.11 g, 14.3 mmol) and cyclohexanol (1.51 ml, 14.2 mmol) in ethyl acetate (40 ml) at 2-5° was treated dropwise with N,N'-dicylohexylcarbodiimide (3.27 g, 15.8 mmol), warmed to room temperature, filtered and evaporated in vacuo to give a yellow oil.

A solution of the oil in propanol (20 ml) was heated under reflux for 1 day, cooled to room temperature, evaporated in vacuo, and the residue purified by chromatography [silica; ether-hexane (1:3) and silica; di-iso-propyl ether] to give the free base as an oil (1.1 g).

Formation of the salt in the usual manner gave the hydrochloride (0.95 g), m.p. 200-204°.
(Found: C, 60.9; H, 7.25; N ,6.3. $C_{23}H_{30}N_2O_3$. 2HCl requires C, 60.7; H, 7.1; N, 6.15%.)

EXAMPLE 6

3-{1-[4-(2-Methoxyphenyl)piperazinyl]}-2-phenyl-N-phenylpropanamide

A solution of the product of Example 1 (1.102 g, 3.2 mmol) in dichloromethane (50 ml) was treated with 1,1'-carbonyldiimidazole (0.58 g, 3.6 mmol), stirred for 1 hour, treated with aniline (0.4 ml, 4.4 mmol), stirred for 18h, evaporated in vacuo, and the residue purified by chromatography (silica; di-iso-propyl ether --> ether). The foam was dissolved in hot propan-2-ol (10 ml) and the solution acidified with ethereal hydrogen chloride. Evaporation in vacuo gave a glass which crystallised upon trituration with ether as the dihydrochloride quarter hydrate salt of the product (0.897 g), m.p. 250-255° (dec.).
(Found: C, 63.4; H, 6.8; N, 8.5.
$C_{26}H_{29}N_3O_2.2HCl\frac{1}{4}H_2O$ requires C, 63.35; H, 6.4; N, 8.5%.)

EXAMPLES 7-21

The following3-{1-[4-(2-methoxyphenyl)piperazinyl]}2-phenylpropanamides were prepared following the procedure of Example 6 but using the indicated amine reactant instead of aniline.

3-{1-4-(2-Methoxyphenyl)piperazinyl]}-2-phenylpropanamides

| Example | Amine Reactant | Amide Product | Formula | Found % (Required) C | H | N | m.p. (°C) |
|---|---|---|---|---|---|---|---|
| 7 | $NH_3$-THF | amide | $C_{20}H_{25}N_3O_2 2HCl$ | 58.05 (58.25) | 6.7 (6.6) | 10.2 (10.2) | 194-195 |
| 8 | $MeNH_2$-EtOH (33% W/W) | methylamide | $C_{21}H_{27}N_3O_2 2HCl \frac{3}{4}H_2O$ | 57.6 (57.3) | 7.15 (7.0) | 9.2 (9.55) | 203-205 |
| 9 | $EtNH_2$-EtOH (33% W/W) | ethylamide | $C_{22}H_{29}N_3O_2 2HCl \frac{1}{4}H_2O$ | 59.4 (59.4) | 7.2 (7.1) | 9.5 (9.4) | 204-206 |
| 10 | $PrNH_2$ | propylamide | $C_{23}H_{31}N_3O_2 2HCl$ | 60.6 (60.8) | 7.6 (7.3) | 9.7 (9.25) | 213-215 |
| 11 | $BuNH_2$ | butylamide | $C_{24}H_{33}N_3O_2 2HCl$ | 61.6 (61.5) | 7.8 (7.5) | 9.2 (9.0) | 199-200 |
| 12 | iso-$PrNH_2$ | iso-propylamide | $C_{23}H_{31}N_3O_2 2HCl$ | 60.55 (60.8) | 7.6 (7.3) | 9.6 (9.25) | 221-224 |
| 13 | iso-$BuNH_2$ | iso-butylamide | $C_{24}H_{33}N_3O_2 2HCl$ | 61.4 (61.5) | 7.7 (7.5) | 9.0 (9.0) | 202-203 |

| | | | | | | | |
|---|---|---|---|---|---|---|---|
| 14 | $C_3H_5CH_2NH_2$ | cyclopropyl-methylamide | $C_{24}H_{31}N_3O_2 2HCl$ | 61.9 (61.8) | 7.0 (7.1) | 8.7 (9.0) | 200-202 |
| 15 | t-$BuCH_2NH_2$ | neopentylamide | $C_{25}H_{35}N_3O_2 2HCl$ | 61.9 (62.2) | 7.6 (7.7) | 8.7 (8.7) | 200-203 |
| 16 | $C_6H_{13}NH_2$ | hexylamide | $C_{26}H_{37}N_3O_2 2HCl$ | 62.5 (62.9) | 7.9 (7.5) | 8.4 (8.5) | 189-190 |
| 17 | $C_6H_{11}CH_2NH_2$ | cyclohexyl-methylamide | $C_{27}H_{37}N_3O_2 2HCl$ | 63.5 (63.8) | 8.0 (7.7) | 8.4 (8.3) | 205-207 |
| 18 | $C_5H_9NH_2$ | cyclopentylamide | $C_{25}H_{33}N_3O_2 2HCl \frac{1}{4}H_2O$ | 62.0 (62.0) | 7.3 (7.4) | 8.9 (8.7) | 213-214 |
| 19 | $C_6H_{11}NH_2$ | cyclohexylamide | $C_{26}H_{35}N_3O_2 2HCl$ | 63.0 (63.15) | 7.8 (7.5) | 8.1 (8.5) | 216-219 |
| 20 | $C_7H_{13}NH_2$ | cycloheptylamide | $C_{27}H_{37}N_3O_2 2HCl$ | 63.6 (63.8) | 7.9 (7.7) | 8.0 (8.3) | 206-208 |
| 21 | $C_{11}H_{18}N_2O_2$ a | b | $C_{31}H_{40}N_4O_4 2HCl$ | 61.4 (61.5) | 7.3 (7.0) | 9.2 (9.25) | 148-152 |

a 8-(Aminoethyl)-8-azaspiro[4.5]deca-7,9-dione

b The product was 8-{α-{1-[4-(2-methoxyphenyl)piperazinyl]methyl}phenylacetamidoethyl}-8-azaspiro[4.5]deca-7,9-dione

EXAMPLE 22

N,N-Dimethyl-3-{1-[4-(2-methoxyphenyl)piperazinyl]}-2-phenylpropanamide

A stirred solution of the product of Example 1 (1.786 g, 5.2 mmol) and N-methylmorpholine (0.65 ml, 5.9 mmol) in dichloromethane (20 ml) at -30° was treated with diphenylphosphinyl chloride (1.1 ml, 5.8 mmol) under an atmosphere of nitrogen. After 1 hour, 25-30% w/v dimethylamine in water (1.2 ml, ca. 7.5 mmol) was added and the solution warmed to room temperature over 3 hours. Evaporation in vacuo and chromatography (silica, ethyl acetate) gave the free base (0.532 g.)

The solid was dissolved in hot methanol (5 ml) and the solution acidified with ethereal hydrogen chloride and evaporated in vacuo to give the dihydrochloride three-quarter hydrate salt of the product (0.581 g) as colourless crystals, mp. 236-238° (dec.).
(Found: C, 58.1; H, 7.4; N, 9.1.
$C_{22}H_{29}N_3O_2.2HCl.\frac{3}{4}H_2O$ requires C, 58.2; H, 7.2; N, 9.3%.)

EXAMPLE 23

3-{1-[4-(2-Methoxyphenyl)piperazinyl]}-2-phenyl-N-(phenylmethyl)propanamide

A solution of the product of Example 3 (1.25 g, 5.3 mmol), 1-(2-methoxyphenyl)piperazine (1.00 g, 5.2 mmol), and acetic acid (3 drops) was heated under reflux under an atmosphere of nitrogen for 40 hours, cooled to room temperature, and evaporated in vacuo to give an oil which crystallised from ethyl acetate. A suspension of the crystals in hot propan-2-ol was acidified with ethereal hydrogen chloride. The hot solution was cooled to room temperature and the precipitate filtered and washed with propan-2-ol and ether to give the product (1.94 g), m.p. 211-214°.
(Found: C, 64.5; H, 7.1; N, 7.9.
$C_{27}H_{31}N_3O_2.2HCl$ requires C, 64.5; H, 6.6; N, 8.4%.)

EXAMPLE 24

N-Cyclohexyl-2-phenyl-3-{1-[4-(2-pyrimidinyl)piperazinyl]}propanamide

A solution of the product of Example 4 (0.90 g, 3.9 mmol) and N-(2-pyrimidyl)piperazine dihydrochloride (0.95 g, 4.0 mmol) in 1 N-NaOH (8.0 ml), propanol (10 ml), and acetic acid (8 drops) was heated under reflux for 48 hours, cooled to room temperature, and concentrated in vacuo. The aqueous residue was diluted with water (50 ml) and extracted with dichloromethane (50 ml). The extracts were washed with water (50 ml), dried ($MgSO_4$), and evaporated in vacuo to give an oil. Purification by column chromatography (alumina; ether) gave the product free base (0.63 g) as colourless crystals.

The crystals were dissolved in hot propan-2-ol (20 ml), acidified with ethereal hydrogen chloride, and evaporated in vacuo to give a foam which crystallised upon trituration with ether as the dihydrochloride three-quarter hydrate salt of the product (0.67g) m.p. 165-175°.
(Found: C, 62.5; H, 7.6; N, 15.6.
$C_{23}H_{31}N_5O.HCl.\frac{3}{4}H_2O$ requires C, 62.3; H, 7.6; N, 15.8%.)

EXAMPLE 25

3,N-Bis{1-[4-(2-methoxyphenyl)piperazinyl]}-2-phenylpropanamide

This compound was isolated as a side-product from the reaction following the procedure of Example 6 but substituting t-butylamine for aniline. The dihydrochloride trihydrate of the product was produced by standard methods as colourless crystals, m.p. 180-190° (dec.)
(Found: C, 57.9; H, 7.3; N, 8.3.
$C_{31}H_{38}N_4O_3.2HCl.3H_2O$ requires C, 58.0; H, 7.2; N, 8.7%.)

EXAMPLE 26

2-Methylpropyl 3-{1-[4-(2-methoxyphenyl)piperazinyl]}-2-phenylpropanoate

Step 1: 2-Methylpropyl 2-phenylpropenoate

This compound was isolated as a side-product from the reaction described in Example 3 as a colourless oil (1.03 g) and was used without further purification in Step 2.

Step 2: 2-Methylpropyl 3-{1-[4-(2-methoxyphenyl)piperazinyl]}-2-phenylpropanoate

A solution of the product of Step 1 (0.97 g, 4.7 mmol) and 1-(2-methoxyphenyl)piperazine (0.91 g, 4.7 mmol) in propanol (10 ml) was maintained at room temperature for 90 hours and evaporated in vacuo. The residue was purified by chromatography (silica; di-iso-propyl ether) to give an oil. The oil was dissolved in propan-2-ol (10 ml) and the solution acidified with ethereal hydrogen chloride and evaporated in vacuo. The solid was triturated with ether to give the dihydrochloride salt of the product (1.173 g) m.p. 208-212°.
(Found: C, 61.6; H 7.6; N, 5.8.
$C_{24}H_{32}N_2O_3 2HCl$ requires C, 61.4; H, 7.3; N, 6.0%.)

EXAMPLE 27

Ethyl 3-{1-[4-(2-methoxyphenyl)piperazinyl]}-2-phenylpropanoate

This compound was isolated as a side-product from the reaction described in Example 9. The dihydrochloride salt of the product, mp. 220-222° was produced in the conventional manner.
(Found: C, 59.5; H, 6.9; N, 6.5.
$C_{22}H_{28}N_2O_3 2HCl$ requires C, 59.9; H, 6.85; N, 6.35%).

EXAMPLE 28

2-{1-[4-(2-Methoxyphenyl)piperazinyl]methyl}-N-methyl-3-phenypropanamide

This compound was prepared from the acid of Example 2 (2.00 g, 5.65 mmol) 1,1'-carbonyldiimidazole (0.92 g, 5.65 mmol), and methylamine solution, approx 33% w/w in industrial methylated spirit (0.58 g, ca 6.2 mmol) using the processes outlined in Example 6. The hydrochloride hydrate salt of the product was isolated as crystals (0.73 g), m.p. 186.5-188.5° (from methyl acetate-ether).
(Found: C, 62.8; H, 7.6; N, 10.0.
$C_{22}H_{29}N_3O_2.HCl.H_2O$ requires C, 62.6; H, 7.9; N, 10.0%.)

EXAMPLE 29

Ethyl 2-{1-[4-(2-methoxyphenyl)piperazinyl]methyl}-3-phenylpropanoate

This compound was prepared from the acid of Example 2 (2.00 g, 5.65 mmol) by a method analogous to that described in Example 28 with the exception that excess ethanol was used in place of methylamine. The dihydrochloride quarter hydrate salt of the salt (1.20 g) was isolated as crystals, m.p. 197-201°.
(Found: C, 60.2; H, 7.3; N, 6.0.
$C_{23}H_{30}N_2O_3.2HCl.\frac{1}{4}H_2O$ requires C, 60.1; H, 7.1; N, 6.1%.)

EXAMPLES 30-36

The following 3-{1-[4-(2-methoxyphenyl)piperazinyl]}-2-phenylpropanamides were prepared following the procedure of Example 6, but using the indicated amine reactant instead of aniline.

3-{1-[4-(2-Methoxyphenyl)piperazinyl]}-2-phenylpropanamides

| Example | Amine Reactant | Amide Product | Formula | Found % (Required) C | H | N | m.p. (°C) |
|---|---|---|---|---|---|---|---|
| 30 | 3,3-dimethyl-butylamine | (3,3-dimethyl)-butylamide | $C_{26}H_{37}N_3O_2.2HCl$ | 62.6 (62.9) | 8.0 (7.9) | 8.5 (8.5) | 187-193 |
| 31 | cyclopropyl-amine | cyclopropyl-amide | $C_{23}H_{29}N_3O_2.2HCl$ | 60.7 (60.5) | 7.2 (7.0) | 9.2 (9.2) | 193-195 |
| 32 | pyrrolidine | c | $C_{24}H_{31}N_3O_2.2HCl.0.25H_2O$ | 61.4 (61.3) | 7.4 (7.2) | 9.0 (8.9) | 212-213 |
| 33 | piperidine | d | $C_{25}H_{33}N_3O_2.1.75HCl$ | 63.7 (63.7) | 7.5 (7.4) | 8.8 (8.9) | 191-194 |
| 34 | hexamethylene-imine | e | $C_{26}H_{35}N_3O_2.1.5HCl$ | 65.75 (65.6) | 7.9 (7.9) | 8.8 (8.8) | 203-204 |
| 35 | cyclooctyl-amine | cyclooctylamide | $C_{28}H_{39}N_3O_2.2HCl$ | 64.3 (64.3) | 7.9 (7.9) | 8.0 (8.0) | 204-206 |
| 36 | cyclododecyl-amine | cyclododecyl-amide | $C_{32}H_{47}N_3O_2.2HCl.0.25H_2O$ | 66.0 (65.9) | 8.5 (8.6) | 7.2 (7.2) | 184-188 |

c 1-{3-{1-[4-(2-methoxyphenyl)piperazinyl]}-2-phenylpropionyl}pyrrolidine

d 1-{3-{1-[4-(2-methoxyphenyl)piperazinyl]}-2-phenylpropionyl}piperidine

e 2,3,4,5,6,7-hexahydro-1-{3-{1-[4-(2-methoxyphenyl)piperazinyl]}-2-phenylpropionyl}-1H-azepine

EXAMPLE 37

(S)-N-[2-[1-[4-(2-Methoxyphenyl)piperazinyl]]-1-phenylethyl]cyclohexanecarboxylic acid amide

A solution of (S)-2-{1-[4-(2-methoxyphenyl)piperazinyl]}-1-phenylethylamine (1.03 g, 3.3 mmol) in dichloromethane (50 ml) was treated with cyclohexanecarboxylic acid chloride (0.5 ml, 3.7 mmol), after 40 minutes washed with 0.1 N-NaOH (100 ml), dried ($MgSO_4$), evaporated in vacuo, and the residual oil purified by column chromatography [silica; di-isopropyl ether --> ether] to give the product free base (0.83 g) as white crystals.

The crystals were dissolved in hot propan-2-ol, acidified with ethereal hydrogen chloride, evaporated in vacuo, and the resulting pink crystals dried in vacuo at 70° for 24 hours to give the title compound as the hydrochloride hydrate, m.p. 141-143°.
(Found: C, 65.7; H, 8.0; N, 9.2.
$C_{26}H_{35}N_3O_2.HCl.H_2O$ requires C, 65.6; H, 8.05; N, 8.8%.)

EXAMPLE 38

O-[2-[1-(4-(2-Methoxyphenyl)piperazinyl)]-1-phenylethyl]-N-cyclohexylcarbamate

Tributyltin methoxide (0.05 ml) was added to a solution of 2-[1-(4-(2-methoxyphenyl)piperazinyl)]-1-phenylethanol (1.00 g, 3.2 mmol) and cyclohexylisocyanate (0.44 g, 3.5 mmol) in dry toluene (10.0 ml). The reaction mixture was stirred at room temperature overnight and the suspension was treated with dichloromethane to afford a solution which was chromatographed on silica gel, gradient eluting with hexane ethyl acetate (2:1 to 1:2) to afford a white solid. The solid was dissolved in ethyl acetate and the solution acidifed with ethereal hydrogen chloride to afford the title compound as the dihydrochloride semihydrate (1.3 g), m.p. 182.4-186.3°.
(Found: C, 60.4; H, 7.2; N, 8.0.
$C_{26}H_{35}N_3O_3.2HCl.\frac{1}{2}H_2O$ requires C, 60.1; H, 7.4; N, 8.1%.)

EXAMPLE 39

O-[2-[1-[4-(2-Methoxyphenyl)piperazinyl]]-1-phenylethyl]-N-phenylcarbamate

The above compound was prepared following the procedure of Example 38 but substituting phenylisocyanate for cyclohexylisocyanate. The product was obtained as the dihydrochloride, m.p. 189.4-191.7°.

EXAMPLE 40

2-{1-[4-(2-Methoxyphenyl)piperazinyl]}-1-phenylethyl cyclohexanecarboxylate

2-[1-(4-(2-Methoxyphenyl)piperazinyl)]-1-phenyl ethanol dihydrochloride (1.50 g, 3.9 mmol) was treated with cyclohexanecarbonyl chloride prepared from the corresponding acid (1.0 g, 7.8 mmol) by reaction with thionyl chloride in chloroform and diisopropylethylamine (2.26 g, 17.5 mmol) in chloroform (15 ml). The crude product was chromatographed and the oil obtained was dissolved in acetonitrile and acidified with ethereal hydrogen chloride to afford the title compound as the dihydrochloride, m.p. 213.2-217.4°.
(Found: C, 63.0; H, 7.3; N, 5.6.
$C_{26}H_{34}N_2O_3.2HCl$ requires C, 63.0; H, 7.3; N, 5.7%.)

Example 41

Ethyl(S)-N-[2-[1-[4-(2-methoxyphenyl)piperazinyl]]-1-phenylethyl]carbamate

(S)-2-[1-[4-(2-methoxyphenyl)piperazinyl]]-1-phenylethylamine (0.94 g, 3.0 mmol) in dichloromethane (20 ml) was treated with ethyl chloroformate (0.4 ml, 4.2 mmol), and after 4 days evaporated in vacuo. The residue was chromatographed, dissolved in ethanol, acidified with constant boiling hydrobromic acid, and evaporated in vacuo to give an oil. Crystallisation from ethyl acetate-propan-2-ol gave the product as the dihydrobromide (0.08 g), m.p. 170-180° (dec.).
(Found: C, 48.6; H, 6.0; N, 7.4.
$C_{22}H_{29}N_3O_3.2HBr$ requires C, 48.5; H, 5.7; N, 7.7%.)

EXAMPLE 42

Methyl 3-[1-[4-(2-methoxyphenyl)piperazinyl]]-2-phenylpropionate

1,1'-Carbonyldiimidazole (1.62 g, 10.0 mmol) was added to a stirred suspension of 3-[1-[4-(2-methoxyphenyl)-piperazinyl]]-2-phenylpropionic acid (3.40 g, 10.0 mmol) in dry tetrahydrofuran (40 ml). The mixture was stirred at room temperature for 1 hour and methanol (40 ml, 32 g, 990 mmol) was added. The solution was stirred at room temperature for 18 hours, and was concentrated in vacuo to give a pale yellow oil. The product was chromatographed on silica with eluant ether to give the title compound as the free base (2.37 g). A portion of the product (0.65 g) was dissolved in ethyl acetate (30 ml) and the solution was acidified with ethereal hydrogen chloride (5 ml). The mixture was concentrated in vacuo, the product was dissolved in methanol, and the solution was concentrated in vacuo. The product was triturated with acetonitrile to give the title compound as the dihydrochloride (691 mg), m.p. 211-212°.
(Found: C, 58.8; H, 6.9; N, 6.3.
$C_{21}H_{26}N_2O_3$.2HCl requires C, 59.0; H, 6.6; N, 6.5%.)

EXAMPLE 43

N-(1-Ethylpropyl)-3-[1-[4-(2-methoxyphenyl)-piperazinyl]]-2-phenylpropionamide

1,1'-Carbonyldiimidazole (649 mg, 4.0 mmol) was added to a stirred suspension of 3-[1-[4-(2-methoxyphenyl] piperazinyl]]-2-phenylpropionic acid (1.36 g, 4.0 mmol) in dry tetrahydrofuran (20 ml). The mixture was stirred at room temperature for 1 hour, and 1-ethylpropylamine (0.6 ml, 0.45 g, 5.1 mmol) was added dropwise. The mixture was stirred at room temperature for 18 hours, and was concentrated in vacuo to give a white semi solid. The product was chromatographed on silica with eluant ethyl acetate to give the title compound as the free base (0.90 g). The product was dissolved in ethyl acetate (45 ml), and the solution was acidified to give the dihydrochloride half hydrate (0.90 g), m. p. 204-208°.
(Found: C, 61.3; H, 7.8; N, 8.5.
$C_{25}H_{35}N_3O_2$.2HCl.O.5$H_2O$ requires C, 61.1; H, 7.8; N, 8.55%.)

EXAMPLE 44

4-[3-[1-[4-(2-methoxyphenyl)piperazinyl]]-2-phenylpropionyl]morpholine

The above compound was prepared following the procedure of Example 43 substituting morpholine for 1-ethylpropylamine. The product was isolated as the dihydrochloride, m.p. 213-217°.

Example 45

1-{3-{1-[4-(2-Methoxyphenyl)piperazinyl]}-2-phenylpropionyl}azetidine

1,1-carbonyldiimidazole ().81g, 5 mmol) was added to a stirred suspension of 3-{1-[4-(2-methoxyphenyl)piperazinyl]}-2-phenylpropionic acid (1.70g, 5mmol) in dry tetrahydrofuran (25ml). The suspension was stirred at room temperature for lh, and azetidine (1.12g, 20mmol) was added in one portion.
After 21h the solution was concentrated in vacuo to give a solid which was purified by chromatography (silica; ethyl acetate). The free base was dissolved in methanol, the solution was acidified with ethereal hydrogen chloride and evaporated in vacuo, and the solid triturated with acetonitrile to give the product as the dihydrochloride quarter hydrate (0.25g), m.p. 181°-184° (Found: C, 60.4; H, 7.0; N, 9.3;
$C_{23}H_{29}N_3O_2$.2HCl.0.25$H_2O$ requires C, 60.3; H, 6.9; N, 9.2%).

Example 47

O-{2-[1-[4-(2-Methoxyphenyl)piperazinyl]]-1-phenylethyl}-N-(3-chlorophenyl)carbamate

Tributyltin methoxide (0.10ml) was added to a stirred solution of 2-{1-[4-(2-methoxyphenyl)piperazinyl]}-1-phenylethanol (1.60g, 5.1mmol) and 3-chlorophenylisocyanate (0.87g, 5.7mmol) in dichloromethane (15ml). The mixture was stirred for 70h, filtered, and the filtrate evaporated in vacuo. The residue was purified by chromatography [silica; hexane-ethyl acetate (1:1->1:2] to afford an oil which solidified on standing. The off white solid was dissolved in acetonitrile (10ml) and acidified with ethereal hydrogen chloride to afford the product as the one and eight-tenth

hydrochloride salt (1.57g), m.p. 159.2-163.0° (Found: C, 58.6; H, 5.9; N, 7.9;$C_{26}H_{28}N_3O_3Cl$.1.8HCl requires C, 58.5; H, 5.6; N, 7.9%

Example 47

1-Ethyl-3-{2-[1-[4-2-methoxyphenyl)piperazinyl]]-1-phenylethyl} carbonate

Ethyl chloroformate (0.60g, 5.5 mmol) was added to a solution of 2-{1-[4-(2-methoxyphenyl)piperazinyl]}-1-phenylethanol (1.57g, 5.0mmol) and triethylamine (0.56g, 5.5mmol) in dichloromethane (15ml). The mixture was stirred for 70h, evaporated in vacuo, and the residue purified by chromatography [silica; hexane-ethyl acetate (1:1)] to afford an oil. The oil was dissolved in a acetonitrile (10ml) and acidified with ethereal hydrogen chloride to afford the dihydrochloride salt of the product (0.41g), m.p. 213-215° (dec.) Found C, 57.6; H, 6.7; N. 6.1; $C_{22}H_{28}N_2O_4$.2HCl requires C, 57.8; H, 6.6; N, 6.1%)

Example 48

2,3-Dihydrobenzo[1,4]dioxin-2-ylcarboxylic acid 2-[4-(2-methoxyphenyl)piperazin-1-yl]-1-phenylethyl ester

2,3-Dihydrobenzo[1,4]dioxin-2-ylcarboxylic acid (2.10g, 11.7mmol) in thionyl chloride (10.0ml) was heated under reflux for 1h and the excess thionyl chloride removed under reduced pressure. The crude acid chloride was dissolved in dichloromethane (10.0ml) and a solution of 2-{1-[4-(2-methoxyphenyl)piperazinyl]}-1-phenylethanol dihydrochloride (1.50g, 3.9mmol) in dichloro-methane (5.0ml) added, followed by triethylamine (1.22g, 12.1mmol). The mixture was stirred for 18h, filtered and the filtrate evaporated in vacuo. The oil was purified by chromatography [silica; hexane-ethyl acetate (2:1->2;3], dissolved in acetonitrile (10ml) and acidified with ethereal hydrogen to give the product as a dihydrochloride salt (1.37g), m.p. 202-206° (Found C, 61.5; H, 6.0: N, 5.1.$C_{28}H_{30}N_2O_5$.2HCl requires C, 61.4; H, 5,9; N, 5.1%)

Example 49

(S)-8-{2-[1-[4-(2-Methoxyphenyl)piperazinyl]]-1-phenylethyl}-8-azaspiro[4.5]decan-7,9-dione

A solution of (S)-2-{1-[4-(2-methoxyphenyl) piperazinyl]}-1-phenethylamine (1.0g, 3,2mmol) and 3,3-tetramethyleneglutaric anhydride (0.543g, 3,2mmol) in pyridine (10ml) was heated under reflux under an atmosphere of nitrogen for 21h, cooled to room temperature, and evaporated in vacuo to give a brown oil. A solution of the oil in acetic anhydride (15ml) was heated under reflux for 21h, cooled to room temperature, and evaporated in vacuo. The residue in water was basified with saturated aqueous ammonia and extracted with ethyl acetate (2 x 50ml). The extracts were washed with water (100ml), dried ($MgSO_4$), and evaporated in vacuo. The residue was purified by chromatography [silica; hexane-ethyl acetate (3:2)] to give the product free base a yellow oil (0.82g).

The oil was dissolved in methanol (5ml) and the solution acidifed with ethereal hydrogen chloride, evaporated in vacuo, and the crystals triturated with ether to give the dihydrochloride salt of the product (0.63g) m.p. 203-206° (Found: C, 62.9; H, 7.0; N, 7.9;.$C_{28}H_{35}N_3O_3$.2HCl requires C, 62.5; H, 7.1; N, 8.0%).

Example 50

2,3,4,5,6,7-Hexahydro-1-{2-[1-(4-(2-methoxyphenyl) piperazinyl)methyl]-3-phenylpropanoyl}-1H-azepine

This compound was prepared from the acid of Example 2 (20g, 5.65mmol),1,1'-carbonyldiimidazole (0.92g, 5.7mmol), and hexahydro-1H-azepine (0.62g, 6.3mmol) using the method outlined in Example 6 and the crude product was purified by chromatography [silica; ethyl acetate-hexane (1:1)]. The sesquihydrochloride salt of the product was isolated as crystals (0.96g), m.p. 195-195.5° (from ethyl acetate).
(Found: C, 65.9; H, 8.0; N, 8.5.
$C_{27}H_{37}N_3O_2.1\frac{1}{2}$HCl requires C, 66.1; H, 7.9; N,8.6%)

Example 51

N-Cycloheptyl-2-{1-[4-(2-methoxyphenyl) piperazinyl]methyl}-3-phenylpropanamide

This compound was prepared from the dihydrochloride salt of the acid of Example 2 (2.2g, 4.5mmol), 1,1'-carbonyldiimidazole (0.8g, 4.9mmol), and cycloheptylamine (0.56g, 4.9mml) in the presence of triethylamine (1.18g, 11.7mmol) using the method outlined in Example 50. The dihydrochloride salt of the product was isolated as off white crystals (0.91g), m.p. 178.5 - 181.5°.
(Found: C, 64.1; H, 7.9; N, 8.0.
$C_{28}H_{39}N_3O_2$.2HCl requires C, 64.4; H, 7.9; N, 8.0%).

Example 52

N-Cyclopropyl-2-{1-[4-(2-methoxyphenyl) piperazinyl]methyl}-3-phenylpropanamide

This compound was prepared from the acid of Example 2 (2.5g, 7.0mmol), 1,1'-carbonyldiimidazole (1.2g, 7.4mol) and cyclopropylamine (0.44g, 7.7mmol) using the method outlined in Example 51.. The hydrochloride quarter hydrate salt of the product was isolated as a powder (2.12g,), m.p. 169 - 170.5°
(Found: C, 66.3; H 7.6; N, 9.4.
$C_{24}H_{31}N_3O_2$.HCl. 1/4$H_2O$ requires C, 66.3; H, 7.5; N, 9.7%).

Example 53

(a) a-{[1-(4-Phenylpiperazinyl)]methyl}benzeneacetic acid

1-Phenylpiperazine and atropic acid in ethanol is heated under reflux for 18h, cooled to room temperature, and evaporated in vacuo. The solid is triturated with acetone to give the title product.

(b) N-Cyclopropyl-2-phenyl-3-[1-(4-phenylpiperazinyl)] propanamide

This compound is prepared by the reaction of the product of Example 53(a) with 1,1'-carbonyldiimidazole and cyclopropylamine following the procedure outlined in Example 6.

Example 54

N-cyclohexyl-2-phenyl-3-{1-[4-(3-trifluoromethylphenyl) piperazinyl]}propanamide

A solution of the product of Example 4 and N-(3-trifluoromethylphenyl)piperazine is heated in propanol in the presence of a small quantity of acetic acid as catalyst to give the title product.

Example 55

(a) 2-{1-[4-(1-Naphthyl)piperazinyl]}-1-phenylethanol

Styrene oxide and 1-(1-naphthyl)piperazine in acetonitrile are heated under reflux. Concentration in vacuo and purification by chromatography (silica; ethyl acetate) gives the product.

(b) 2-{1-[4-(1-Naphthyl)piperazinyl]}-1-phenylethyl cyclohexananecarboxylate

This compound is prepared by the reaction of the product of Example 55(a) with cyclohexanecarbonyl chloride using the method described in Example 40.

## Claims

**Claims for the following Contracting States : AT, BE, CH, DE, DK, FR, IT, LI, LU, NL, SE**

**1.** A compound of the general formula (I)

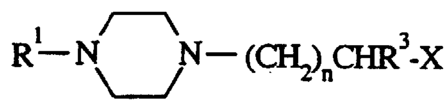

(I)

or a pharmaceutically acceptable acid addition salt thereof wherein

n is one of the integers 1 or 2,

$R^1$ is Ar or Het,

$R^3$ is Ar or an Ar($C_{1-6}$)alkyl radical,

X is $-OCOR^{10}$, $-CO_2R^6$, $-CONR^5R^9$, $-OCO_2R^6$, $-NR^4COR^6$, $OCONHR^{11}$, $-NHCO_2R^6$, $-NR^4CONHR^6$, $-CONHNHR^6$, $-CONHOR^6$,

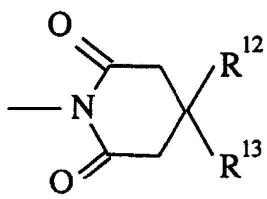

or

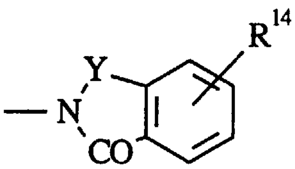

$R^4$ and $R^5$ are each hydrogen or $C_{1-6}$ alkyl $R^6$ is $-CHR^7R^8$, cycloalkyl of 3 to 12 carbon atoms or Ar($C_{1-6}$)alkyl (where $R^7$ and $R^8$ are each hydrogen or $C_{1-6}$ alkyl)

$R^9$ is hydrogen, an alkyl group of 1 to 8 carbon atoms other than a tertiary alkyl group, cycloalkyl of 3 to 12 carbon atoms, cycloalkyl($C_{1-6}$)alkyl, Ar($C_{1-6}$)alkyl or 8-azaspiro [4.5]deca-7,9-dione-8-yl-($C_{1-6}$)alkyl [with the proviso that when $R^9$ is hydrogen, alkyl or Ar($C_{1-6}$)alkyl, $R^5$ is other than a tertiary alkyl group],

or R5 and $R^9$ together with the nitrogen atom to which they are attached represent an azetidino, pyrrolidino, piperidino, hexahydroazepino, morpholino or piperazino ring which may be optionally substituted by $C_{1-6}$ alkyl, Ar or Ar($C_{1-6}$)alkyl

$R^{10}$ is cycloalkyl of 3 to 12 carbon atoms, or 2,3-dihydro[1,4]benzodioxinyl optionally substituted by $C_{1-6}$ alkyl, $C_{1-6}$ alkoxy or halogen,

$R^{11}$ is cycloalkyl of 3 to 12 carbon atoms, Ar or Ar($C_{1-6}$)alkyl,

$R^{12}$ and $R^{13}$ are each $C_{1-6}$ alkyl or together with the carbon atom to which they are both attached represent $C_{4-6}$ cycloalkyl,

$R^{14}$ represents hydrogen, halogen, $C_{1-6}$ alkyl or $C_{1-6}$ alkoxy
and

Y is CO or $SO_2$

with the further provisos that

(a) when $R^1$ is phenyl optionally substituted by $CH_3$, $OCH_3$, Cl or $CF_3$, $R^3$ is phenyl optionally substituted by $C_{1-6}$ alkyl, methoxy or halogen and X is $-CONH_2$, $-CON(CH_3)_2$ or $-COOC_2H_5$ then n is 1

and (b) when $R^1$ is 2-pyrimidyl, $R^3$ is phenyl optionally substituted at one or two ring positions with $C_{1-6}$ alkyl, halogen, trifluoromethyl, cyano, nitro, ($C_{1-6}$)alkylamino, di($C_{1-6}$)alkylamino, $C_{1-6}$-alkyloxy and X is

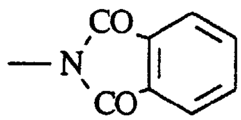

or $-NR^4COR^6$ where $R^4$ is hydrogen or $C_{1-6}$ alkyl and $R^6$ is $-CHR^7R^8$ (where $R^7$ and $R^8$ are each hydrogen or $C_{1-6}$ alkyl and $-CHR^7R^8$ contains a total of 1 to 6 carbon atoms) then n is 2;

and wherein the term "Ar" means an aromatic radical having 6 to 12 carbon atoms which optionally may be substituted by one or more $C_{1-6}$ alkoxy, halogen, trifluoromethyl, nitro, carbalkoxy, carboxamido, cyano, amino, $(C_{1-6})$alkylamino or di$(C_{1-6})$alkylamino substituents and the term "Het" means an aromatic radical containing one or more nitrogen atoms as heteroatoms and which may be optionally substituted by one or more $C_{1-6}$ alkyl, $C_{1-6}$ alkoxy, halogen, trifluoromethyl, amino, $(C_{1-6})$alkylamino or di$(C_{1-6})$alkylamino substituents.

**2.** A compound as claimed in claim 1 in which $R^1$ is a phenyl radical optionally substituted by one or more $C_{1-6}$ alkoxy, halogen, trifluoromethyl, nitro, carbalkoxy, carboxamido, cyano, amino, $(C_{1-6})$alkylamino or di$(C_{1-6})$alkylamino substituents.

**3.** A compound as claimed in claim 1 or 2 in which $R^3$ is a phenyl radical optionally substituted by one or more $C_{1-6}$ alkoxy, halogen, trifluoromethyl, nitro, carbalkoxy, carboxamido, cyano, amino, $(C_{1-6})$alkylamino or di$(C_{1-6})$alkylamino substituents.

**4.** A compound as claimed in any one of claims 1 to 3 in which X represents $-CO_2R^6$ or $-CONR^5R^9$ where $R^6$, $R^5$ and $R^9$ are as defined in claim 1.

**5.** A compound as claimed in any one of claims 1 to 3 where X is $-CONR^5R^9$ wherein $R^5$ and $R^9$ together with the nitrogen atom to which they are attached represent an azetidino, pyrrolidino, piperidino, hexahydroazepino, morpholino or piperazino ring.

**6.** A compound as claimed in claim 1 which is 1-{3-{1-[4-(2-methoxyphenyl)-piperazinyl]}-2-phenylpropionyl}pyrrolidine or a pharmaceutically acceptable acid addition salt thereof.

**7.** A compound as claimed in claim 1 which is 1-{3-{1-[4-(2-methoxyphenyl)-piperazinyl]}-2-phenylpropionyl}piperidine or a pharmaceutically acceptable acid addition salt thereof.

**8.** A compound as claimed in claim 1 in which is

propyl 3-{1-[4-(2-methoxyphenyl)piperazinyl]}-2-phenylpropanoate,

3-{1-[4-(2-methoxyphenyl)piperazinyl]}-2-phenyl-N-phenylpropanamide,

3-{1-[4-(2-methoxyphenyl)piperazinyl]}-2-phenylpropanamide,

N-methyl-3-{1-[4-(2-methoxyphenyl)piperazinyl]}-2-phenylpropanamide,

N-ethyl-3-{1-[4-(2-methoxyphenyl)piperazinyl]}-2-phenylpropanamide,

N-propyl-3-{1-[4-(2-methoxyphenyl)piperazinyl]}-2-phenylpropanamide,

N-butyl-3-{1-[4-(2-methoxyphenyl)piperazinyl]}-2-phenylpropanamide,

N-isobutyl-3-{1-[4-(2-methoxyphenyl)piperazinyl]}-2-phenylpropanamide,

N-cyclopropylmethyl-3-{1-[4-(2-methoxyphenyl)piperazinyl]}-2-phenylpropanamide,

N-neopentyl-3-{1-[4-(2-methoxyphenyl)piperazinyl]}-2-phenylpropanamide,

N-hexyl-3-{1-[4-(2-methoxyphenyl)piperazinyl]}-2-phenylpropanamide,

N-cyclohexylmethyl-3-{1-[4-(2-methoxyphenyl)piperazinyl]}-2-phenylpropanamide,

8-{α-{1-[4-(2-methoxyphenyl)piperazinyl]methyl}phenylacetamidoethyl-8-azaspiso[4.5]deca-7,9-dione,

N,N-dimethyl-3-{1-[4-(2-methoxphenyl)piperazinyl]}-2-phenylpropanamide,

3-{1-[4-(2-methoxyphenyl)piperazinyl]}-2-phenyl-N-(phenylmethyl)propanamide,

N-cyclohexyl-2-phenyl-3-{1-[4-(2-pyrimidinyl)-piperazinyl]}propanamide,

3,N-bis{1-[4-(2-methoxyphenyl)piperazinyl]}-2-phenylpropanamide,

2-methylpropyl 3-{1-[4-(2-methoxyphenyl)piperazinyl]}-2-phenylpropanoate,

ethyl 3-{1-[4-(2-methoxyphenyl)piperazinyl]}-2-phenylpropanoate,

2-{1-[4-(2-methoxyphenyl)piperazinyl]methyl}-N-methyl-3-phenylpropanamide,

ethyl 2-{1-[4-(2-methoxyphenyl)piperazinyl]methyl}-3-phenylpropanoate,

3-{1-[4-(2-methoxyphenyl)piperazinyl]}-N-(3,3-dimethyl)butyl-2-phenylpropionamide,

N-cyclopropyl-3-{1-[4-(2-methoxyphenyl)piperazinyl]}2-phenylpropanamide,

1-{3-{1-[4-(2-methoxyphenyl)piperazinyl]}-2-phenylpropionyl}pyrrolidine,

N-cyclooctyl-3-{1-[4-(2-methoxyphenyl)piperazinyl]}-2-phenylpropionamide,

N-cyclododecyl-3-{1-[4-(2-methoxyphenyl)piperazinyl]}-2-phenylpropionamide,

(S)-N-[2-[1-[4-(2-methoxyphenyl)piperazinyl]]-1-phenylethyl]cyclohexanecarboxylic acid amide,

O-[2-[1-(4-(2-methoxyphenyl)piperazinyl)]-1-phenylethyl]-N-cyclohexylcarbamate,

O-[2-[1-[4-(2-methoxyphenyl)piperazinyl]]-1-phenylethyl]-N-phenylcarbamate,

2-{1-[4-(2-methoxyphenyl)piperazinyl]}-1-phenylethyl cyclohexanecarboxylate,

ethyl (S)-N-[2-[1-[4-(2-methoxyphenyl)piperazinyl]]-1-phenylethyl]carbamate,

methyl 3-[1-[4-(2-methoxyphenyl)piperazinyl]]-2-phenylpropionate,

N-(1-ethylpropyl)-3-[1-4-(2-methoxyphenyl)-piperazinyl]]-2-phenylpropionamide,

4-[3-[1-[4-(2-methoxyphenyl)piperazinyl]]-2-phenylpropionyl]morpholine,

1-{3-{1-[4-(2-methoxyphenyl)piperazinyl]}-2-phenylpropionyl}azetidine,

O-{2-[1-[4-(2-methoxyphenyl)piperazinyl]]-1-phenylethyl}-N-(3-chlorophenyl)carbamate,

1-ethyl-3-{2-[1-[4-(2-methoxyphenol)piperazinyl]]-1-phenylethyl}carbonate,

2,3-dihydrobenzo[1,4]dioxin-2-ylcarboxylic acid 2-[4-(2-methoxyphenyl)piperazin-1-yl]-1-phenylethyl ester

(S)-8-{2-[1-[4-(2-methoxyphenyl)piperazinyl]]-1-phenylethyl]}-8-azaspiro[4.5]decan-7,9-dione,

2,3,4,5,6,7-hexahydro-1-{2-[1-(4-(2-methoxyphenyl)piperazinyl)methyl]-3-phenylpropanoyl}-1H-azepine,

N-cycloheptyl-2-{1-[4-(2-methoxyphenyl)piperazinyl]methyl}-3-phenylpropanamide
or

N-cyclopropyl-2-{1-[4-(2-methoxyphenyl)piperazinyl]methyl}-3-phenylpropanamide

or a pharmaceutically acceptable acid addition salt thereof.

**9.** A process for preparing a compound claimed in claim 1 in which X represents $-CONR^5R^9$ which comprises acylating an amine of formula

$$NHR^5R^9 \quad (II)$$

(where $R^5$ and $R^9$ are as defined in claim 1) with an acid of formula

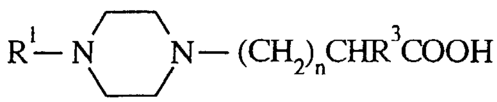

(III)

(where $R^1$ and $R^3$ are as defined in claim 1) or with an acylating derivative thereof

**10.** A process for preparing a compound claimed in claim 1 in which X is $-NR^4COR^6$ which comprises acylating a piperazine alkylamine of formula

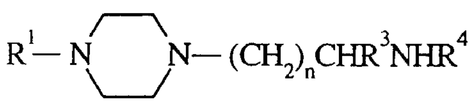

with an acid of formula

$$R^6COOH$$

or with an acylating derivative thereof.

**11.** A process for preparing a compound claimed in claim 1 in which X is

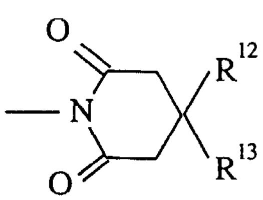

or

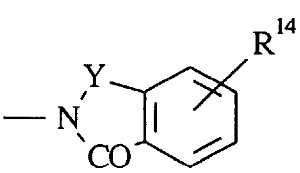

which comprises reacting an amine of formula

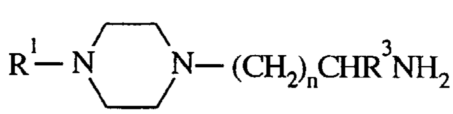

with an anhydride of formula

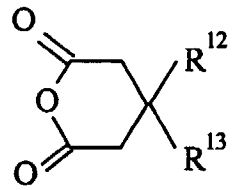

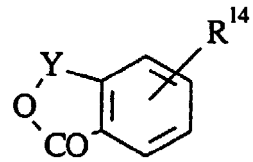

or, for preparing the compound where Y is $SO_2$, reacting the amine with an acid of formula

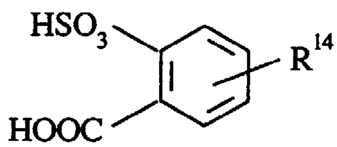

**12.** A process for preparing a compound claimed in claim 1 in which X is $-CO_2R^6$ which comprises esterifying an acid of formula (III), as given in claim 9 above with an alcohol of formula $R^6$ OH.

**13.** A process for preparing a compound claimed in claim 1 in which X is $-OCOR^{10}$ which comprises esterifying a piperazine alcohol of formula

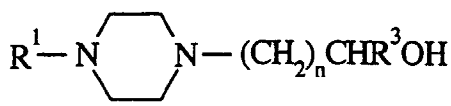

with an acid of formula $R^{10}$ COOH.

**14.** A process for preparing a compound claimed in claim 1 in which X is $-NR^4CONHR^6$ or $-O.CO.NHR^{11}$ which comprises reacting an isocyanate with a piperazinyl-alkanol of formula

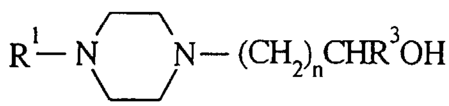

or with a piperazinyl-alkylamine of formula

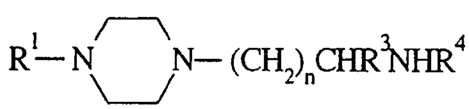

**15.** A process for preparing a compound claimed in claim 1 in which X is $-NHCO_2R^6$ which comprises reacting a piperazine isocyanate derivative of formula

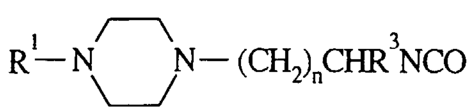

with an alcohol of formula

$R^6OH$.

**16.** A process for preparing a compound claimed in claim 1 in which X is $-NHCO_2R^6$ which comprises reacting an amine of formula

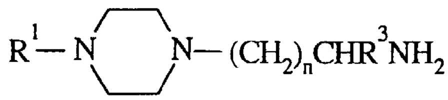

with a compound of formula $R^6OCOHal$ (where Hal is halogen).

**17.** A process for preparing a compound as claimed in claim 1 in which X is $-CONHOR^6$ which comprises reacing an acid of formula (III) given in claim 9 with a hydroxylamine of formula $NH_2$ $OR^6$.

**18.** A process for preparing a compound claimed in claim 1 which comprises alkylating a piperazine of formula

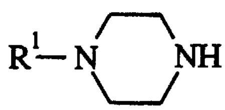

(IV)

with an alkylating agent providing the group $-(CH_2)_n$ $CHR^3X$.

**19.** A process for preparing a compound claimed in claim 1 in which X is $-CONHR^9$ where $R^9$ is a secondary $(C_{1-6})$ alkyl group or X is $-CONH_2$ which comprises reacting a nitrile of formula (IX)

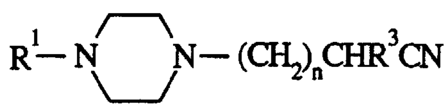

with a secondary alcohol to give a compound of formula (I) in which X is $-CONR^5R^9$ where $R^5$ is hydrogen and $R^9$ is a secondary $(C_{1-6})$alkyl group or subjecting the nitrile to acid hydrolysis to give a compound of formula (I) in which X is -CONH2.

**20.** A process for preparing a compound claimed in claim 1 in which X is $-CONHR^9$ which comprises desulphurising a sulphur containing compound of formula

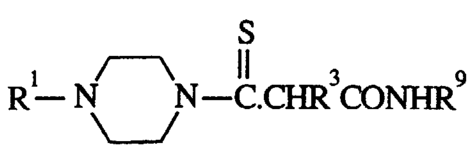

(XI)

**21.** A process for preparing a pharmaceutically acceptable salt of a compound claimed in claim 1 which comprises converting a free base of formula (I) into the salt.

**22.** A process which comprises resolving a racemic compound of formula (I) claimed in claim 1 into an optically active enantiomer.

**23.** A pharmaceutical composition comprising a compound claimed in any one of claims 1 to 8 in association with a pharmaceutically acceptable carrier.

**24.** A compound as claimed in any one of claims 1 to 8 for use as a pharmaceutical.

**25.** An acid of formula

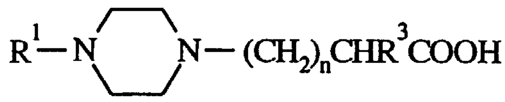

where n, $R^1$, and $R^3$ are as defined in claim 1.

**26.** A process for preparing a compound as claimed in claim 1 in which X is -CONHNHR6 which comprises reacting an acid of formula (III) as defined in claim 9 with a hydrazide of formula $NH_2NHR^6$.

**Claims for the following Contracting State : ES**

**1.** A process for preparing a compound of the formula (I)

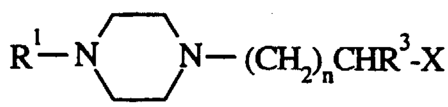

(I)

or a pharmaceutically acceptable acid addition salt thereof wherein

n is one of the integers 1 or 2,

$R^1$ is Ar or Het,

$R^3$ is Ar or an Ar($C_{1-6}$)alkyl radical,

X is -OCOR$^{10}$, -CO$_2$R$^6$, -CONR$^5$R$^9$, -OCO$_2$R$^6$, -NR$^4$COR$^6$, OCONHR$^{11}$, -NHCO$_2$R$^6$, -NR$^4$CONHR$^6$, -CONHNHR$^6$, -CONHOR$^6$,

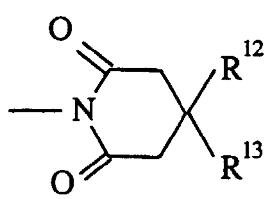

or

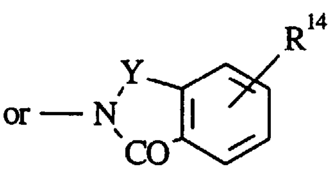

$R^4$ and $R^5$ are each hydrogen or $C_{1-6}$ alkyl

$R^6$ is -CHR$^7$R$^8$, cycloalkyl of 3 to 12 carbon atoms

or Ar($C_{1-6}$)alkyl (where $R^7$ and $R^8$ are each hydrogen or $C_{1-6}$ alkyl)

$R^9$ is hydrogen, an alkyl group of 1 to 8 carbon atoms other than a tertiary alkyl group, cycloalkyl of 3 to 12 carbon atoms, cycloalkyl($C_{1-6}$)alkyl, Ar($C_{1-6}$)alkyl or 8-azaspiro [4.5]deca-7,9-dione-8-yl-($C_{1-6}$)alkyl [with the proviso that when $R^9$ is hydrogen, alkyl or Ar($C_{1-6}$)alkyl, $R^5$ is other than a tertiary alkyl group],

or $R^5$ and $R^9$ together with the nitrogen atom to which they are attached represent an azetidino, pyrrolidino, piperidino, hexahydroazepino, morpholino or piperazino ring which may be optionally substituted by $C_{1-6}$ alkyl, Ar or Ar($C_{1-6}$)alkyl

$R^{10}$ is cycloalkyl of 3 to 12 carbon atoms, or 2,3-dihydro[1,4]benzodioxinyl optionally substituted by $C_{1-6}$ alkyl, $C_{1-6}$ alkoxy or halogen,

$R^{11}$ is cycloalkyl of 3 to 12 carbon atoms, Ar or Ar($C_{1-6}$)alkyl,

$R^{12}$ and $R^{13}$ are each $C_{1-6}$ alkyl or together with the carbon atom to which they are both attached represent $C_{4-6}$ cycloalkyl,

$R^{14}$ represents hydrogen, halogen, $C_{1-6}$ alkyl or $C_{1-6}$ alkoxy
and

Y is CO or $SO_2$

with the further provisos that

(a) when $R^1$ is phenyl optionally substituted by $CH_3$, $OCH_3$, Cl or $CF_3$, $R^3$ is phenyl optionally substituted by $C_{1-6}$ alkyl, methoxy or halogen and X is $-CONH_2$, $-CON(CH_3)_2$ or $-COOC_2H_5$ then n is 1

and (b) when $R^1$ is 2-pyrimidyl, $R^3$ is phenyl optionally substituted at one or two ring positions with $C_{1-6}$ alkyl, halogen, trifluoromethyl, cyano, nitro, ($C_{1-6}$)alkylamino, di($C_{1-6}$)alkylamino, $C_{1-6}$-alkyloxy and X is

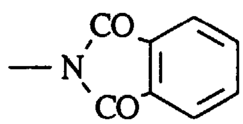

or $-NR^4COR^6$ where $R^4$ is hydrogen or $C_{1-6}$ alkyl and $R^6$ is $-CHR^7R^8$ (where $R^7$ and $R^8$ are each hydrogen or $C_{1-6}$ alkyl and $-CHR^7R^8$ contains a total of 1 to 6 carbon atoms) then n is 2;

and wherein the term "Ar" means an aromatic radical having 6 to 12 carbon atoms which optionally may be substituted by one or more $C_{1-6}$ alkoxy, halogen, trifluoromethyl, nitro, carbalkoxy, carboxamido, cyano, amino, ($C_{1-6}$) alkylamino or di($C_{1-6}$)alkylamino substituents and the term "Het" means an aromatic radical containing one or more nitrogen atoms as heteroatoms and which may be optionally substituted by one or more $C_{1-6}$ alkyl, $C_{1-6}$ alkoxy, halogen, trifluoromethyl, amino, ($C_{1-6}$)alkylamino or di($C_{1-6}$)alkylamino substituents;
which process comprises

(a) acylating an amine of formula

$$NHR^5R^9 \qquad (II)$$

(where $R^5$ and $R^9$ are as defined above) with an acid of formula

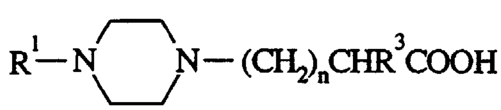

(III)

(where $R^1$ and $R^3$ are as defined above) or with an acylating derivative thereof to give a compound of formula (I) where X represents $-CONR^5R^9$

or

(b) acylating a piperazine alkylamine of formula

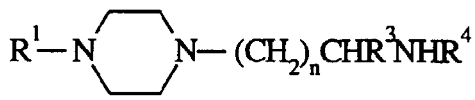

with an acid of formula

$R^6COOH$

or with an acylating derivative thereof to give a compound of formula (I) in which X is $-NR^4COR^6$
or

(c) reacting an amine of formula

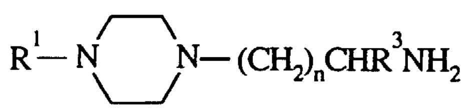

with an anhydride of formula

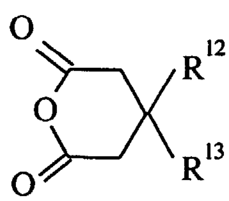

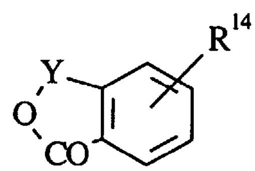

or with an acid of formula

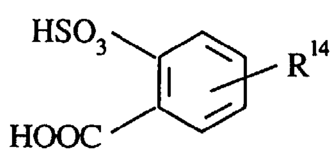

to give a compound of formula I in which X is

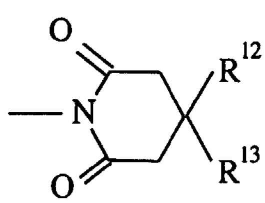

or

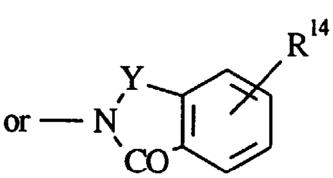

or

(d) esterifying an acid of formula (III), as given above with an alcohol of formula $R^6$ OH to give a compound

of formula (I) in which X is $-CO_2R^6$
or

(e) esterifying a piperazine alcohol of formula

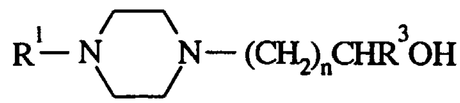

with an acid of formula $R^{10}$ COOH to give a compound of formula (I) in which X is $-OCOR^{10}$
or

(f) reacting an isocyanate with a piperazinyl-alkanol of formula

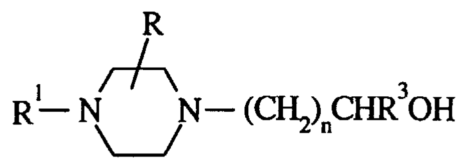

or with a piperazinyl-alkylamine of formula

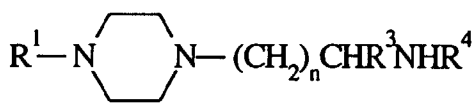

to give a compound of formula (I) in which X is $-NR^4CONHR^6$ or $-O.CO.NHR^{11}$
or

(g) reacting a piperazine isocyanate derivative of formula

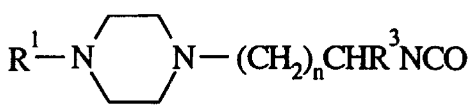

with an alcohol of formula

$R^6OH$

to give a compound of formula (I) in which X is $-NHCO_2R^6$
or

(h) reacting an amine of formula

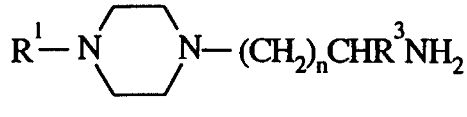

with a compound of formula $R^6OCOHal$ (where Hal is halogen) to give a compound of formula (I) in which X is $-NHCO_2R^6$
or

(i) reacting an acid of formula (III) given above with a hydroxylamine of formula $NH_2 OR^6$ or with a hydrazide of formula $NH_2 NHR^6$ to give a compound of formula I in which X is respectively $-CONHOR^6$ or $-CONHNHR^6$ or

(j) alkylating a piperazine of formula

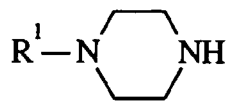

(IV)

with an alkylating agent providing the group

$$-(CH_2)_n CHR^3X$$

or

(k) reacting a nitrile of formula (IX)

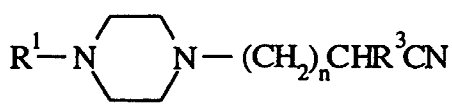

with a secondary alcohol to give a compound of formula (I) in which X is $-CONR^5R^9$ where $R^5$ is hydrogen and $R^9$ is a secondary (lower)alkyl group or subjecting the nitrile to acid hydrolysis to give a compound of formula (I) in which X is $-CONH_2$
or

(l) desulphurising a sulphur containing compound of formula

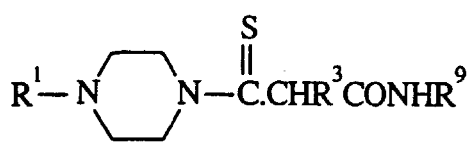

(XI)

to give a compound of formula (I) in which X is $-CONHR^9$
or

(m) converting a free base of formula (I) into a pharmaceutically acceptable salt thereof or

(n) resolving a racemic compound of formula (I) to give an optically active enantiomer.

**2.** A process as claimed in claim 1 in which, in the product, $R^1$ is a phenyl radical optionally substituted by one or more $C_{1-6}$ alkoxy, halogen, trifluoromethyl, nitro, carbalkoxy, carboxamido, cyano, amino, $(C_{1-6})$alkylamino or di $(C_{1-6})$alkylamino substituents.

**3.** A process as claimed in claim 1 or claim 2 in which, in the product, $R^3$ is a phenyl radical optionally substituted by one or more $C_{1-6}$ alkoxy, halogen, trifluoromethyl, nitro, carbalkoxy, carboxamido, cyano, amino,$(C_{1-6})$alkylamino or di$(C_{1-6})$alkylamino substituents.

4. A process as claimed in any one of claims 1 to 3 in which, in the product, X represents $-CO_2R^6$ or $-CONR^5R^9$ where $R^6$, $R^5$ and $R^9$ are as defined in claim 1.

5. A process as claimed in any one of claims l to 3 where, in the product, X is $-CONR^5R^9$ wherein $R^5$ and $R^9$ together with the nitrogen atom to which they are attached represent an azetidino, pyrrolidino, piperidino, hexahydroazepino, morpholino or piperazino ring.

6. A process as claimed in claim 1 in which the product is 1-{3-{1-[4-(2-methoxyphenyl)-piperazinyl]}-2-phenylpropionyl}pyrrolidine or a pharmaceutically acceptable acid addition salt thereof.

7. A process as claimed in claim 1 in which the product is 1-{3-{1-[4-(2-methoxyphenyl)-piperazinyl]}-2-phenylpropionyl}piperidine or a pharmaceutically acceptable acid addition salt thereof.

8. A process as claimed in claim 1 in which the product is

propyl 3-{1-[4-(2-methoxyphenyl)piperazinyl]}-2-phenylpropanoate,

3-{1-[4-(2-methoxyphenyl)piperazinyl]}-2-phenyl-N-phenylpropanamide,

3-{1-[4-(2-methoxyphenyl)piperazinyl]}-2-phenylpropanamide,

N-methyl-3-{1-[4-(2-methoxyphenyl)piperazinyl]}-2-phenylpropanamide,

N-ethyl-3-{-1-[4-(2-methoxyphenyl)piperazinyl]}-2-phenylpropanamide,

N-propyl-3-{1-[4-(2-methoxyphenyl)piperazinyl]}-2-phenylpropanamide,

N-butyl-3-{1-[4-(2-methoxyphenyl)piperazinyl]}-2-phenylpropanamide,

N-isobutyl-3-{1-[4-(2-methoxyphenyl)piperazinyl]}-2-phenylpropanamide,

N-cyclopropylmethyl-3-{1-[4-(2-methoxyphenyl)piperazinyl]}-2-phenylpropanamide,

N-neopentyl-3-{1-[4-(2-methoxyphenyl)piperazinyl]}-2-phenylpropanamide,

N-hexyl-3-{1-[4-(2-methoxyphenyl)piperazinyl]}-2-phenylpropanamide,

N-cyclohexylmethyl-3-{1-[4-(2-methoxyphenyl)piperazinyl]}-2-phenylpropanamide,

8-{α-{1-[4-(2-methoxyphenyl)piperazinyl]methyl}phenylacetamidoethyl-8-azaspiso[4.5]deca-7,9-dione,

N,N-dimethyl-3-{1-[4-(2-methoxphenyl)piperazinyl]}-2-phenylpropanamide,

3-{1-[4-(2-methoxyphenyl)piperazinyl]}-2-phenyl-N-(phenylmethyl)propanamide,

N-cyclohexyl-2-phenyl-3-{1-[4-(2-pyrimidinyl)-piperazinyl]}propanamide,

3,N-bis{1-[4-(2-methoxyphenyl)piperazinyl]}-2-phenylpropanamide,

2-methylpropyl 3-{1-[4-(2-methoxyphenyl)piperazinyl]}-2-phenylpropanoate,

ethyl 3-{1-[4-(2-methoxyphenyl)piperazinyl]}-2-phenylpropanoate,

2-{1-[4-(2-methoxyphenyl)piperazinyl]methyl}-N-methyl-3-phenylpropanamide,

ethyl 2-{1-[4-(2-methoxyphenyl)piperazinyl]methyl}-3-phenylpropanoate,

3-{1-[4-(2-methoxyphenyl)piperazinyl]}-N-(3,3-dimethyl)butyl-2-phenylpropionamide,

N-cyclopropyl-3-{-1-[4-(2-methoxyphenyl)piperazinyl]}2-phenylpropanamide,

1-{3-{1-[4-(2-methoxyphenyl)piperazinyl]}-2-phenylpropionyl}pyrrolidine,

N-cyclooctyl-3-{1-[4-(2-methoxyphenyl)piperazinyl]}-2-phenylpropionamide,

N-cyclododecyl-3-{1-[4-(2-methoxyphenyl)piperazinyl]}-2-phenylpropionamide,

(S)-N-[2-[1-[4-(2-methoxyphenyl)piperazinyl]]-1-phenylethyl]cyclohexanecarboxylic acid amide,

O-[2-[1-(4-(2-methoxyphenyl)piperazinyl)]-1-phenylethyl]-N-cyclohexylcarbamate,

O-[2-[1-[4-(2-methoxyphenyl)piperazinyl]]-1-phenylethyl]-N-phenylcarbamate,

2-{1-[4-(2-methoxyphenyl)piperazinyl]}-1-phenylethyl cyclohexanecarboxylate,

ethyl (S)-N-[2-[1-[4-(2-methoxyphenyl)piperazinyl]]-1-phenylethyl]carbamate,

methyl 3-[1-[4-(2-methoxyphenyl)piperazinyl]]-2-phenylpropionate,

N-(1-ethylpropyl)-3-[1-4-(2-methoxyphenyl)-piperazinyl]]-2-phenylpropionamide,

4-[3-[1-[4-(2-methoxyphenyl)piperazinyl]]-2-phenylpropionyl]morpholine,

1-{3-{1-[4-(2-methoxyphenyl)piperazinyl]}-2-phenylpropionyl}azetidine,

O-{2-[1-[4-(2-methoxyphenyl)piperazinyl]]-1-phenylethyl}-N-(3-chlorophenyl)carbamate,

1-ethyl-3-{2-[1-[4-(2-methoxyphenol)piperazinyl]]-1-phenylethyl}carbonate,

2,3-dihydrobenzo[1,4]dioxin-2-ylcarboxylic acid 2-[4-(2-methoxyphenyl)piperazin-1-yl]-1-phenylethyl ester

(S)-8-{2-[1-[4-(2-methoxyphenyl)piperazinyl]]-1-phenylethyl]}-8-azaspiro[4.5]decan-7,9-dione,

2,3,4,5,6,7-hexahydro-1-{2-[1-(4-(2-methoxyphenyl)piperazinyl)methyl]-3-phenylpropanoyl}-1H-azepine,

N-cycloheptyl-2-{1-[4-(2-methoxyphenyl)piperazinyl]methyl}-3-phenylpropanamide
or

N-cyclopropyl-2-{1-[4-(2-methoxyphenyl)piperazinyl]methyl}-3-phenylpropanamide

or a pharmaceutically acceptable acid addition salt thereof.

**9.** A process for preparing a pharmaceutical composition comprising bringing a compound of formula (I) as defined in claim 1 or a pharmaceutically acceptable acid addition salt thereof into association with a pharmaceutically acceptable carrier.

**10.** A process as claimed in claim 9 wherein the compound of formula (I) or the salt thereof is prepared by the process claimed in any one of claims 1 to 8.

**11.** A process for preparing an acid of formula

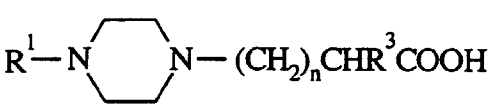

(where n, $R^1$, and $R^3$ are as defined in claim 1) which comprises

(a) reacting an acid of formula

$$CH_2 = CR^3COOH \qquad (VIII)$$

and a piperazine of formula

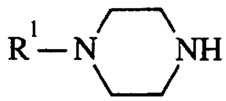

(IV)

by means of a Michael reaction
or

(b) hydrolysing a nitrile of formula

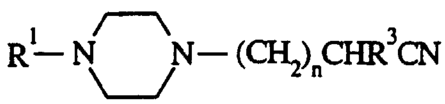

(IX)

**Claims for the following Contracting State : GR**

1. A process for preparing a compound of the formula (I)

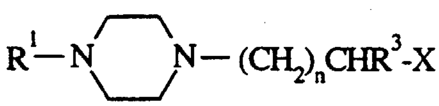

(I)

or a pharmaceutically acceptable acid addition salt thereof wherein

n is one of the integers 1 or 2,

$R^1$ is Ar or Het,

$R^3$ is Ar or an Ar($C_{1-6}$)alkyl radical,

X is $-OCOR^{10}$, $-CO_2R^6$, $-CONR^5R^9$, $-OCO_2R^6$, $-NR^4COR^6$, $OCONHR^{11}$, $-NHCO_2R^6$, $-NR^4CONHR^6$, $-CONHNHR^6$, $-CONHOR^6$,

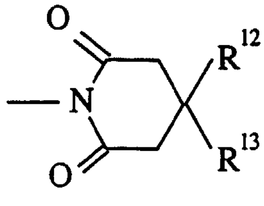 or 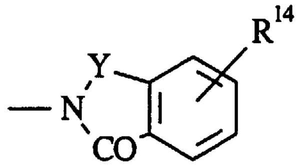

$R^4$ and $R^5$ are each hydrogen or $C_{1-6}$ alkyl

$R^6$ is $-CHR^7R^8$, cycloalkyl of 3 to 12 carbon atoms

or Ar($C_{1-6}$)alkyl (where $R^7$ and $R^8$ are each hydrogen or $C_{1-6}$ alkyl)

$R^9$ is hydrogen, an alkyl group of 1 to 8 carbon atoms other than a tertiary alkyl group, cycloalkyl of 3 to 12 carbon atoms, cycloalkyl($C_{1-6}$)alkyl, Ar($C_{1-6}$)alkyl or 8-azaspiro [4.5]deca-7,9-dione-8-yl-($C_{1-6}$)alkyl [with the proviso that when $R^9$ is hydrogen, alkyl or Ar($C_{1-6}$)alkyl, $R^5$ is other than a tertiary alkyl group],

or $R^5$ and $R^9$ together with the nitrogen atom to which they are attached represent an azetidino, pyrrolidino, piperidino, hexahydroazepino, morpholino or piperazino ring which may be optionally substituted by $C_{1-6}$ alkyl, Ar or Ar($C_{1-6}$)alkyl

$R^{10}$ is cycloalkyl of 3 to 12 carbon atoms, or 2,3-dihydro[1,4]benzodioxinyl optionally substituted by $C_{1-6}$ alkyl, $C_{1-6}$ alkoxy or halogen,

$R^{11}$ is cycloalkyl of 3 to 12 carbon atoms, Ar or Ar($C_{1-6}$)alkyl,

$R^{12}$ and $R^{13}$ are each $C_{1-6}$ alkyl or together with the carbon atom to which they are both attached represent $C_{4-6}$ cycloalkyl,

$R^{14}$ represents hydrogen, halogen, $C_{1-6}$ alkyl or $C_{1-6}$ alkoxy
and

Y is CO or $SO_2$

with the further provisos that

(a) when $R^1$ is phenyl optionally substituted by $CH_3$, $OCH_3$, Cl or $CF_3$, $R^3$ is phenyl optionally substituted by $C_{1-6}$ alkyl, methoxy or halogen and X is $-CONH_2$, $-CON(CH_3)_2$ or $-COOC_2H_5$ then n is 1

and (b) when $R^1$ is 2-pyrimidyl, $R^3$ is phenyl optionally substituted at one or two ring positions with $C_{1-6}$ alkyl, halogen, trifluoromethyl, cyano, nitro, ($C_{1-6}$)alkylamino, di($C_{1-6}$)alkylamino, $C_{1-6}$-alkyloxy and X is

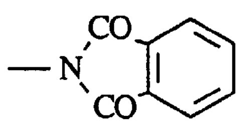

or $-NR^4COR^6$ where $R^4$ is hydrogen or $C_{1-6}$ alkyl and $R^6$ is $-CHR^7R^8$ (where $R^7$ and $R^8$ are each hydrogen or $C_{1-6}$ alkyl and $-CHR^7R^8$ contains a total of 1 to 6 carbon atoms) then n is 2;

and wherein the term "Ar" means an aromatic radical having 6 to 12 carbon atoms which optionally may be substituted by one or more $C_{1-6}$ alkoxy, halogen, trifluoromethyl, nitro, carbalkoxy, carboxamido, cyano, amino, ($C_{1-6}$) alkylamino or di($C_{1-6}$)alkylamino substituents and the term "Het" means an aromatic radical containing one or more nitrogen atoms as heteroatoms and which may be optionally substituted by one or more $C_{1-6}$ alkyl, $C_{1-6}$ alkoxy, halogen, trifluoromethyl, amino, ($C_{1-6}$)alkylamino or di($C_{1-6}$)alkylamino substituents;
which process comprises

(a) acylating an amine of formula

$$NHR^5R^9 \qquad (II)$$

(where $R^5$ and $R^9$ are as defined above) with an acid of formula

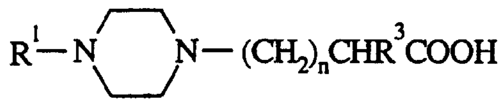

(III)

(where $R^1$ and $R^3$ are as defined above) or with an acylating derivative thereof to give a compound of formula (I) where X represents $-CONR^5R^9$
or

(b) acylating a piperazine alkylamine of formula

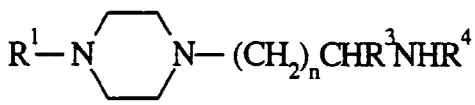

with an acid of formula

$R^6COOH$

or with an acylating derivative thereof to give a compound of formula (I) in which X is $-NR^4COR^6$
or

(c) reacting an amine of formula

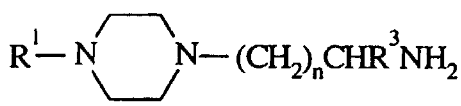

with an anhydride of formula

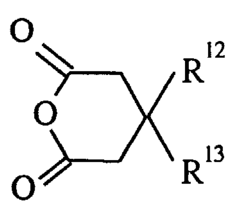

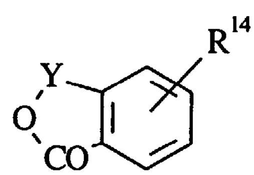

or with an acid of formula

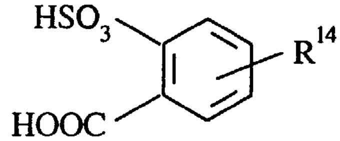

to give a compound of formula I in which X is

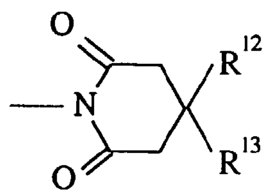

or

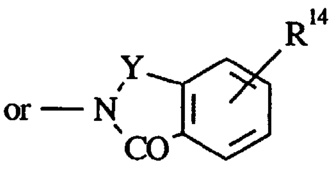

or

(d) esterifying an acid of formula (III), as given above with an alcohol of formula $R^6$ OH to give a compound of formula (I) in which X is $-CO_2R^6$
or

(e) esterifying a piperazine alcohol of formula

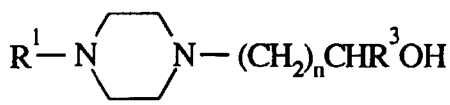

with an acid of formula $R^{10}$ COOH to give a compound of formula (I) in which X is $-OCOR^{10}$
or

(f) reacting an isocyanate with a piperazinyl-alkanol of formula

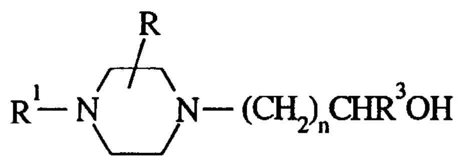

or with a piperazinyl-alkylamine of formula

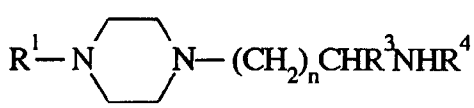

to give a compound of formula (I) in which X is $-NR^4CONHR^6$ or $-O.CO.NHR^{11}$
or

(g) reacting a piperazine isocyanate derivative of formula

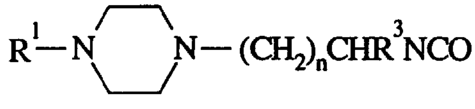

with an alcohol of formula

$R^6OH$

to give a compound of formula (I) in which X is $-NHCO_2R^6$
or

(h) reacting an amine of formula

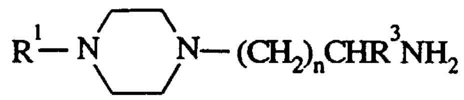

with a compound of formula $R^6OCOHal$ (where Hal is halogen) to give a compound of formula (I) in which X is $-NHCO_2R^6$
or

(i) reacting an acid of formula (III) given above with a hydroxylamine of formula $NH_2\ OR^6$ or with a hydrazide of formula $NH_2\ NHR^6$ to give a compound of formula I in which X is respectively $-CONHOR^6$ or $-CONHNHR^6$
or

(j) alkylating a piperazine of formula

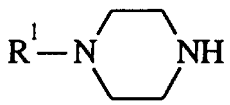

(IV)

with an alkylating agent providing the group $-(CH_2)_n\ CHR^3X$
or

(k) reacting a nitrile of formula (IX)

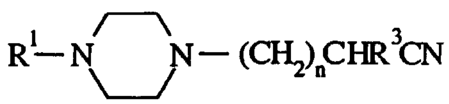

with a secondary alcohol to give a compound of formula (I) in which X is $-CONR^5R^9$ where $R^5$ is hydrogen and $R^9$ is a secondary (lower)alkyl group or subjecting the nitrile to acid hydrolysis to give a compound of formula (I) in which X is -CONH2
or

(l) desulphurising a sulphur containing compound of formula

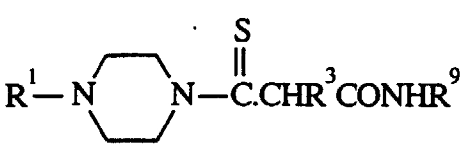

(XI)

to give a compound of formula (I) in which X is $-CONHR^9$
or

(m) converting a free base of formula (I) into a pharmaceutically acceptable salt thereof or

(n) resolving a racemic compound of formula (I) to give an optically active enantiomer.

**2.** A process as claimed in claim 1 in which, in the product, $R^1$ is a phenyl radical optionally substituted by one or more $C_{1-6}$ alkoxy, halogen, trifluoromethyl, nitro, carbalkoxy, carboxamido, cyano, amino, $(C_{1-6})$alkylamino or di

($C_{1-6}$)alkylamino substituents.

**3.** A process as claimed in claim 1 or claim 2 in which, in the product, $R^3$ is a phenyl radical optionally substituted by one or more $C_{1-6}$ alkoxy, halogen, trifluoromethyl, nitro, carbalkoxy, carboxamido, cyano, amino,($C_{1-6}$)alkylamino or di($C_{1-6}$)alkylamino substituents.

**4.** A process as claimed in any one of claims 1 to 3 in which, in the product, X represents $-CO_2R^6$ or $-CONR^5R^9$ where $R^6$, R5 and $R^9$ are as defined in claim 1.

**5.** A process as claimed in any one of claims 1 to 3 where, in the product, X is $-CONR^5R^9$ wherein $R^5$ and $R^9$ together with the nitrogen atom to which they are attached represent an azetidino, pyrrolidino, piperidino, hexahydroazepino, morpholino or piperazino ring.

**6.** A process as claimed in claim 1 in which the product is 1-{3-{1-[4-(2-methoxyphenyl)-piperazinyl]}-2-phenylpropionyl}pyrrolidine or a pharmaceutically acceptable acid addition salt thereof.

**7.** A process as claimed in claim 1 in which the product is 1-{3-{1-[4-(2-methoxyphenyl)-piperazinyl]}-2-phenylpropionyl}piperidine or a pharmaceutically acceptable acid addition salt thereof.

**8.** A process as claimed in claim 1 in which the product is

propyl 3-{1-[4-(2-methoxyphenyl)piperazinyl]}-2-phenylpropanoate,

3-{1-[4-(2-methoxyphenyl)piperazinyl]}-2-phenyl-N-phenylpropanamide,

3-{1-[4-(2-methoxyphenyl)piperazinyl]}-2-phenylpropanamide,

N-methyl-3-{1-[4-(2-methoxyphenyl)piperazinyl]}-2-phenylpropanamide,

N-ethyl-3-{1-[4-(2-methoxyphenyl)piperazinyl]}-2-phenylpropanamide,

N-propyl-3-{1-[4-(2-methoxyphenyl)piperazinyl]}-2-phenylpropanamide,

N-butyl-3-{1-[4-(2-methoxyphenyl)piperazinyl]}-2-phenylpropanamide,

N-isobutyl-3-{1-[4-(2-methoxyphenyl)piperazinyl]}-2-phenylpropanamide,

N-cyclopropylmethyl-3-{1-[4-(2-methoxyphenyl)piperazinyl]}-2-phenylpropanamide,

N-neopentyl-3-{1-[4-(2-methoxyphenyl)piperazinyl]}-2-phenylpropanamide,

N-hexyl-3-{1-[4-(2-methoxyphenyl)piperazinyl]}-2-phenylpropanamide,

N-cyclohexylmethyl-3-{1-[4-(2-methoxyphenyl)piperazinyl]}-2-phenylpropanamide,

8-{α-{1-[4-(2-methoxyphenyl)piperazinyl]methyl}phenylacetamidoethyl-8-azaspiso[4.5]deca-7,9-dione,

N,N-dimethyl-3-{1-[4-(2-methoxphenyl)piperazinyl]}-2-phenylpropanamide,

3-{1-[4-(2-methoxyphenyl)piperazinyl]}-2-phenyl-N-(phenylmethyl)propanamide,

N-cyclohexyl-2-phenyl-3-{1-[4-(2-pyrimidinyl)-piperazinyl]}propanamide,

3,N-bis{1-[4-(2-methoxyphenyl)piperazinyl]}-2-phenylpropanamide,

2-methylpropyl 3-{1-[4-(2-methoxyphenyl)piperazinyl]}-2-phenylpropanoate,

ethyl 3-{1-[4-(2-methoxyphenyl)piperazinyl]}-2-phenylpropanoate,

2-{1-[4-(2-methoxyphenyl)piperazinyl]methyl}-N-methyl-3-phenylpropanamide,

ethyl 2-{1-[4-(2-methoxyphenyl)piperazinyl]methyl}-3-phenylpropanoate,

3-{1-[4-(2-methoxyphenyl)piperazinyl]}-N-(3,3-dimethyl)butyl-2-phenylpropionamide,

N-cyclopropyl-3-{1-[4-(2-methoxyphenyl)piperazinyl]}2-phenylpropanamide,

1-{3-{1-[4-(2-methoxyphenyl)piperazinyl]}-2-phenylpropionyl}pyrrolidine,

N-cyclooctyl-3-{1-[4-(2-methoxyphenyl)piperazinyl]}-2-phenylpropionamide,

N-cyclododecyl-3-{1-[4-(2-methoxyphenyl)piperazinyl]}-2-phenylpropionamide,

(S)-N-[2-[1-[4-(2-methoxyphenyl)piperazinyl]]-1-phenylethyl]cyclohexanecarboxylic acid amide,

O-[2-[1-(4-(2-methoxyphenyl)piperazinyl)]-1-phenylethyl]-N-cyclohexylcarbamate,

O-[2-[1-[4(2-methoxyphenyl)piperazinyl]]-1-phenylethyl]-N-phenylcarbamate,

2-{1-[4-(2-methoxyphenyl)piperazinyl]}-1-phenylethyl cyclohexanecarboxylate,

ethyl (S)-N-[2-[1-[4-(2-methoxyphenyl)piperazinyl]]-1-phenylethyl]carbamate,

methyl 3-[1-[4-(2-methoxyphenyl)piperazinyl]]-2-phenylpropionate,

N-(1-ethylpropyl)-3-[1-4-(2-methoxyphenyl)-piperazinyl]]-2-phenylpropionamide,

4-[3-[1-[4-(2-methoxyphenyl)piperazinyl]]-2-phenylpropionyl]morpholine,

1-{3-{1-[4-(2-methoxyphenyl)piperazinyl]}-2-phenylpropionyl}azetidine,

O-{2-[1-[4-(2-methoxyphenyl)piperazinyl]]-1-phenylethyl}-N-(3-chlorophenyl)carbamate,

1-ethyl-3-{2-[1-[4-(2-methoxyphenol)piperazinyl]]-1-phenylethyl}carbonate,

2,3-dihydrobenzo[1,4]dioxin-2-ylcarboxylic acid 2-[4-(2-methoxyphenyl)piperazin-1-yl]-1-phenylethyl ester

(S)-8-{2-[1-[4-(2-methoxyphenyl)piperazinyl]]-1-phenylethyl]}-8-azaspiro[4.5]decan-7,9-dione,

2,3,4,5,6,7-hexahydro-1-{2-[1-(4-(2-methoxyphenyl)piperazinyl)methyl]-3-phenylpropanoyl)-1H-azepine,

N-cycloheptyl-2-{1-[4-(2-methoxyphenyl)piperazinyl]methyl}-3-phenylpropanamide
or

N-cyclopropyl-2-{1-[4-(2-methoxyphenyl)piperazinyl]methyl}-3-phenylpropanamide

or a pharmaceutically acceptable acid addition salt thereof.

**9.** A process for preparing a pharmaceutical composition comprising bringing a compound of formula (I) as defined in claim 1 or a pharmaceutically acceptable acid addition salt thereof into association with a pharmaceutically acceptable carrier.

**10.** A process as claimed in claim 9 wherein the compound of formula (I) or the salt thereof is prepared by the process claimed in any one of claims 1 to 8.

**11.** A process for preparing an acid of formula

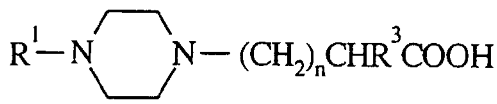

(where n, $R^1$, and $R^3$ are as defined in claim 1) which comprises

(a) reacting an acid of formula

$$CH_2 = CR^3COOH \qquad (VIII)$$

and a piperazine of formula

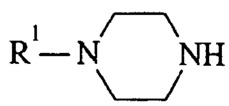

(IV)

by means of a Michael reaction
or

(b) hydrolysing a nitrile of formula

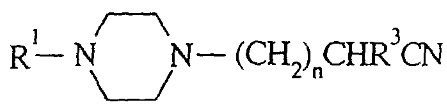

(IX)

**12.** An acid of formula

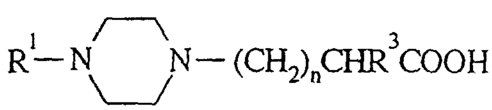

(where n, $R^1$ and $R^3$ are as defined in claim 1).

**Patentansprüche**

**Patentansprüche für folgende Vertragsstaaten : AT, BE, CH, DE, DK, FR, IT, LI, LU, NL, SE**

**1.** Verbindung der allgemeinen Formel (I)

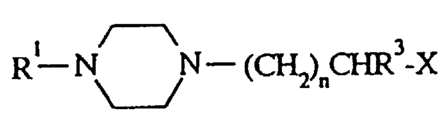

**(I)**

oder ein pharmazeutisch annehmbares Säureadditionssalz davon, worin n eine der ganzen Zahlen 1 oder 2 ist, $R^1$ Ar oder Het ist, $R^3$ Ar oder ein Ar($C_{1-6}$)-alkylrest ist, X $-OCOR^{10}$, $-CO_2R^6$, $-CONR^5R^9$, $-OCO_2R^6$, $-NR^4COR^6$, $OCONHR^{11}$, $-NHCO_2R^6$, $-NR^4CONHR^6$, $-CONHNHR^6$, $-CONHOR^6$,

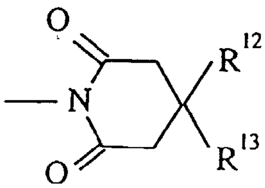

oder

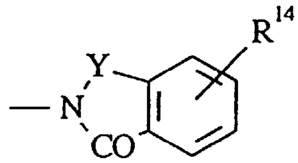

ist, $R^4$ und $R^5$ jeweils Wasserstoff oder $C_{1-6}$-Alkyl sind, $R^6$ $-CHR^7R^8$, Cycloalkyl mit 3 bis 12 Kohlenstoffatomen oder Ar($C_{1-6}$)-alkyl ist (worin $R^7$ und $R^8$ jeweils Wasserstoff oder $C_{1-6}$-Alkyl sind), $R^9$ Wasserstoff, eine Alkylgruppe mit 1 bis 8 Kohlenstoffatomen, die keine tertiäre Alkylgruppe ist, Cycloalkyl mit 3 bis 12 Kohlenstoffatomen, Cycloalkyl($C_{1-6}$)-alkyl, Ar($C_{1-6}$)-alkyl oder 8-Azaspiro[4.5]deca-7,9-dion-8-yl-($C_{1-6}$)-alkyl ist [mit der Maßgabe, dass, wenn $R^9$ Wasserstoff, Alkyl oder Ar($C_{1-6}$)-alkyl ist, $R^5$ keine tertiäre Alkylgruppe ist], oder $R^5$ und $R^9$ zusammen mit dem Stickstoffatom, an das sie gebunden sind, einen Azetidino-, Pyrrolidino-, Piperidino-, Hexahydroazepino-, Morpholino- oder Piperazinoring darstellen, der gegebenenfalls mit $C_{1-6}$-Alkyl, Ar oder Ar($C_{1-6}$)-alkyl substituiert sein kann, $R^{10}$ Cycloalkyl mit 3 bis 12 Kohlenstoffatomen oder 2,3-Dihydro[1,4]benzodioxinyl ist, gegebenenfalls substituiert mit $C_{1-6}$-Alkyl, $C_{1-6}$-Alkoxy oder Halogen, $R^{11}$ Cycloalkyl mit 3 bis 12 Kohlenstoffatomen, Ar oder Ar ($C_{1-6}$)-alkyl ist, $R^{12}$ und $R^{13}$ jeweils $C_{1-6}$-Alkyl sind oder zusammen mit dem Kohlenstoffatom, an das sie beide gebunden sind, $C_{4-6}$-Cycloalkyl darstellen, $R^{14}$ Wasserstoff, Halogen, $C_{1-6}$-Alkyl oder $C_{1-6}$-Alkoxy darstellt und Y CO oder $SO_2$ ist, mit den weiteren Maßgaben, dass

(a) wenn $R^1$ Phenyl ist, gegebenenfalls substituiert mit $CH_3$, $OCH_3$, Cl oder $CF_3$, $R^3$ Phenyl ist, gegebenenfalls substituiert mit $C_{1-6}$-Alkyl, Methoxy oder Halogen, und X $-CONH_2$, $-CON(CH_3)_2$ oder $-COOC_2H_5$ ist, dann n 1 ist, und

(b) wenn $R^1$ 2-Pyrimidyl ist, $R^3$ Phenyl ist, gegebenenfalls an einer oder zwei Ringpositionen substituiert mit $C_{1-6}$-Alkyl, Halogen, Trifluormethyl, Cyano, Nitro, ($C_{1-6}$)-Alkylamino, Di-($C_{1-6}$)-alkylamino, $C_{1-6}$-Alkyloxy, und X

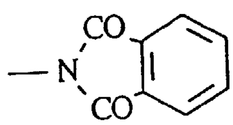

oder $-NR^4COR^6$ ist, worin $R^4$ Wasserstoff oder $C_{1-6}$-Alkyl ist und $R^6$ $-CHR^7R^8$ ist (worin $R^7$ und $R^8$ jeweils Wasserstoff oder $C_{1-6}$-Alkyl sind und $-CHR^7R^8$ insgesamt 1 bis 6 Kohlenstoffatome enthält), dann n 2 ist;

und worin der Ausdruck "Ar" einen aromatischen Rest bezeichnet, der 6 bis 12 Kohlenstoffatome aufweist, der gegebenenfalls mit einem oder mehreren $C_{1-6}$-Alkoxy-, Halogen-, Trifluormethyl-, Nitro-, Carbalkoxy-, Carboxamido-, Cyano-, Amino-, ($C_{1-6}$)-Alkylamino- oder Di-($C_{1-6}$)-alkylamino-Substituenten substituiert sein kann, und der Ausdruck "Het" einen aromatischen Rest bezeichnet, der ein oder mehrere Stickstoffatome als Heteroatome enthält und der gegebenenfalls mit einem oder mehreren $C_{1-6}$-Alkyl-, $C_{1-6}$-Alkoxy-, Halogen-, Trifluormethyl-, Amino-, ($C_{1-6}$)-Alkylamino- oder Di($C_{1-6}$)-alkylamino-Substituenten substituiert sein kann.

2. Verbindung gemäß Anspruch 1, worin $R^1$ ein Phenylrest ist, gegebenenfalls substituiert mit einem oder mehreren $C_{1-6}$-Alkoxy-, Halogen-, Trifluormethyl-, Nitro-, Carbalkoxy-, Carboxamido-, Cyano-, Amino-, ($C_{1-6}$)-Alkylamino oder Di($C_{1-6}$)-alkylamino-Substituenten.

3. Verbindung gemäß Anspruch 1 oder 2, worin $R^3$ ein Phenylrest ist, gegebenenfalls substituiert mit einem oder mehreren $C_{1-6}$-Alkoxy-, Halogen-, Trifluormethyl-, Nitro-, Carbalkoxy-, Carboxamido-, Cyano-, Amino-, ($C_{1-6}$)-Alkylamino- oder Di($C_{1-6}$)-alkylamino-Substituenten.

4. Verbindung gemäß einem der Ansprüche 1 bis 3, worin X $-CO_2R^6$ oder $-CONR^5R^9$ darstellt, worin $R^6$, $R^5$ und $R^9$ wie in Anspruch 1 definiert sind.

5. Verbindung gemäß einem der Ansprüche 1 bis 3, worin X $-CONR^5R^9$ ist, worin $R^5$ und $R^9$ zusammen mit dem Stickstoffatom, an das sie gebunden sind, einen Azetidino-, Pyrrolidino-, Piperidino-, Hexahydroazepino-, Morpholino- oder Piperazinoring darstellen.

6. Verbindung gemäß Anspruch 1, die 1-{3-{1-[4-(2-Methoxyphenyl)-piperazinyl]}-2-phenylpropionyl}pyrrolidin oder ein pharmazeutisch annehmbares Säureadditionssalz davon ist.

7. Verbindung gemäß Anspruch 1, die 1-{3-{1-[4-(2-Methoxyphenyl)piperazinyl]}-2-phenylpropionyl}piperidin oder ein pharmazeutisch annehmbares Säureadditionssalz davon ist.

8. Verbindung gemäß Anspruch 1, welche ist:

Propyl-3-{1-[4-(2-methoxyphenyl)piperazinyl]}-2-phenylpropanoat,
3-{1-[4-(2-Methoxyphenyl)piperazinyl]}-2-phenyl-N-phenylpropanamid,
3-{1-[4-(2-Methoxyphenyl)piperazinyl]}-2-phenylpropanamid, N-Methyl-3-{1-[4-(2-methoxyphenyl)piperazinyl]}-2-phenylpropanamid,
N-Ethyl-3-{1-[4-(2-methoxyphenyl)piperazinyl]}-2-phenylpropanamid,
N-Propyl-3-{1-[4-(2-methoxyphenyl)piperazinyl]}-2-phenylpropanamid,
N-Butyl-3-{1-[4-(2-methoxyphenyl)piperazinyl]}-2-phenylpropanamid,
N-Isobutyl-3-{1-[4-(2-methoxyphenyl)piperazinyl]}-2-phenylpropanamid,
N-Cyclopropylmethyl-3-{1-[4-(2-methoxyphenyl)piperazinyl]}-2-phenylpropanamid,
N-Neopentyl-3-{1-[4-(2-methoxyphenyl)piperazinyl]}-2-phenyl-propanamid,
N-Hexyl-3-{1-[4-(2-methoxyphenyl)piperazinyl]}-2-phenylpropanamid,
N-Cyclohexylmethyl-3-{1-[4-(2-methoxyphenyl)piperazinyl]}-2-phenylpropanamid,
8-{alpha-{1-[4-(2-Methoxyphenyl)piperazinyl]methyl}phenylacetamidoethyl-8-azaspiro[4.5]deca-7,9-dion,
N,N-Dimethyl-3-{1-[4-(2-methoxphenyl)piperazinyl]}-2-phenylpropanamid,
3-{1-[4-(2-Methoxyphenyl)piperazinyl]}-2-phenyl-N-(phenylmethyl)propanamid,
N-Cyclohexyl-2-phenyl-3-{1-[4-(2-pyrimidinyl)-piperazinyl]}propanamid,
3,N-bis{1-[4-(2-Methoxyphenyl)piperazinyl]}-2-phenylpropanamid,
2-Methylpropyl-3-{1-[4-(2-methoxyphenyl)piperazinyl]}-2-phenylpropanoat,
Ethyl-3-{1-[4-(2-methoxyphenyl)piperazinyl]}-2-phenylpropanoat, 2-{1-[4-(2-Methoxyphenyl)piperazinyl]methyl}-N-methyl-3-phenylpropanamid,
Ethyl-2-{1-[4-(2-methoxyphenyl)piperazinyl]methyl}-3-phenylpropanoat,
3-{1-[4-(2-Methoxyphenyl)piperazinyl]}-N-(3,3-dimethyl)butyl-2-phenylpropionamid,
N-Cyclopropyl-3-{1-[4-(2-methoxyphenyl)piperazinyl]}-2-phenylpropanamid,
1-{3-{1-[4-(2-Methoxyphenyl)piperazinyl]}-2-phenylpropionyl}pyrrolidin,
N-Cyclooctyl-3-{1-[4-(2-methoxyphenyl)piperazinyl]}-2-phenylpropionamid,
N-Cyclododecyl-3-{1-[4-(2-methoxyphenyl)piperazinyl]}-2-phenyl-propionamid,
(S)-N-[2-[1-[4-(2-Methoxyphenyl)piperazinyl]]-1-phenylethyl]cyclohexancarbonsäureamid,
O-[2-[1-(4-(2-Methoxyphenyl)piperazinyl)]-1-phenylethyl]-N-cyclohexylcarbamat,
O-[2-[1-[4-(2-Methoxyphenyl)piperazinyl]]-1-phenylethyl]-N-phenylcarbamat,
2-{1-[4-(2-Methoxyphenyl)piperazinyl]}-1-phenylethylcyclohexancarboxylat,
Ethyl-(S)-N-[2-[1-[4-(2-Methoxyphenyl)piperazinyl]]-1-phenylethyl]carbamat,
Methyl-3-[1-[4-(2-methoxyphenyl)piperazinyl]]-2-phenylpropionat, N-(1-Ethylpropyl)-3-[1-4-(2-methoxyphenyl)piperazinyl]]-2-phenylpropionamid,
4-[3-[1-[4-(2-Methoxyphenyl)piperazinyl]]-2-phenylpropionyl]-morpholin,
1-{3-{1-[4-(2-Methoxyphenyl)piperazinyl]}-2-phenylpropionyl}-azetidin,
O-{2-[1-[4-(2-Methoxyphenyl)piperazinyl]]-1-phenylethyl}-N-(3-chlorphenyl)carbamat,
1-Ethyl-3-{2-[1-[4-(2-methoxyphenol)piperazinyl]]-1-phenylethyl}carbonat,
2,3-Dihydrobenzo[1,4]dioxin-2-ylcarbonsäure-2-[4-(2-methoxyphenyl)piperazin-1-yl]-1-phenylethylester (S)-8-{2-[1-[4-(2-Methoxyphenyl)piperazinyl]]-1-phenylethyl]}-8-azaspiro[4.5]decan-7,9-dion,
2,3,4,5,6,7-Hexahydro-1-{2-[1-(4-(2-methoxyphenyl)piperazinyl)-methyl]-3-phenylpropanoyl}-1H-azepin,
N-Cycloheptyl-2-{1-[4-(2-methoxyphenyl)piperazinyl]methyl}-3-phenylpropanamid
oder
N-Cyclopropyl-2-{1-[4-(2-methoxyphenyl)piperazinyl]methyl}-3-phenylpropanamid

oder ein pharmazeutisch annehmbares Säureadditionssalz davon.

**9.** Verfahren zur Herstellung einer Verbindung gemäß Anspruch 1, worin X -$CONR^5R^9$ darstellt, welches das Acylieren eines Amins der Formel

$$NHR^5R^9 \quad (II)$$

(worin $R^5$ und $R^9$ wie in Anspruch 1 definiert sind) mit einer Säure der Formel

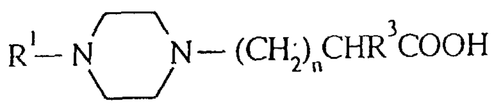

(III)

(worin $R^1$ und $R^3$ wie in Anspruch 1 definiert sind) oder mit einem Acylierungsderivat davon umfaßt.

**10.** Verfahren zur Herstellung einer Verbindung gemäß Anspruch 1, worin X -$NR^4COR^6$ ist, welches das Acylieren eines Piperazinalkylamins der Formel

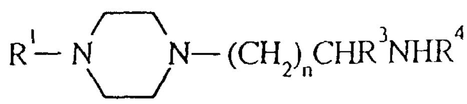

mit einer Säure der Formel

$$R^6COOH$$

oder mit einem Acylierungsderivat davon umfaßt.

**11.** Verfahren zur Herstellung einer Verbindung gemäß Anspruch 1, worin X

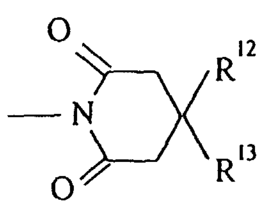

**oder**

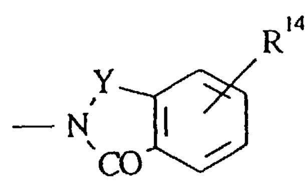

ist, welches das Umsetzen eines Amins der Formel

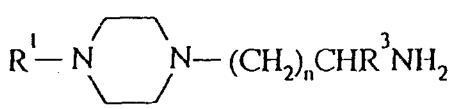

mit einem Anhydrid der Formel

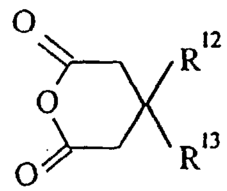

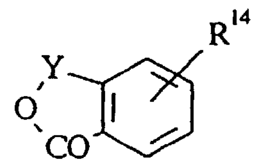

oder, zur Herstellung der Verbindung, worin Y $SO_2$ ist, das Umsetzen des Amins mit einer Säure der Formel

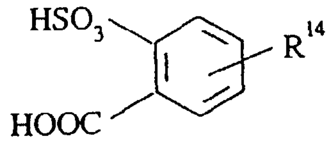

umfaßt.

**12.** Verfahren zur Herstellung einer Verbindung gemäß Anspruch 1, worin X $-CO_2R^6$ ist, welches die Veresterung einer Säure der Formel (III), wie oben in Anspruch 9 angegeben ist, mit einem Alkohol der Formel $R^6OH$ umfaßt.

**13.** Verfahren zur Herstellung einer Verbindung gemäß Anspruch 1, worin X $-OCOR^{10}$ ist, welches das Verestern eines Piperazinalkohols der Formel

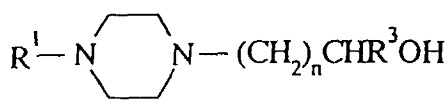

mit einer Säure der Formel $R^{10}COOH$ umfaßt.

**14.** Verfahren zur Herstellung einer Verbindung gemäß Anspruch 1, worin X $-NR^4CONHR^6$ oder $-O.CO.NHR^{11}$ ist, welches das Umsetzen eines Isocyanates mit einem Piperazinyl-Alkanol der Formel

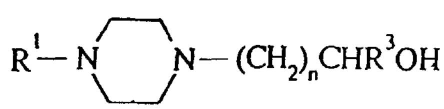

oder mit einem Piperazinyl-Alkylamin der Formel

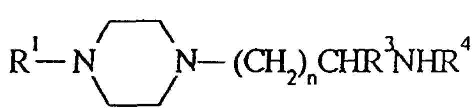

umfaßt.

**15.** Verfahren zur Herstellung einer Verbindung gemäß Anspruch 1, worin X $-NHCO_2R^6$ ist, welches das Umsetzen eines Piperazinisocyanat-Derivates der Formel

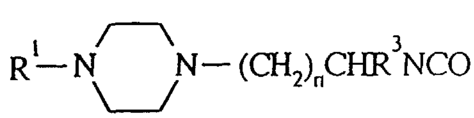

mit einem Alkohol der Formel

$$R^6OH$$

umfaßt.

**16.** Verfahren zur Herstellung einer Verbindung gemäß Anspruch 1, worin X $-NHCO_2R^6$ ist, welches das Umsetzen eines Amins der Formel

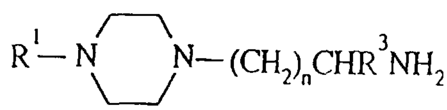

mit einer Verbindung der Formel $R^6OCOHal$ (worin Hal Halogen ist) umfaßt.

**17.** Verfahren zur Herstellung einer Verbindung gemäß Anspruch 1, worin X $-CONHOR^6$ ist, welches das Umsetzen einer Säure der Formel (III), die in Anspruch 9 angegeben ist, mit einem Hydroxylamin der Formel $NH_2OR^6$ umfaßt.

**18.** Verfahren zur Herstellung einer Verbindung gemäß Anspruch 1, welches das Alkylieren eines Piperazins der Formel

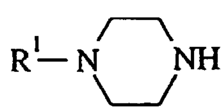

**(IV)**

mit einem die Gruppe $-(CH_2)_nCHR^3X$ liefernden Alkylierungsmittel umfaßt.

**19.** Verfahren zur Herstellung einer Verbindung gemäß Anspruch 1, worin X $-CONHR^9$ ist, worin $R^9$ eine sekundäre $(C_{1-6})$-Alkylgruppe ist oder X $-CONH_2$ ist, welches das Umsetzen eines Nitrils der Formel (IX)

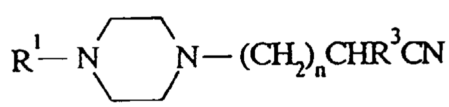

mit einem sekundären Alkohol umfaßt, um eine Verbindung der Formel (I) zu ergeben, worin X $-CONR^5R^9$ ist, worin $R^5$ Wasserstoff ist und $R^9$ eine sekundäre $(C_{1-6})$-Alkylgruppe ist, oder das Unterwerfen des Nitrils einer sauren Hydrolyse, um eine Verbindung der Formel (I) zu ergeben, worin X $-CONH_2$ ist.

**20.** Verfahren zur Herstellung einer Verbindung gemäß Anspruch 1, worin X $-CONHR^9$ ist, welches das Entschwefeln einer schwefelhältigen Verbindung der Formel

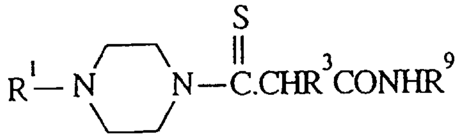

(XI)

umfaßt.

**21.** Verfahren zur Herstellung eines pharmazeutisch annehmbaren Salzes einer Verbindung gemäß Anspruch 1, welches das Umwandeln einer freien Base der Formel (I) in das Salz umfaßt.

**22.** Verfahren, welches das Trennen einer racemischen Verbindung der Formel (I) gemäß Anspruch 1 in ein optisch aktives Enantiomer umfaßt.

**23.** Pharmazeutische Zusammensetzung, umfassend eine Verbindung gemäß einem der Ansprüche 1 bis 8 in Assoziation mit einem pharmazeutisch annehmbaren Träger.

**24.** Verbindung gemäß einem der Ansprüche 1 bis 8 zur Verwendung als Arzneimittel.

**25.** Säure der Formel

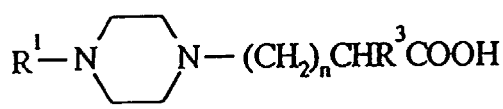

(worin n, $R^1$ und $R^3$ wie in Anspruch 1 definiert sind).

**26.** Verfahren zur Herstellung einer Verbindung gemäß Anspruch 1, worin X -CONHNHR$^6$ ist, welches das Umsetzen einer Säure der Formel (III), wie in Anspruch 9 definiert ist, mit einem Hydrazin der Formel $NH_2NHR^6$ umfaßt.

**Patentansprüche für folgenden Vertragsstaat : ES**

**1.** Verfahren zur Herstellung einer Verbindung der Formel (I)

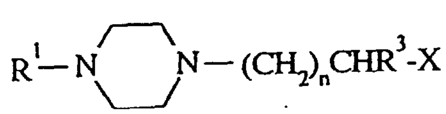

(I)

oder eines pharmazeutisch annehmbaren Säureadditionssalzes davon, worin n eine der ganzen Zahlen 1 oder 2 ist, $R^1$ Ar oder Het ist, $R^3$ Ar oder ein Ar($C_{1-6}$)-alkylrest ist, X -OCOR$^{10}$, -$CO_2R^6$, -CONR$^5$R$^9$, -$OCO_2R^6$, -NR$^4$COR$^6$, OCONHR$^{11}$, -$NHCO_2R^6$,-NR$^4$CONHR$^6$, -CONHNHR$^6$, -CONHOR$^6$,

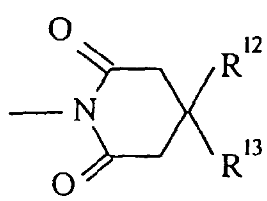

oder

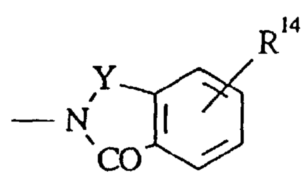

ist, $R^4$ und $R^5$ jeweils Wasserstoff oder $C_{1-6}$-Alkyl sind, $R^6$ -CHR$^7$R$^8$, Cycloalkyl mit 3 bis 12 Kohlenstoffatomen oder Ar($C_{1-6}$)-alkyl ist (worin $R^7$ und $R^8$ jeweils Wasserstoff oder $C_{1-6}$-Alkyl sind), $R^9$ Wasserstoff, eine Alkylgruppe mit 1 bis 8 Kohlenstoffatomen, die keine tertiäre Alkylgruppe ist, Cycloalkyl mit 3 bis 12 Kohlenstoffatomen, Cycloalkyl($C_{1-6}$)-alkyl, Ar($C_{1-6}$)-alkyl oder 8-Azaspiro[4.5]deca-7,9-dion-8-yl-($C_{1-6}$)-alkyl ist [mit der Maßgabe, dass, wenn $R^9$ Wasserstoff, Alkyl oder Ar($C_{1-6}$)-alkyl ist, $R^5$ keine tertiäre Alkylgruppe ist], oder $R^5$ und $R^9$ zusammen mit dem Stickstoffatom, an das sie gebunden sind, einen Azetidino-, Pyrrolidino-, Piperidino-, Hexahydroazepino-, Morpholino- oder Piperazinoring darstellen, der gegebenenfalls mit $C_{1-6}$-Alkyl, Ar oder Ar($C_{1-6}$)-alkyl substituiert sein kann, $R^{10}$ Cycloalkyl mit 3 bis 12 Kohlenstoffatomen oder 2,3-Dihydro[1,4]benzodioxinyl ist, gegebenenfalls substituiert mit $C_{1-6}$-Alkyl, $C_{1-6}$-Alkoxy oder Halogen, $R^{11}$ Cycloalkyl mit 3 bis 12 Kohlenstoffatomen, Ar oder Ar ($C_{1-6}$)-alkyl ist, $R^{12}$ und $R^{13}$ jeweils $C_{1-6}$-Alkyl sind oder zusammen mit dem Kohlenstoffatom, an das sie beide gebunden sind, $C_{4-6}$-Cycloalkyl darstellen, $R^{14}$ Wasserstoff, Halogen, $C_{1-6}$-Alkyl oder $C_{1-6}$-Alkoxy darstellt und Y

CO oder $SO_2$ ist, mit den weiteren Maßgaben, dass

(a) wenn $R^1$ Phenyl ist, gegebenenfalls substituiert mit $CH_3$, $OCH_3$, Cl oder $CF_3$, $R^3$ Phenyl ist, gegebenenfalls substituiert mit $C_{1-6}$-Alkyl, Methoxy oder Halogen, und X $-CONH_2$, $-CON(CH_3)_2$ oder $-COOC_2H_5$ ist, dann n 1 ist, und
(b) wenn $R^1$ 2-Pyrimidyl ist, $R^3$ Phenyl ist, gegebenenfalls an einer oder zwei Ringpositionen substituiert mit $C_{1-6}$-Alkyl, Halogen, Trifluormethyl, Cyano, Nitro, $(C_{1-6})$-Alkylamino, Di-$(C_{1-6})$-alkylamino, $C_{1-6}$-Alkyloxy, und X

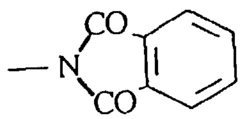

oder $-NR^4COR^6$ ist, worin $R^4$ Wasserstoff oder $C_{1-6}$-Alkyl ist und $R^6$ $-CHR^7R^8$ ist (worin $R^7$ und $R^8$ jeweils Wasserstoff oder $C_{1-6}$-Alkyl sind und $-CHR^7R^8$ insgesamt 1 bis 6 Kohlenstoffatome enthält), dann n 2 ist;

und worin der Ausdruck "Ar" einen aromatischen Rest bezeichnet, der 6 bis 12 Kohlenstoffatome aufweist, der gegebenenfalls mit einem oder mehreren $C_{1-6}$-Alkoxy-, Halogen-, Trifluormethyl-, Nitro-, Carbalkoxy-, Carboxamido-, Cyano-, Amino-, $(C_{1-6})$-Alkylamino- oder Di-$(C_{1-6})$-alkylamino-Substituenten substituiert sein kann, und der Ausdruck "Het" einen aromatischen Rest bezeichnet, der ein oder mehrere Stickstoffatome als Heteroatome enthält und der gegebenenfalls mit einem oder mehreren $C_{1-6}$-Alkyl-, $C_{1-6}$-Alkoxy-, Halogen-, Trifluormethyl-, Amino-, $(C_{1-6})$-Alkylamino- oder Di$(C_{1-6})$-alkylamino-Substituenten substituiert sein kann, welches Verfahren umfaßt:

(a) Acylieren eines Amins der Formel

$$NHR^5R^9 \qquad (II)$$

(worin $R^5$ und $R^9$ wie oben definiert sind) mit einer Säure der Formel

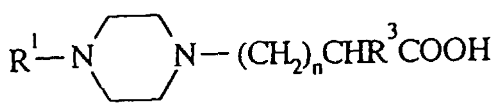

(III)

(worin $R^1$ und $R^3$ wie oben definiert sind) oder mit einem Acylierungsderivat davon, um eine Verbindung der Formel (I) zu ergeben, worin X $-CONR^5R^9$ darstellt, oder
(b) Acylieren eines Piperazinalkylamins der Formel

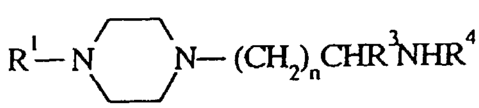

mit einer Säure der Formel

$$R^6COOH$$

oder mit einem Acylierungsderivat davon, um eine Verbindung der Formel (I) zu ergeben, worin X $-NR^4COR^6$ ist, oder
(c) Umsetzen eines Amins der Formel

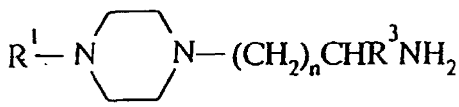

mit einem Anhydrid der Formel

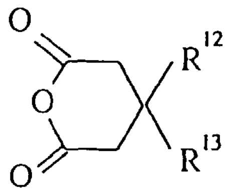

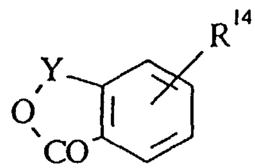

oder mit einer Säure der Formel

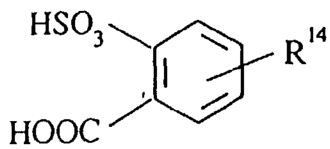

um eine Verbindung der Formel (I) zu ergeben, worin X

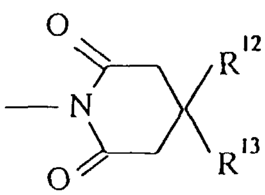

oder

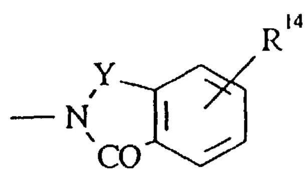

ist, oder

(d) Veresterung einer Säure der Formel (III), wie oben angegeben ist, mit einem Alkohol der Formel $R^6OH$, um eine Verbindung der Formel (I) zu ergeben, worin X $-CO_2R^6$ ist, oder

(e) Verestern eines Piperazinalkohols der Formel

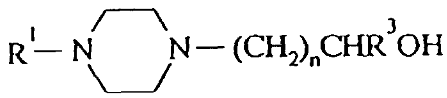

mit einer Säure der Formel $R^{10}COOH$, um eine Verbindung der Formel (I) zu ergeben, worin X $-OCOR^{10}$ ist, oder

(f) Umsetzen eines Isocyanates mit einem Piperazinyl-Alkanol der Formel

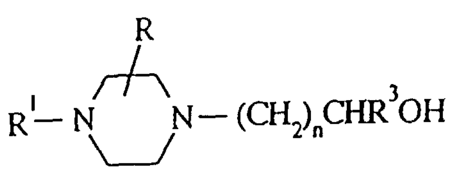

oder mit einem Piperazinyl-Alkylamin der Formel

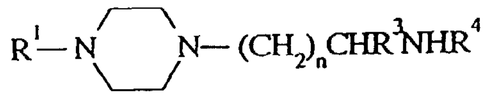

um eine Verbindung der Formel (I) zu ergeben, worin X $-NR^4CONHR^6$ oder $-O.CO.NHR^{11}$ ist, oder
(g) Umsetzen eines Piperazinisocyanat-Derivates der Formel

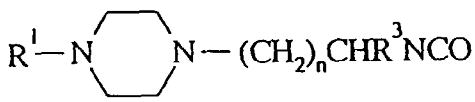

mit einem Alkohol der Formel

$$R^6OH$$

um eine Verbindung der Formel (I) zu ergeben, worin X $-NHCO_2R^6$ ist, oder
(h) Umsetzen eines Amins der Formel

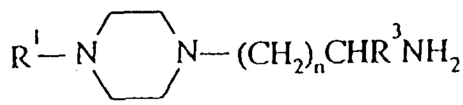

mit einer Verbindung der Formel $R^6OCOHal$ (worin Hal Halogen ist), um eine Verbindung der Formel (I) zu ergeben, worin X $-NHCO_2R^6$ ist, oder
(i) Umsetzen einer Säure der Formel (III), die oben angegeben ist, mit einem Hydroxylamin der Formel $NH_2OR^6$ oder mit einem Hydrazid der Formel $NH_2NHR^6$, um eine Verbindung der Formel (I) zu ergeben, worin X $-CONHOR^6$ bzw. $-CONHNHR^6$ ist, oder
(j) Alkylieren eines Piperazins der Formel

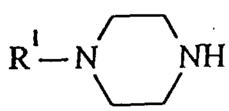

(IV)

mit einem die Gruppe $-(CH_2)_nCHR^3X$ liefernden Alkylierungsmittel, oder
(k) Umsetzen eines Nitrils der Formel (IX)

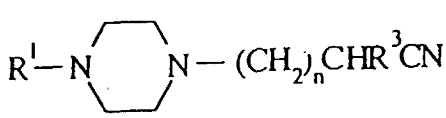

mit einem sekundären Alkohol, um eine Verbindung der Formel (I) zu ergeben, worin X $-CONR^5R^9$ ist, worin $R^5$ Wasserstoff ist und $R^9$ eine sekundäre (nied.)Alkylgruppe ist, oder Unterwerfen des Nitrils einer sauren Hydrolyse, um eine Verbindung der Formel (I) zu ergeben, worin X $-CONH_2$ ist, oder
(l) Entschwefeln einer schwefelhältigen Verbindung der Formel

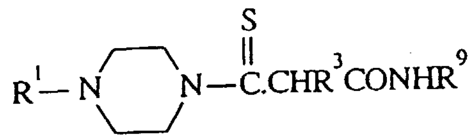

(XI)

um eine Verbindung der Formel (I) zu ergeben, worin X -$CONHR^9$ ist, oder

(m) Umwandeln einer freien Base der Formel (I) in ein pharmazeutisch annehmbares Salz davon, oder

(n) Trennen einer racemischen Verbindung der Formel (I), um ein optisch aktives Enantiomer zu ergeben.

**2.** Verfahren gemäß Anspruch 1, worin im Produkt $R^1$ ein Phenylrest ist, gegebenenfalls substituiert mit einem oder mehreren $C_{1-6}$-Alkoxy-, Halogen-, Trifluormethyl-, Nitro-, Carbalkoxy-, Carboxamido-, Cyano-, Amino-, ($C_{1-6}$)-Alkylamino- oder Di($C_{1-6}$)-alkylamino-Substituenten.

**3.** Verfahren gemäß Anspruch 1 oder 2, worin im Produkt $R^3$ ein Phenylrest ist, gegebenenfalls substituiert mit einem oder mehreren $C_{1-6}$-Alkoxy-, Halogen-, Trifluormethyl-, Nitro-, Carbalkoxy-, Carboxamido-, Cyano-, Amino-, ($C_{1-6}$)-Alkylamino- oder Di($C_{1-6}$)-alkylamino-Substituenten.

**4.** Verfahren gemäß einem der Ansprüche 1 bis 3, worin im Produkt X -$CO_2R^6$ oder -$CONR^5R^9$ darstellt, worin $R^6$, $R^5$ und $R^9$ wie in Anspruch 1 definiert sind.

**5.** Verfahren gemäß einem der Ansprüche 1 bis 3, worin im Produkt X -$CONR^5R^9$ ist, worin $R^5$ und $R^9$ zusammen mit dem Stickstoffatom, an das sie gebunden sind, einen Azetidino-, Pyrrolidino-, Piperidino-, Hexahydroazepino-, Morpholino- oder Piperazinoring darstellen.

**6.** Verfahren gemäß Anspruch 1, worin das Produkt 1-{3-{1-[4-(2-Methoxyphenyl)piperazinyl]}-2-phenylpropionyl} pyrrolidin oder ein pharmazeutisch annehmbares Säureadditionssalz davon ist.

**7.** Verfahren gemäß Anspruch 1, worin das Produkt 1-{3-{1-[4-(2-Methoxyphenyl)piperazinyl]}-2-phenylpropionyl} piperidin oder ein pharmazeutisch annehmbares Säureadditionssalz davon ist.

**8.** Verfahren gemäß Anspruch 1, worin das Produkt ist:

Propyl-3-{1-[4-(2-methoxyphenyl)piperazinyl]}-2-phenylpropanoat,
3-{1-[4-(2-Methoxyphenyl)piperazinyl]}-2-phenyl-N-phenylpropanamid,
3-{1-[4-(2-Methoxyphenyl)piperazinyl]}-2-phenylpropanamid,
N-Methyl-3-{1-[4-(2-methoxyphenyl)piperazinyl]}-2-phenylpropanamid,
N-Ethyl-3-{1-[4-(2-methoxyphenyl)piperazinyl]}-2-phenylpropanamid,
N-Propyl-3-{1-[4-(2-methoxyphenyl)piperazinyl]}-2-phenylpropanamid,
N-Butyl-3-{1-[4-(2-methoxyphenyl)piperazinyl]}-2-phenylpropanamid,
N-Isobutyl-3-{1-[4-(2-methoxyphenyl)piperazinyl]}-2-phenylpropanamid,
N-Cyclopropylmethyl-3-{1-[4-(2-methoxyphenyl)piperazinyl]}-2-phenylpropanamid,
N-Neopentyl-3-{1-[4-(2-methoxyphenyl)piperazinyl]}-2-phenylpropanamid,
N-Hexyl-3-{1-[4-(2-methoxyphenyl)piperazinyl]}-2-phenylpropanamid,
N-Cyclohexylmethyl-3-{1-[4-(2-methoxyphenyl)piperazinyl]}-2-phenylpropanamid,
8-{alpha-{1-[4-(2-Methoxyphenyl)piperazinyl]methyl}phenylacetamidoethyl-8-azaspiro[4.5]deca-7,9-dion,
N,N-Dimethyl-3-{1-[4-(2-methoxyphenyl)piperazinyl]}-2-phenylpropanamid,
3-{1-[4-(2-Methoxyphenyl)piperazinyl]}-2-phenyl-N-(phenylmethyl)propanamid,
N-Cyclohexyl-2-phenyl-3-{1-[4-(2-pyrimidinyl)-piperazinyl]}-propanamid,
3,N-bis{1-[4-(2-Methoxyphenyl)piperazinyl]}-2-phenylpropanamid, 2-Methylpropyl-3-{1-[4-(2-methoxyphenyl) piperazinyl]}-2-phenylpropanoat,
Ethyl-3-{1-[4-(2-methoxyphenyl)piperazinyl]}-2-phenylpropanoat, 2-{1-[4-(2-Methoxyphenyl)piperazinyl]methyl}-N-methyl-3-phenyl-propanamid,
Ethyl-2-{1-[4-(2-methoxyphenyl)piperazinyl]methyl}-3-phenylpropanoat,
3-{1-[4-(2-Methoxyphenyl)piperazinyl]}-N-(3,3-dimethyl)butyl-2-phenylpropionamid,
N-Cyclopropyl-3-{1-[4-(2-methoxyphenyl)piperazinyl]}-2-phenylpropanamid,
1-{3-{1-[4-(2-Methoxyphenyl)piperazinyl]}-2-phenylpropionyl}-pyrrolidin,

N-Cyclooctyl-3-{1-[4-(2-methoxyphenyl)piperazinyl]}-2-phenylpropionamid,
N-Cyclododecyl-3-{1-[4-(2-methoxyphenyl)piperazinyl]}-2-phenylpropionamid,
(S)-N-[2-[1-[4-(2-Methoxyphenyl)piperazinyl]]-1-phenylethyl]-cyclohexancarbonsäureamid,
O-[2-[1-(4-(2-Methoxyphenyl)piperazinyl)]-1-phenylethyl]-N-cyclohexylcarbamat,
O-[2-[1-[4-(2-Methoxyphenyl)piperazinyl]]-1-phenylethyl]-N-phenylcarbamat,
2-{1-[4-(2-Methoxyphenyl)piperazinyl]}-1-phenylethylcyclohexancarboxylat,
Ethyl-(S)-N-[2-[1-[4-(2-Methoxyphenyl)piperazinyl]]-1-phenylethyl]carbamat,
Methyl-3-[1-[4-(2-methoxyphenyl)piperazinyl]]-2-phenylpropionat, N-(1-Ethylpropyl)-3-[1-4-(2-methoxyphenyl)piperazinyl]]-2-phenylpropionamid,
4-[3-[1-[4-(2-Methoxyphenyl)piperazinyl]]-2-phenylpropionyl]-morpholin,
1-{3-{1-[4-(2-Methoxyphenyl)piperazinyl]}-2-phenylpropionyl}-azetidin,
O-{2-[1-[4-(2-Methoxyphenyl)piperazinyl]]-1-phenylethyl}-N-(3-chlorphenyl)carbamat,
1-Ethyl-3-{2-[1-[4-(2-methoxyphenol)piperazinyl]]-1-phenylethyl}carbonat,
2,3-Dihydrobenzo[1,4]dioxin-2-ylcarbonsäure-2-[4-(2-methoxyphenyl)piperazin-1-yl]-1-phenylethylester (S)-8-{2-[1-[4-(2-Methoxyphenyl)piperazinyl]]-1-phenylethyl]}-8-azaspiro[4.5]decan-7,9-dion,
2,3,4,5,6,7-Hexahydro-1-{2-[1-(4-(2-methoxyphenyl)piperazinyl)-methyl]-3-phenylpropanoyl}-1H-azepin,
N-Cycloheptyl-2-{1-[4-(2-methoxyphenyl)piperazinyl]methyl}-3-phenylpropanamid
oder
N-Cyclopropyl-2-{1-[4-(2-methoxyphenyl)piperazinyl]methyl}-3-phenylpropanamid

oder ein pharmazeutisch annehmbares Säureadditionssalz davon.

**9.** Verfahren zur Herstellung einer pharmazeutischen Zusammensetzung, umfassend das Bringen einer Verbindung der Formel (I), wie in Anspruch 1 definiert, oder eines pharmazeutisch annehmbaren Säureadditionssalzes davon in Assoziation mit einem pharmazeutisch annehmbaren Träger.

**10.** Verfahren gemäß Anspruch 9, worin die Verbindung der Formel (I) oder das Salz davon nach dem Verfahren gemäß einem der Ansprüche 1 bis 8 hergestellt wird.

**11.** Verfahren zur Herstellung einer Säure der Formel

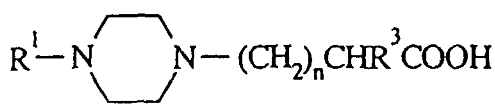

(worin n, $R^1$ und $R^3$ wie in Anspruch 1 definiert sind), welches

(a) das Umsetzen einer Säure der Formel

$$CH_2{=}CR^3COOH \qquad (VIII)$$

und eines Piperazins der Formel

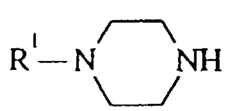

(IV)

mit Hilfe einer Michael-Reaktion, oder
(b) die Hydrolyse eines Nitrils der Formel

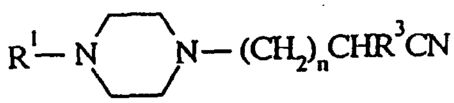

(IX)

umfaßt.

**Patentansprüche für folgenden Vertragsstaat : GR**

1. Verfahren zur Herstellung einer Verbindung der Formel (I)

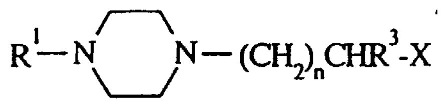

(I)

oder eines pharmazeutisch annehmbaren Säureadditionssalzes davon, worin n eine der ganzen Zahlen 1 oder 2 ist, $R^1$ Ar oder Het ist, $R^3$ Ar oder ein Ar($C_{1-6}$)-alkylrest ist, X -$OCOR^{10}$, -$CO_2R^6$, -$CONR^5R^9$, -$OCO_2R^6$, -$NR^4COR^6$, $OCONHR^{11}$, -$NHCO_2R^6$, -$NR^4CONHR^6$, -$CONHNHR^6$, -$CONHOR^6$,

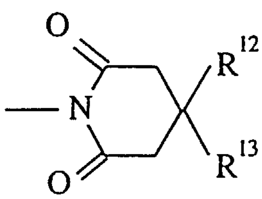

oder

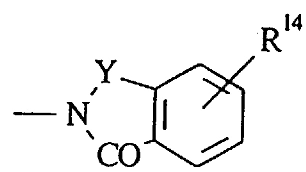

ist, $R^4$ und $R^5$ jeweils Wasserstoff oder $C_{1-6}$-Alkyl sind, $R^6$ -$CHR^7R^8$, Cycloalkyl mit 3 bis 12 Kohlenstoffatomen oder Ar($C_{1-6}$)-alkyl ist (worin $R^7$ und $R^8$ jeweils Wasserstoff oder $C_{1-6}$-Alkyl sind), $R^9$ Wasserstoff, eine Alkylgruppe mit 1 bis 8 Kohlenstoffatomen, die keine tertiäre Alkylgruppe ist, Cycloalkyl mit 3 bis 12 Kohlenstoffatomen, Cycloalkyl($C_{1-6}$)-alkyl, Ar($C_{1-6}$)-alkyl oder 8-Azaspiro[4.5]deca-7,9-dion-8-yl-($C_{1-6}$)-alkyl ist [mit der Maßgabe, dass, wenn $R^9$ Wasserstoff, Alkyl oder Ar($C_{1-6}$)-alkyl ist, $R^5$ keine tertiäre Alkylgruppe ist], oder $R^5$ und $R^9$ zusammen mit dem Stickstoffatom, an das sie gebunden sind, einen Azetidino-, Pyrrolidino-, Piperidino-, Hexahydroazepino-, Morpholino- oder Piperazinoring darstellen, der gegebenenfalls mit $C_{1-6}$-Alkyl, Ar oder Ar($C_{1-6}$)-alkyl substituiert sein kann, $R^{10}$ Cycloalkyl mit 3 bis 12 Kohlenstoffatomen oder 2,3-Dihydro[1,4]benzodioxinyl ist, gegebenenfalls substituiert mit $C_{1-6}$-Alkyl, $C_{1-6}$-Alkoxy oder Halogen, $R^{11}$ Cycloalkyl mit 3 bis 12 Kohlenstoffatomen, Ar oder Ar ($C_{1-6}$)-alkyl ist, $R^{12}$ und $R^{13}$ jeweils $C_{1-6}$-Alkyl sind oder zusammen mit dem Kohlenstoffatom, an das sie beide gebunden sind, $C_{4-6}$-Cycloalkyl darstellen, $R^{14}$ Wasserstoff, Halogen, $C_{1-6}$-Alkyl oder $C_{1-6}$-Alkoxy darstellt und Y CO oder $SO_2$ ist, mit den weiteren Maßgaben, dass

(a) wenn $R^1$ Phenyl ist, gegebenenfalls substituiert mit $CH_3$, $OCH_3$, Cl oder $CF_3$, $R^3$ Phenyl ist, gegebenenfalls substituiert mit $C_{1-6}$-Alkyl, Methoxy oder Halogen, und X -$CONH_2$, -$CON(CH_3)_2$ oder -$COOC_2H_5$ ist, dann n 1 ist, und
(b) wenn $R^1$ 2-Pyrimidyl ist, $R^3$ Phenyl ist, gegebenenfalls an einer oder zwei Ringpositionen substituiert mit $C_{1-6}$-Alkyl, Halogen, Trifluormethyl, Cyano, Nitro, ($C_{1-6}$)-Alkylamino, Di-($C_{1-6}$)-alkylamino, $C_{1-6}$-Alkyloxy, und X

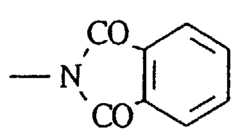

oder -$NR^4COR^6$ ist, worin $R^4$ Wasserstoff oder $C_{1-6}$-Alkyl ist und $R^6$ -$CHR^7R^8$ ist (worin $R^7$ und $R^8$ jeweils

Wasserstoff oder $C_{1-6}$-Alkyl sind und $-CHR^7R^8$ insgesamt 1 bis 6 Kohlenstoffatome enthält), dann n 2 ist;

und worin der Ausdruck "Ar" einen aromatischen Rest bezeichnet, der 6 bis 12 Kohlenstoffatome aufweist, der gegebenenfalls mit einem oder mehreren $C_{1-6}$-Alkoxy-, Halogen-, Trifluormethyl-, Nitro-, Carbalkoxy-, Carboxamido-, Cyano-, Amino-, $(C_{1-6})$-Alkylamino- oder Di-$(C_{1-6})$-alkylamino-Substituenten substituiert sein kann, und der Ausdruck "Het" einen aromatischen Rest bezeichnet, der ein oder mehrere Stickstoffatome als Heteroatome enthält und der gegebenenfalls mit einem oder mehreren $C_{1-6}$-Alkyl-, $C_{1-6}$-Alkoxy-, Halogen-, Trifluormethyl-, Amino-, $(C_{1-6})$-Alkylamino- oder Di$(C_{1-6})$-alkylamino-Substituenten substituiert sein kann, welches Verfahren umfaßt:

(a) Acylieren eines Amins der Formel

$$NHR^5R^9 \qquad (II)$$

(worin $R^5$ und $R^9$ wie oben definiert sind) mit einer Säure der Formel

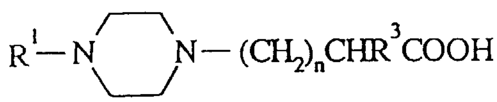

(III)

(worin $R^1$ und $R^3$ wie oben definiert sind) oder mit einem Acylierungsderivat davon, um eine Verbindung der Formel (I) zu ergeben, worin X $-CONR^5R^9$ darstellt, oder

(b) Acylieren eines Piperazinalkylamins der Formel

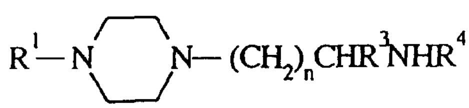

mit einer Säure der Formel

$$R^6COOH$$

oder mit einem Acylierungsderivat davon, um eine Verbindung der Formel (I) zu ergeben, worin X $-NR^4COR^6$ ist, oder

(c) Umsetzen eines Amins der Formel

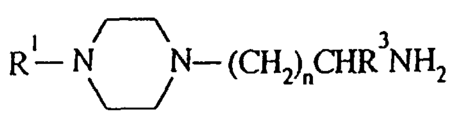

mit einem Anhydrid der Formel

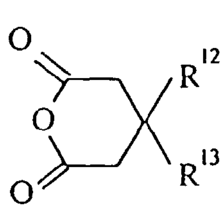

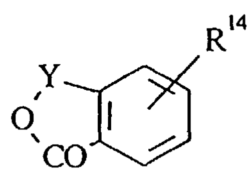

oder mit einer Säure der Formel

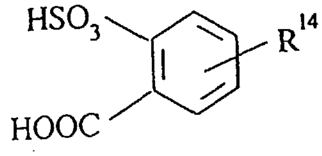

um eine Verbindung der Formel (I) zu ergeben, worin X

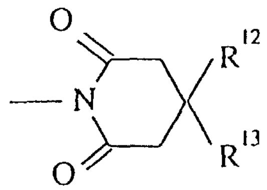 oder 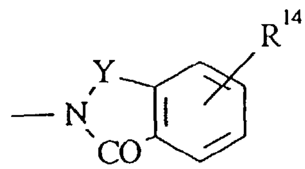

ist, oder

(d) Veresterung einer Säure der Formel (III), wie oben angegeben ist, mit einem Alkohol der Formel $R^6OH$, um eine Verbindung der Formel (I) zu ergeben, worin X $-CO_2R^6$ ist, oder

(e) Verestern eines Piperazinalkohols der Formel

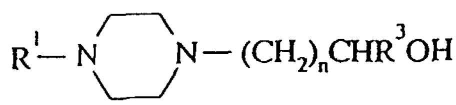

mit einer Säure der Formel $R^{10}COOH$, um eine Verbindung der Formel (I) zu ergeben, worin X $-OCOR^{10}$ ist, oder

(f) Umsetzen eines Isocyanates mit einem Piperazinyl-Alkanol der Formel

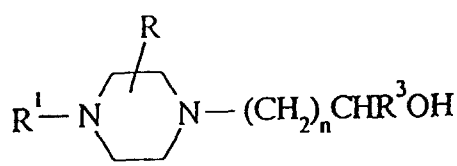

oder mit einem Piperazinyl-Alkylamin der Formel

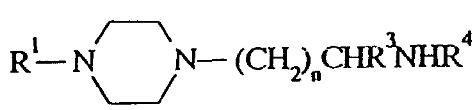

um eine Verbindung der Formel (I) zu ergeben, worin X $-NR^4CONHR^6$ oder $-O.CO.NHR^{11}$ ist, oder

(g) Umsetzen eines Piperazinisocyanat-Derivates der Formel

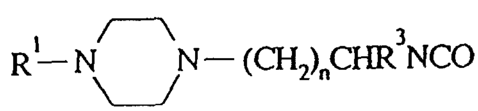

mit einem Alkohol der Formel

$R^6OH$

um eine Verbindung der Formel (I) zu ergeben, worin X $-NHCO_2R^6$ ist, oder

(h) Umsetzen eines Amins der Formel

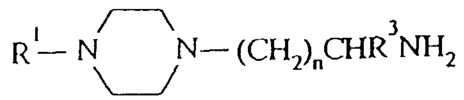

mit einer Verbindung der Formel $R^6OCOHal$ (worin Hal Halogen ist), um eine Verbindung der Formel (I) zu ergeben, worin X $-NHCO_2R^6$ ist, oder

(i) Umsetzen einer Säure der Formel (III), die oben angegeben ist, mit einem Hydroxylamin der Formel $NH_2OR^6$ oder mit einem Hydrazid der Formel $NH_2NHR^6$, um eine Verbindung der Formel (I) zu ergeben, worin X $-CONHOR^6$ bzw. $-CONHNHR^6$ ist, oder

(j) Alkylieren eines Piperazins der Formel

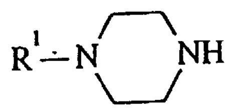

(IV)

mit einem die Gruppe $-(CH_2)_nCHR^3X$ liefernden Alkylierungsmittel, oder

(k) Umsetzen eines Nitrils der Formel (IX)

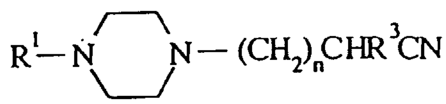

mit einem sekundären Alkohol, um eine Verbindung der Formel (I) zu ergeben, worin X $-CONR^5R^9$ ist, worin $R^5$ Wasserstoff ist und $R^9$ eine sekundäre (nied.)Alkylgruppe ist, oder Unterwerfen des Nitrils einer sauren Hydrolyse, um eine Verbindung der Formel (I) zu ergeben, worin X $-CONH_2$ ist, oder

(l) Entschwefeln einer schwefelhältigen Verbindung der Formel

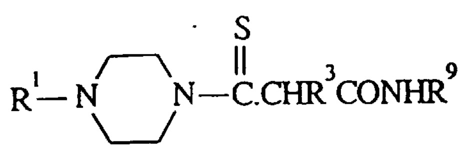

(XI)

um eine Verbindung der Formel (I) zu ergeben, worin X $-CONHR^9$ ist, oder

(m) Umwandeln einer freien Base der Formel (I) in ein pharmazeutisch annehmbares Salz davon, oder

(n) Trennen einer racemischen Verbindung der Formel (I), um ein optisch aktives Enantiomer zu ergeben.

**2.** Verfahren gemäß Anspruch 1, worin im Produkt $R^1$ ein Phenylrest ist, gegebenenfalls substituiert mit einem oder mehreren $C_{1-6}$-Alkoxy-, Halogen-, Trifluormethyl-, Nitro-, Carbalkoxy-, Carboxamido-, Cyano-, Amino-, $(C_{1-6})$-Alkylamino- oder Di$(C_{1-6})$-alkylamino-Substituenten.

**3.** Verfahren gemäß Anspruch 1 oder 2, worin im Produkt $R^3$ ein Phenylrest ist, gegebenenfalls substituiert mit einem oder mehreren $C_{1-6}$-Alkoxy-, Halogen-, Trifluormethyl-, Nitro-, Carbalkoxy-, Carboxamido-, Cyano-, Amino-, $(C_{1-6})$-Alkylamino- oder Di$(C_{1-6})$-alkylamino-Substituenten.

**4.** Verfahren gemäß einem der Ansprüche 1 bis 3, worin im Produkt X $-CO_2R^6$ oder $-CONR^5R^9$ darstellt, worin $R^6$, $R^5$ und $R^9$ wie in Anspruch 1 definiert sind.

**5.** Verfahren gemäß einem der Ansprüche 1 bis 3, worin im Produkt X $-CONR^5R^9$ ist, worin $R^5$ und $R^9$ zusammen mit dem Stickstoffatom, an das sie gebunden sind, einen Azetidino-, Pyrrolidino-, Piperidino-, Hexahydroazepino-, Morpholino- oder Piperazinoring darstellen.

**6.** Verfahren gemäß Anspruch 1, worin das Produkt 1-{3-{1-[4-(2-Methoxyphenyl)piperazinyl]}-2-phenylpropionyl} pyrrolidin oder ein pharmazeutisch annehmbares Säureadditionssalz davon ist.

**7.** Verfahren gemäß Anspruch 1, worin das Produkt 1-{3-(1-[4-(2-Methoxyphenyl)piperazinyl]}-2-phenylpropionyl} piperidin oder ein pharmazeutisch annehmbares Säureadditionssalz davon ist.

**8.** Verfahren gemäß Anspruch 1, worin das Produkt ist:

Propyl-3-{1-[4-(2-methoxyphenyl)piperazinyl]}-2-phenylpropanoat,
3-{1-[4-(2-Methoxyphenyl)piperazinyl]}-2-phenyl-N-phenylpropanamid,
3-{1-[4-(2-Methoxyphenyl)piperazinyl]}-2-phenylpropanamid,
N-Methyl-3-{1-[4-(2-methoxyphenyl)piperazinyl]}-2-phenylpropanamid,
N-Ethyl-3-{1-[4-(2-methoxyphenyl)piperazinyl]}-2-phenylpropanamid,
N-Propyl-3-{1-[4-(2-methoxyphenyl)piperazinyl]}-2-phenylpropanamid,
N-Butyl-3-{1-[4-(2-methoxyphenyl)piperazinyl]}-2-phenylpropanamid,
N-Isobutyl-3-{1-[4-(2-methoxyphenyl)piperazinyl]}-2-phenylpropanamid,
N-Cyclopropylmethyl-3-{1-[4-(2-methoxyphenyl)piperazinyl]}-2-phenylpropanamid,
N-Neopentyl-3-[1-[4-(2-methoxyphenyl)piperazinyl]}-2-phenylpropanamid,
N-Hexyl-3-{1-[4-(2-methoxyphenyl)piperazinyl]}-2-phenylpropanamid,
N-Cyclohexylmethyl-3-{1-[4-(2-methoxyphenyl)piperazinyl]}-2-phenylpropanamid,
8-{alpha-{1-[4-(2-Methoxyphenyl)piperazinyl]methyl}phenylacetamidoethyl-8-azaspiro[4.5]deca-7,9-dion,
N,N-Dimethyl-3-{1-[4-(2-methoxphenyl)piperazinyl]}-2-phenylpropanamid,
3-{1-[4-(2-Methoxyphenyl)piperazinyl]}-2-phenyl-N-(phenylmethyl)propanamid,
N-Cyclohexyl-2-phenyl-3-{1-[4-(2-pyrimidinyl)-piperazinyl]}propanamid,
3,N-bis{1-[4-(2-Methoxyphenyl)piperazinyl]}-2-phenylpropanamid, 2-Methylpropyl-3-{1-[4-(2-methoxyphenyl) piperazinyl]}-2-phenylpropanoat,
Ethyl-3-{1-[4-(2-methoxyphenyl)piperazinyl]}-2-phenylpropanoat, 2-{1-[4-(2-Methoxyphenyl)piperazinyl]methyl}-N-methyl-3-phenylpropanamid,
Ethyl-2-{1-[4-(2-methoxyphenyl)piperazinyl]methyl}-3-phenylpropanoat,
3-{1-[4-(2-Methoxyphenyl)piperazinyl]}-N-(3,3-dimethyl)butyl-2-phenylpropionamid,
N-Cyclopropyl-3-{1-[4-(2-methoxyphenyl)piperazinyl]}-2-phenylpropanamid,
1-{3-{1-[4-(2-Methoxyphenyl)piperazinyl]}-2-phenylpropionyl}-pyrrolidin,
N-Cyclooctyl-3-{1-[4-(2-methoxyphenyl)piperazinyl]}-2-phenylpropionamid,
N-Cyclododecyl-3-{1-[4-(2-methoxyphenyl)piperazinyl]}-2-phenylpropionamid,
(S)-N-[2-[1-[4-(2-Methoxyphenyl)piperazinyl]]-1-phenylethyl]-cyclohexancarbonsäureamid,
O-[2-[1-(4-(2-Methoxyphenyl)piperazinyl)]-1-phenylethyl]-N-cyclohexylcarbamat,
O-[2-[1-[4-(2-Methoxyphenyl)piperazinyl]]-1-phenylethyl]-N-phenylcarbamat,
2-{1-[4-(2-Methoxyphenyl)piperazinyl]}-1-phenylethylcyclohexancarboxylat,
Ethyl-(S)-N-[2-[1-[4-(2-Methoxyphenyl)piperazinyl]]-1-phenylethyl]carbamat,
Methyl-3-[1-[4-(2-methoxyphenyl)piperazinyl]]-2-phenylpropionat, N-(1-Ethylpropyl)-3-[1-4-(2-methoxyphenyl)piperazinyl]]-2-phenylpropionamid,
4-[3-[1-[4-(2-Methoxyphenyl)piperazinyl]]-2-phenylpropionyl]-morpholin,
1-{3-{1-[4-(2-Methoxyphenyl)piperazinyl]}-2-phenylpropionyl}-azetidin,
O-{2-[1-[4-(2-Methoxyphenyl)piperazinyl]]-1-phenylethyl}-N-(3-chlorphenyl)carbamat,
1-Ethyl-3-{2-[4-(2-methoxyphenol)piperazinyl]]-1-phenylethyl}carbonat,
2,3-Dihydrobenzo[1,4]dioxin-2-ylcarbonsäure-2-[4-(2-methoxyphenyl)piperazin-1-yl]-1-phenylethylester (S)-8-{2-[1-[4-(2-Methoxyphenyl)piperazinyl]]-1-phenylethyl]}-8-azaspiro[4.5]decan-7,9-dion,
2,3,4,5,6,7-Hexahydro-1-{2-[1-(4-(2-methoxyphenyl)piperazinyl)-methyl]-3-phenylpropanoyl}-1H-azepin,
N-Cycloheptyl-2-{1-[4-(2-methoxyphenyl)piperazinyl]methyl}-3-phenylpropanamid
oder
N-Cyclopropyl-2-{1-[4-(2-methoxyphenyl)piperazinyl]methyl}-3-phenylpropanamid

oder ein pharmazeutisch annehmbares Säureadditionssalz davon.

**9.** Verfahren zur Herstellung einer pharmazeutischen Zusammensetzung, umfassend das Bringen einer Verbindung der Formel (I), wie in Anspruch 1 definiert, oder eines pharmazeutisch annehmbaren Säureadditionssalzes davon in Assoziation mit einem pharmazeutisch annehmbaren Träger.

**10.** Verfahren gemäß Anspruch 9, worin die Verbindung der Formel (I) oder das Salz davon nach dem Verfahren gemäß einem der Ansprüche 1 bis 8 hergestellt wird.

**11.** Verfahren zur Herstellung einer Säure der Formel

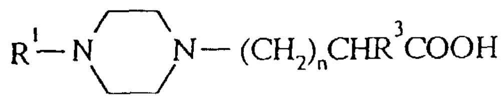

(worin n, $R^1$ und $R^3$ wie in Anspruch 1 definiert sind), welches

(a) das Umsetzen einer Säure der Formel

$$CH_2{=}CR^3COOH \qquad (VIII)$$

und eines Piperazins der Formel

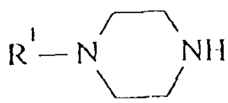

(IV)

mit Hilfe einer Michael-Reaktion, oder
(b) die Hydrolyse eines Nitrils der Formel

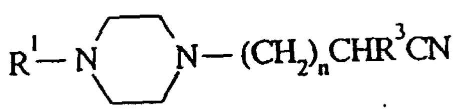

(IX)

umfaßt.

**12.** Säure der Formel

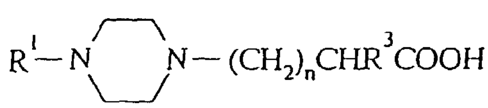

(worin n, $R^1$ und $R^3$ wie in Anspruch 1 definiert sind).

## Revendications

**Revendications pour les Etats contractants suivants : AT, BE, CH, DE, DK, FR, IT, LI, LU, NL, SE**

**1.** Composé de formule générale (I)

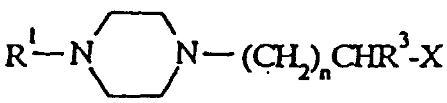

(I)

ou sel d'addition acide pharmaceutiquement acceptable de celui-ci dans lequel

n est un des nombres entiers 1 ou 2,
$R^1$ est Ar ou Hét,
$R^3$ est Ar ou un radical Ar-alkyle($C_{1-6}$),
X est $-OCOR^{10}$, $-CO_2R^6$, $-CONR^5R^9$, $-OCO_2R^6$, $-NR^4COR^6$, $-OCONHR^{11}$, $-NHCO_2R^6$, $-NR^4CONHR^6$,$-CONHNHR^6$,$-CONHOR^6$,

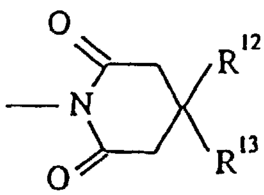

ou

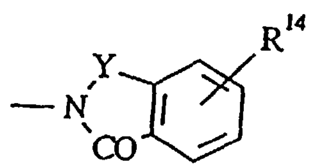

$R^4$ et $R^5$ sont chacun hydrogène ou alkyle en $C_{1-6}$,
$R^6$ est $-CHR^7R^8$, cycloalkyle de 3 à 12 atomes de carbone, ou Ar-alkyle($C_{1-6}$) (où $R^7$ et $R^8$ sont chacun hydrogène ou alkyle en $C_{1-6}$)
$R^9$ est hydrogène, un radical alkyle de 1 à 8 atomes de carbone autre qu'un radical d'alkyle tertiaire, cycloalkyle de 3 à 12 atomes de carbone, cycloalkyl-alkyle($C_{1-6}$), Ar-alkyle($C_{1-6}$) ou 8-azaspiro[4,5]déca-7,9-dione-8-yl-alkyle($C_{1-6}$) [avec la condition que quand $R^9$ est hydrogène, alkyle ou Ar-alkyle($C_{1-6}$), $R^5$ est autre qu'un radical alkyle tertiaire], ou $R^5$ et $R^9$ conjointement avec l'atome d'azote auquel ils sont attachés représentent un cycle azétidino, pyrrolidino, pipéridino, hexahydroazépino, morpholino ou pipérazino qui peut être facultativement substitué par alkyle en $C_{1-6}$, Ar ou Ar-alkyle($C_{1-6}$),
$R^{10}$ est cycloalkyle de 3 à 12 atomes de carbone, ou 2,3-dihydro[1,4]benzodioxinyle facultativement substitué par alkyle en $C_{1-6}$, alkoxy en $C_{1-6}$ ou halogène,
$R^{11}$ est cycloalkyle de 3 à 12 atomes de carbone, Ar ou Ar-alkyle($C_{1-6}$),
$R^{12}$ et $R^{13}$ sont chacun alkyle en $C_{1-6}$ ou conjointement avec l'atome de carbone auquel ils sont attachés tous deux représentent cycloalkyle en $C_{4-6}$,
$R^{14}$ représente hydrogène, halogène, alkyle en $C_{1-6}$ ou alkoxy en $C_{1-6}$, et
Y est CO ou $SO_2$

avec les conditions supplémentaires que

(a) quand $R^1$ est phényle facultativement substitué par $CH_3$, $OCH_3$, Cl ou $CF_3$, $R^3$ est phényle facultativement substitué par alkyle en $C_{1-6}$, méthoxy ou halogène, et X est $-CONH_2$, $-CON(CH_3)_2$ ou $-COOC_2H_5$ alors n est 1, et (b) quand $R^1$ est 2-pyrimidyle, $R^3$ est phényle facultativement substitué à une ou deux positions du cycle avec alkyle en $C_{1-6}$, halogène, trifluorométhyle, cyano, nitro, alkyl($C_{1-6}$)amino, dialkyl($C_{1-6}$)amino, alkyloxy en $C_{1-6}$ et X est

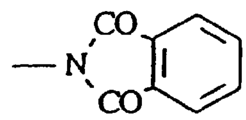

ou $-NR^4COR^6$ où $R^4$ est hydrogène ou alkyle en $C_{1-6}$ et $R^6$ est $-CHR^7R^8$ (où $R^7$ et $R^8$ sont chacun hydrogène ou alkyle en $C_{1-6}$ et $-CHR^7R^8$ contient un total de 1 à 6 atomes de carbone) alors n est 2;

et dans lequel le terme "Ar" désigne un radical aromatique ayant 6 à 12 atomes de carbone qui facultativement peut être substitué par un ou plusieurs substituants alkoxy en $C_{1-6}$, halogène, trifluorométhyle, nitro, carbalkoxy, carboxamido, cyano, amino, alkyl($C_{1-6}$) amino ou dialkyl($C_{1-6}$) amino et le terme "Hét" désigne un radical aromatique contenant un ou plusieurs atomes d'azote comme hétéroatomes et qui peut être substitué par un ou plusieurs substituants alkyle en $C_{1-6}$, alkoxy en $C_{1-6}$, halogène, trifluorométhyle, amino, alkyl($C_{1-6}$) amino ou dialkyl($C_{1-6}$) amino,

**2.** Composé selon la revendication 1, dans lequel $R^1$ est un radical phényle facultativement substitué par un ou plusieurs substituants alkoxy en $C_{1-6}$, halogène, trifluorométhyle, nitro, carbalkoxy, carboxamido, cyano, amino, alkyl($C_{1-6}$)amino ou dialkyl($C_{1-6}$)amino.

**3.** Composé selon la revendication 1 ou 2, dans lequel $R^3$ est un radical phényle facultativement substitué par un ou plusieurs substituants alkoxy en $C_{1-6}$, halogène, trifluorométhyle, nitro, carbalkoxy, carboxamido, cyano, amino, alkyl($C_{1-6}$)amino ou dialkyl($C_{1-6}$)amino.

**4.** Composé selon l'une quelconque des revendications 1 à 3, dans lequel X représente $-CO_2R^6$ ou $-CONR^5R^9$ où $R^6$, $R^3$ et $R^9$ sont tels que définis dans la revendication 1.

**5.** Composé selon l'une quelconque des revendications 1 à 3, dans lequel X est $-CONR^5R^9$ où $R^5$ et $R^9$ conjointement avec l'atome d'azote auquel ils sont attachés représentent un cycle azétidino, pyrrolidino, pipéridino, hexahydroazépino, morpholino ou pipérazino.

**6.** Composé selon la revendication 1, qui est la 1-{3-{1-[4-(2-méthoxyphényl)pipérazinyl]}-2-phénylpropionyl)pyrrolidine ou un sel d'addition acide pharmaceutiquement acceptable de celle-ci.

**7.** Composé selon la revendication 1, qui est la 1-{3-{1-[4-(2-méthoxyphényl)pipérazinyl]}-2-phénylpropionyl}pipéridine ou un sel d'addition acide pharmaceutiquement acceptable de celle-ci.

**8.** Composé selon la revendication 1, qui est

- le 3-{1-[4-(2-méthoxyphényl)pipérazinyl]}-2-phénylpropanoate de propyle,
- le 3-{1-[4-(2-méthoxyphényl)pipérazinyl]}-2-phényl-N-phénylpropanamide,
- le 3-{1-[4-(2-méthoxyphényl)pipérazinyl]}-2-phénylpropanamide,
- le N-méthyl-3-{1-[4-(2-méthoxyphényl)pipérazinyl]}-2-phénylpropanamide,
- le N-éthyl-3-{1-[4-(2-méthoxyphényl)pipérazinyl]}-2-phénylpropanamide,
- le N-propyl-3-{1-[4-(2-méthoxyphényl)pipérazinyl]}-2-phénylpropanamide,
- le N-butyl-3-{1-[4-(2-méthoxyphényl)pipérazinyl]}-2-phénylpropanamide,
- le N-isobutyl-3-{1-[4-(2-méthoxyphényl)pipérazinyl]}-2-phénylpropanamide,
- le N-cyclopropylméthyl-3-{1-[4-(2-méthoxyphényl)-pipérazinyl]}-2-phénylpropanamide,
- le N-néopentyl-3-{1-[4-(2-méthoxyphényl)pipérazinyl]}-2-phénylpropanamide,
- le N-hexyl-3-{1-[4-(2-méthoxyphényl)pipérazinyl]}-2-phénylpropanamide,
- le N-cyclohexylméthyl-3-{1-[4-(2-méthoxyphényl)-pipérazinyl]}-2-phénylpropanamide,
- la 8-{α-{1-[4-(2-méthoxyphényl)pipérazinyl]méthyl}-phénylacétamidoéthyl-8-azaspiro[4,5]déca-7,9-dione,
- le N,N-diméthyl-3-{1-[4-(2-méthoxyphényl)pipérazinyl]}-2-phénylpropanamide,
- le 3-{1-[4-(2-méthoxyphényl)pipérazinyl]}-2-phényl-N-(phénylméthyl)propanamide,
- le N-cyclohexyl-2-phényl-3-{1-[4-(2-pyrimidyl)-pipérazinyl]}propanamide,
- le 3,N-bis{1-[4-(2-méthoxyphényl)pipérazinyl]}-2-phénylpropanamide,
- le 3-{1-[4-(2-méthoxyphényl)pipérazinyl]}-2-phénylpropanoate de 2-méthylpropyle,
- le 3-{1-[4-(2-méthoxyphényl)pipérazinyl]}-2-phénylpropanoate d'éthyle,

- le 2-{1-[4-(2-méthoxyphényl)pipérazinyl]méthyl}-N-méthyl-3-phénylpropanamide,
- le 2-{1-[4-(2-méthoxyphényl)pipérazinyl]méthyl}-3-phénylpropanoate d'éthyle,
- le 3-{1-[4-(2-méthoxyphényl)pipérazinyl]}-N-(3,3-diméthyl)butyl)-2-phénylpropionamide,
- le N-cyclopropyl-3-{1-[4-(2-méthoxyphényl)pipérazinyl]}-2-phénylpropanamide,
- la 1-{3-{1-[4-(2-méthoxyphényl)pipérazinyl]}-2-phénylpropionyl}pyrrolidine,
- le N-cyclooctyl-3-{1-[4-(2-méthoxyphényl)pipérazinyl]}-2-phénylpropionamide,
- le N-cyclododécyl-3-{1-[4-(2-méthoxyphényl)pipérazinyl]}-2-phénylpropionamide,
- l'amide de l'acide (S)-N-[2-[1-[4-(2-méthoxyphényl)-pipérazinyl]]-1-phényléthyl]cyclohexanecarboxylique,
- l'O-[2-[1-[4-(2-méthoxyphényl)pipérazinyl]]-1-phényléthyl]-N-cyclohexylcarbamate,
- l'O-[2-[1-[4-(2-méthoxyphényl)pipérazinyl]]-1-phényléthyl]-N-phénylcarbamate,
- le 2-{1-[4-(2-méthoxyphényl)pipérazinyl]}-1-phényléthylcyclohexanecarboxylate,
- le (S)-N-[2-[1-[4-(2-méthoxyphényl)pipérazinyl]]-1-phényléthyl]carbamate d'éthyle,
- le 3-[1-[4-(2-méthoxyphényl)pipérazinyl]]-2-phénylpropionate de méthyle,
- le N-(1-éthylpropyl)-3-[1-[4-(2-méthoxyphényl)pipérazinyl]]-2-phénylpropionamide,
- la 4-[3-[1-[4-(2-méthoxyphényl)pipérazinyl]]-2-phénylpropionyl]morpholine,
- la 1-{3-{1-[4-(2-méthoxyphényl)pipérazinyl]}-2-phénylpropionyl}azétidine,
- l'O-{2-[1-[4-(2-méthoxyphényl)pipérazinyl]]-1-phényléthyl}-N-(3-chlorophényl)carbamate,
- le 1-éthyl-3-{2-[1-[4-(2-méthoxyphénol)pipérazinyl]]-1-phényléthyl}carbonate,
- l'ester 2-[4-(2-méthoxyphényl)pipérazin-1-yl]-1-phényléthylique de l'acide 2,3-dihydrobenzo[1,4]dioxin-2-yl-carboxylique,
- la (S)-8-{2-[1-[4-(2-méthoxyphényl)pipérazinyl]]-1-phényléthyl]-8-azaspiro[4,5]décan-7,9-dione,
- la 2,3,4,5,6,7-hexahydro-1-{2-[1-(4-(2-méthoxyphényl)-pipérazinyl)méthyl]-3-phénylpropanoyl}-1H-azépine,
- le N-cycloheptyl-2-{1-[4-(2-méthoxyphényl)pipérazinyl]-méthyl}-3-phénylpropanamide,
- ou le N-cyclopropyl-2-{1-[4-(2-méthoxyphényl)-pipérazinyl]méthyl}-3-phénylpropanamide

ou un sel d'addition acide pharmaceutiquement acceptable de ceux-ci.

**9.** Procédé de préparation d'un composé selon la revendication 1, dans lequel X représente $-CONR^5R^9$, qui comprend l'acylation d'une amine de formule

$$NHR^5R^9 \qquad (II)$$

(où $R^3$ et $R^9$ sont comme définis dans la revendication 1) avec un acide de formule

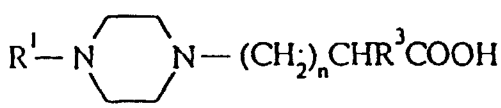

**(III)**

(où $R^1$ et $R^3$ sont comme définis dans la revendication 1) ou avec un dérivé d'acylation de celui-ci.

**10.** Procédé de préparation d'un composé selon la revendication 1, dans lequel X est $-NR^4COR^6$, qui comprend l'acylation d'une alkylamine de pipérazine de formule

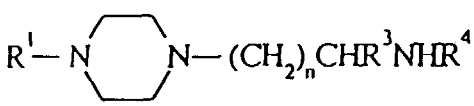

avec un acide de formule

$$R^6COOH$$

ou avec un dérivé d'acylation de celui-ci.

**11.** Procédé de préparation d'un composé selon la revendication 1, dans lequel X est

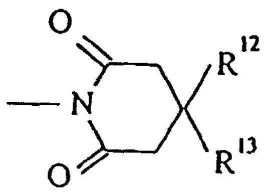

ou

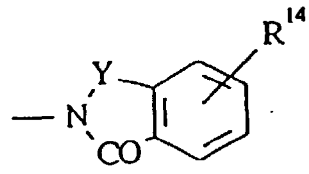

qui comprend la réaction d'une amine de formule

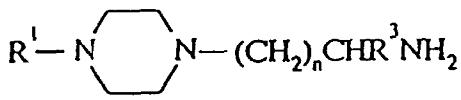

avec un anhydride de formule

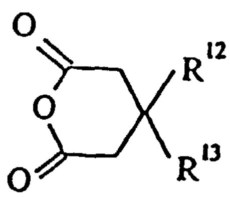

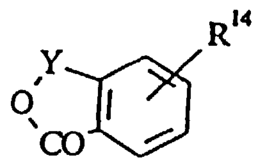

ou, la préparation du composé où Y est $SO_2$, en faisant réagir l'amine avec un acide de formule

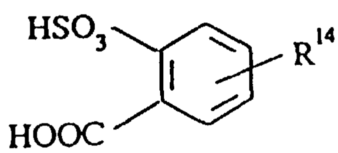

**12.** Procédé de préparation d'un composé selon la revendication 1, dans lequel X est $-CO_2R^6$, qui comprend l'estérification d'un acide de formule (III), comme donné dans la revendication 9 ci-dessus, avec un alcool de formule $R^6OH$.

**13.** Procédé de préparation d'un composé selon la revendication 1, dans lequel X est $-OCOR^{10}$, qui comprend l'estérification d'un alcool de pipérazine de formule

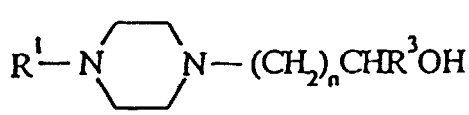

avec un acide de formule $R^{10}COOH$.

**14.** Procédé de préparation d'un composé selon la revendication 1, dans lequel X est $-NR^4CONHR^6$ ou $-O.CO.NHR^{11}$, qui comprend la réaction d'un isocyanate avec un pipérazinyl-alcanol de formule

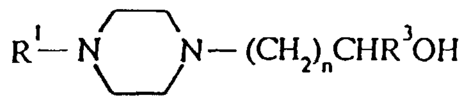

ou avec une pipérazinyl-alkylamine de formule

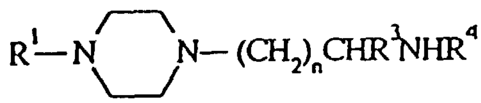

**15.** Procédé de préparation d'un composé selon la revendication 1, dans lequel X est $-NHCO_2R^6$, qui comprend la réaction d'un dérivé isocyanate de pipérazine de formule

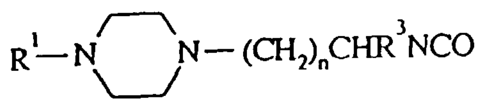

avec un alcool de formule $R^6OH$.

**16.** Procédé de préparation d'un composé selon la revendication 1, dans lequel X est $-NHCO_2R^6$, qui comprend la réaction d'une amine de formule

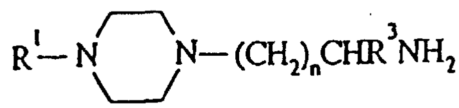

avec un composé de formule $R^6OCOHal$ (où Hal est halogène).

**17.** Procédé de préparation d'un composé selon la revendication 1, dans lequel X est $-CONHOR^6$, qui comprend la réaction d'un acide de formule (III), donné dans la revendication 9, avec une hydroxylamine de formule $NH_2OR^6$.

**18.** Procédé de préparation d'un composé selon la revendication 1, qui comprend l'alkylation d'une pipérazine de formule

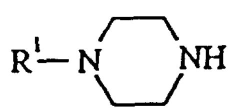

**(IV)**

avec un agent d'alkylation fournissant le radical $-(CH_2)_nCHR^3X$.

**19.** Procédé de préparation d'un composé selon la revendication 1, dans lequel X est $-CONHR^9$ où $R^9$ est un radical alkyle secondaire en $C_{1-6}$ ou X est $-CONH_2$, qui comprend la réaction d'un nitrile de formule (IX)

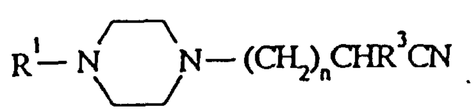

avec un alcool secondaire pour donner un composé de formule (I), dans lequel X est $-CONR^5R^9$ où $R^5$ est hydro-

gène et $R^9$ est un radical alkyle secondaire en $C_{1-6}$ ou en soumettant le nitrile à l'hydrolyse acide pour donner un composé de formule (I), dans lequel X est $-CONH_2$.

**20.** Procédé de préparation d'un composé selon la revendication 1, dans lequel X est $-CONHR^9$, qui comprend la désulfuration d'un composé contenant du soufre de formule

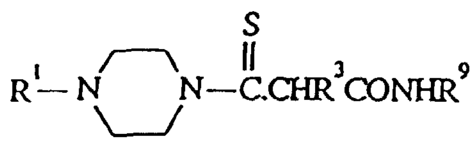

(XI)

**21.** Procédé de préparation d'un sel pharmaceutiquement acceptable d'un composé selon la revendication 1, qui comprend la conversion d'une base libre de formule (I) en son sel.

**22.** Procédé qui comprend la résolution d'un composé racémique de formule (I) selon la revendication 1 en un énantiomère optiquement actif.

**23.** Composition pharmaceutique comprenant un composé selon l'une quelconque des revendications 1 à 8 en association avec un support pharmaceutiquement acceptable.

**24.** Composé selon l'une quelconque des revendications 1 à 8 pour l'utilisation comme produit pharmaceutique.

**25.** Acide de formule

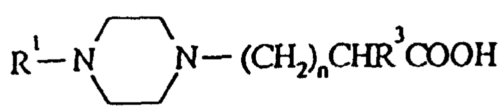

(où n, $R^1$ et $R^3$ sont comme définis dans la revendication 1).

**26.** Procédé de préparation d'un composé selon la revendication 1, dans lequel X est $-CONHNHR^6$, qui comprend la réaction d'un acide de formule (III), comme défini dans la revendication 9, avec un hydrazide de formule $NH_2NHR^6$.

**Revendications pour l'Etat contractant suivant : ES**

**1.** Procédé de préparation d'un composé de formule générale (I)

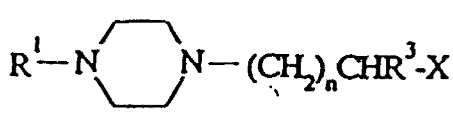

(I)

ou d'un sel d'addition acide pharmaceutiquement acceptable de celui-ci dans lequel

n est un des nombres entiers 1 ou 2,
$R^1$ est Ar ou Hét,
$R^3$ est Ar ou un radical Ar-alkyle($C_{1-6}$),
X est $-OCOR^{10}$, $-CO_2R^6$, $-CONR^5R^9$, $-OCO_2R^6$, $-NR^4COR^6$, $-OCONHR^{11}$, $-NHCO_2R^6$, $-NR^4CONHR^6$, $-CONHNHR^6$, $-CONHOR^6$,

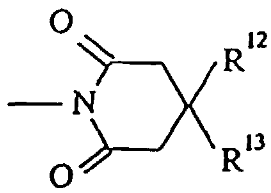

ou

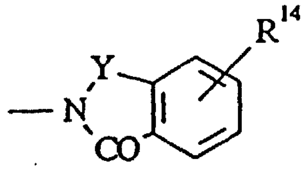

$R^4$ et $R^5$ sont chacun hydrogène ou alkyle en $C_{1-6}$,
$R^6$ est $-CHR^7R^8$, cycloalkyle de 3 à 12 atomes de carbone, ou Ar-alkyle($C_{1-6}$) (où $R^7$ et $R^8$ sont chacun hydrogène ou alkyle en $C_{1-6}$)
$R^9$ est hydrogène, un radical alkyle de 1 à 8 atomes de carbone autre qu'un radical d'alkyle tertiaire, cycloalkyle de 3 à 12 atomes de carbone, cycloalkyl-alkyle($C_{1-6}$), Ar-alkyle($C_{1-6}$) ou 8-azaspiro[4,5]déca-7,9-dione-8-yl-alkyle($C_{1-6}$) [avec la condition que quand $R^9$ est hydrogène, alkyle ou Ar-alkyle($C_{1-6}$), $R^5$ est autre qu'un radical alkyle tertiaire], ou $R^5$ et $R^9$ conjointement avec l'atome d'azote auquel ils sont attachés représentent un cycle azétidino, pyrrolidino, pipéridino, hexahydroazépino, morpholino ou pipérazino qui peut être facultativement substitué par alkyle en $C_{1-6}$, Ar ou Ar-alkyle($C_{1-6}$),
$R^{10}$ est cycloalkyle de 3 à 12 atomes de carbone, ou 2,3-dihydro[1,4]benzodioxinyle facultativement substitué par alkyle en $C_{1-6}$, alkoxy en $C_{1-6}$ ou halogène,
$R^{11}$ est cycloalkyle de 3 à 12 atomes de carbone, Ar ou Ar-alkyle($C_{1-6}$),
$R^{12}$ et $R^{13}$ sont chacun alkyle en $C_{1-6}$ ou conjointement avec l'atome de carbone auquel ils sont attachés tous deux représentent cycloalkyle en $C_{4-6}$,
$R^{14}$ représente hydrogène, halogène, alkyle en $C_{1-6}$ ou alkoxy en $C_{1-6}$, et
Y est CO ou $SO_2$

avec les conditions supplémentaires que

(a) quand $R^1$ est phényle facultativement substitué par $CH_3$, $OCH_3$, Cl ou $CF_3$, $R^3$ est phényle facultativement substitué par alkyle en $C_{1-6}$, méthoxy ou halogène, et X est $-CONH_2$, $-CON(CH_3)_2$ ou $-COOC_2H_5$ alors n est 1, et (b) quand $R^1$ est 2-pyrimidyle, $R^3$ est phényle facultativement substitué à une ou deux positions du cycle avec alkyle en $C_{1-6}$, halogène, trifluorométhyle, cyano, nitro, alkyl($C_{1-6}$)amino, dialkyl($C_{1-6}$)amino, alkyloxy en $C_{1-6}$ et X est

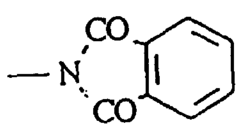

ou $-NR^4COR^6$ où $R^4$ est hydrogène ou alkyle en $C_{1-6}$ et $R^6$ est $-CHR^7R^8$ (où $R^7$ et $R^8$ sont chacun hydrogène ou alkyle en $C_{1-6}$ et $-CHR^7R^8$ contient un total de 1 à 6 atomes de carbone) alors n est 2;

et dans lequel le terme "Ar" désigne un radical aromatique ayant 6 à 12 atomes de carbone qui facultativement peut être substitué par un ou plusieurs substituants alkoxy en $C_{1-6}$, halogène, trifluorométhyle, nitro, carbalkoxy, carboxamido, cyano, amino, alkyl($C_{1-6}$)amino ou dialkyl($C_{1-6}$) amino et le terme "Hét" désigne un radical aromatique contenant un ou plusieurs atomes d'azote comme hétéroatomes et qui peut être substitué par un ou plusieurs substituants alkyle en $C_{1-6}$, alkoxy en $C_{1-6}$, halogène, trifluorométhyle, amino, alkyl($C_{1-6}$) amino ou dialkyl($C_{1-6}$) amino;
procédé qui comprend

(a) l'acylation d'une amine de formule

$$NHR^5R^9 \qquad (II)$$

(où $R^5$ et $R^9$ sont comme définis ci-dessus) avec un acide de formule

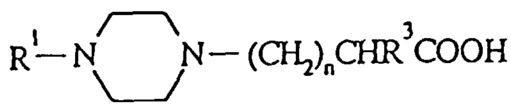

(III)

(où $R^1$ et $R^3$ sont comme définis ci-dessus) ou avec un dérivé d'acylation de celui-ci pour donner un composé de formule (I) où X représente $-CONR^5R^9$, ou

(b) l'acylation d'une alkylamine de pipérazine de formule

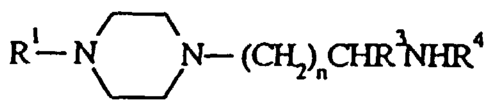

avec un acide de formule $R^6COOH$ ou avec un dérivé d'acylation de celui-ci pour donner un composé de formule (I) dans laquelle X est $-NR^4COR^6$, ou

(c) la réaction d'une amine de formule

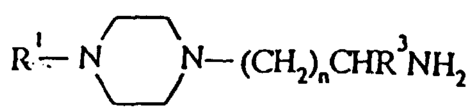

avec un anhydride de formule

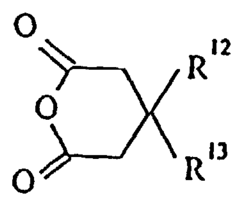

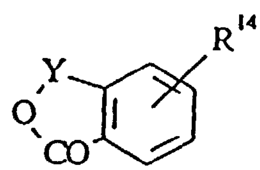

ou avec un acide de formule

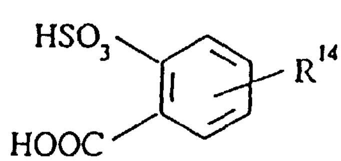

pour donner un composé de formule (I) dans laquelle X est

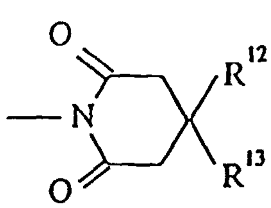

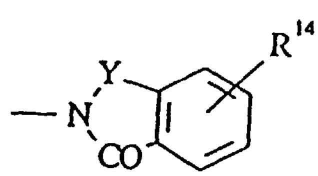

ou

ou (d) l'estérification d'un acide de formule (III), comme donné ci-dessus avec un alcool de formule $R^6OH$ pour donner un composé de formule (I) dans lequel X est $-CO_2R^6$, ou
(e) l'estérification d'un alcool de pipérazine de formule

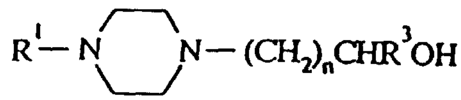

avec un acide de formule $R^{10}COOH$ pour donner un composé de formule (I) dans laquelle X est $-OCOR^{10}$, ou
(f) la réaction d'un isocyanate avec un pipérazinyl-alcanol de formule

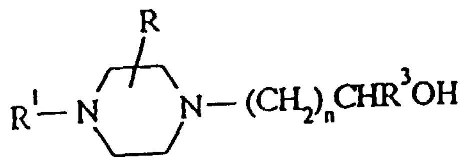

ou avec une pipérazinyl-alkylamine de formule

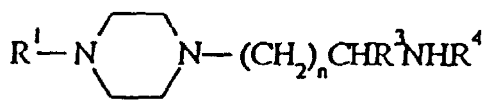

pour donner un composé de formule (I) dans lequel X est $-NR^4CONHR^6$ ou $-OCONHR^{11}$, ou
(g) la réaction d'un dérivé d'isocyanate de pipérazine de formule

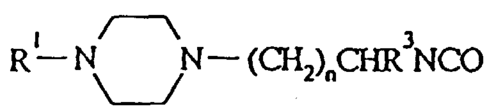

avec un alcool de formule $R^6OH$
pour donner un composé de formule (I) dans lequel X est $-NHCO_2R^6$, ou
(h) la réaction d'une amine de formule

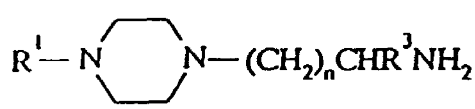

avec un composé de formule $R^6OCOHal$ (où Hal est halogène) pour donner un composé de formule (I) dans lequel X est $-NHCO_2R^6$, ou
(i) la réaction d'un acide de formule (III) comme donné ci-dessus, avec une hydroxylamine de formule $NH_2OR^6$ ou avec un hydrazide de formule $NH_2NHR^6$ pour donner un composé de formule (I) dans lequel X est respectivement $-CONHOR^6$ ou $-CONHNHR^6$, ou
(j) l'alkylation d'une pipérazine de formule

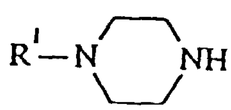

(IV)

avec un agent d'alkylation fournissant le radical $-(CH_2)_nCHR^3X$, ou
(k) la réaction d'un nitrile de formule (IX)

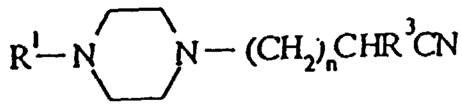

avec un alcool secondaire pour donner un composé de formule (I) dans lequel X est $-CONR^5R^9$ où $R^5$ est hydrogène et $R^9$ est un alkyle secondaire (inférieur) ou en soumettant le nitrile à l'hydrolyse acide pour donner un composé de formule (I) dans lequel X est $-CONH_2$, ou
(l) la désulfuration d'un composé contenant du soufre de formule

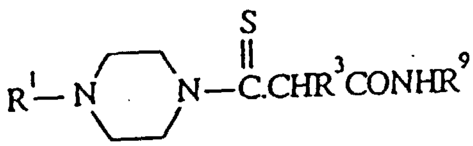

(XI)

pour donner un composé de formule (I) dans lequel X est $-CONHR^9$, ou
(m) la conversion d'une base libre de formule (I) en un sel pharmaceutiquement acceptable de celui-ci, ou
(n) la résolution d'un composé racémique de formule (I) pour donner un énantiomère optiquement actif.

**2.** Procédé selon la revendication 1, dans lequel, dans le produit, $R^1$ est un radical phényle facultativement substitué par un ou plusieurs substituants alkoxy en $C_{1-6}$, halogène, trifluorométhyle, nitro, carbalkoxy, carboxamido, cyano, amino, alkyl($C_{1-6}$)amino ou dialkyl($C_{1-6}$)amino.

**3.** Procédé selon la revendication 1 ou la revendication 2, dans lequel, dans le produit, $R^3$ est un radical phényle facultativement substitué par un ou plusieurs substituants alkoxy en $C_{1-6}$, halogène, trifluorométhyle, nitro, carbalkoxy, carboxamido, cyano, amino, alkyl($C_{1-6}$)amino ou dialkyl($C_{1-6}$)amino.

**4.** Procédé selon l'une quelconque des revendications 1 à 3, dans lequel, dans le produit, X représente $-CO_2R^6$ ou $-CONR^5R^9$ où $R^5$ et $R^9$ sont comme définis dans la revendication 1.

**5.** Procédé selon l'une quelconque des revendications 1 à 3 dans lequel, dans le produit, X est $-CONR^5R^9$ où $R^5$ et $R^9$ conjointement avec l'atome d'azote auquel ils sont liés représentent un cycle azétidino, pyrrolidino, pipéridino, hexahydroazépino, morpholino ou pipérazino.

**6.** Procédé selon la revendication 1 dans lequel le produit est la 1-{3-{1-[4-(2-méthoxyphényl)pipérazinyl]}-2-phénylpropionyl}pyrrolidine ou un sel d'addition acide pharmaceutiquement acceptable de celle-ci.

**7.** Procédé selon la revendication 1 dans lequel le produit est la 1-{3-{1-[4-(2-méthoxyphényl)pipérazinyl]}-2-phénylpropionyl}pipéridine ou un sel d'addition acide pharmaceutiquement acceptable de celle-ci.

**8.** Procédé selon la revendication 1 dans lequel le produit est

- le 3-{1-[4-(2-méthoxyphényl)pipérazinyl]}-2-phénylpropanoate de propyle,
- le 3-{1-[4-(2-méthoxyphényl)pipérazinyl]}-2-phényl-N-phénylpropanamide,
- le 3-{1-[4-(2-méthoxyphényl)pipérazinyl]}-2-phénylpropanamide,
- le N-méthyl-3-{1-[4-(2-méthoxyphényl)pipérazinyl]}-2-phénylpropanamide,
- le N-éthyl-3-{1-[4-(2-méthoxyphényl)pipérazinyl]}-2-phénylpropanamide,
- le N-propyl-3-{1-[4-(2-méthoxyphényl)pipérazinyl]}-2-phénylpropanamide,
- le N-butyl-3-{1-[4-(2-méthoxyphényl)pipérazinyl]}-2-phénylpropanamide,
- le N-isobutyl-3-{1-[4-(2-méthoxyphényl)pipérazinyl)}-2-phénylpropanamide,

- le N-cyclopropylméthyl-3-{1-[4-(2-méthoxyphényl)-piperazinyl]}-2-phénylpropanamide,
- le N-néopentyl-3-{1-[4-(2-méthoxyphényl)pipérazinyl]}-2-phénylpropanamide,
- le N-hexyl-3-{1-[4-(2-méthoxyphényl)pipérazinyl]}-2-phénylpropanamide,
- le N-cyclohexylméthyl-3-{1-[4-(2-méthoxyphényl)-pipérazinyl]}-2-phénylpropanamide,
- la 8-{α-{1-[4-(2-méthoxyphényl)pipérazinyl]méthyl}-phénylacétamidoéthyl-8-azaspiro[4,5]déca-7,9-dione,
- le N,N-diméthyl-3-{1-[4-(2-méthoxyphényl)pipérazinyl]}-2-phénylpropanamide,
- le 3-{1-[4-(2-méthoxyphényl)pipérazinyl]}-2-phényl-N-(phénylméthyl)propanamide,
- le N-cyclohexyl-2-phényl-3-{1-[4-(2-pyrimidyl)-pipérazinyl]}propanamide,
- le 3,N-bis{1-[4-(2-méthoxyphényl)pipérazinyl]}-2-phénylpropanamide,
- le 3-{1-[4-(2-méthoxyphényl)pipérazinyl]}-2-phénylpropanoate de 2-méthylpropyle,
- le 3-{1-[4-(2-méthoxyphényl)pipérazinyl]}-2-phénylpropanoate d'éthyle,
- le 2-{1-[4-(2-méthoxyphényl)pipérazinyl]méthyl}-N-méthyl-3-phénylpropanamide,
- le 2-{1-[4-(2-méthoxyphényl)pipérazinyl]méthyl}-3-phénylpropanoate d'éthyle,
- le 3-{1-[4-(2-méthoxyphényl)pipérazinyl]}-N-(3,3-diméthyl)butyl)-2-phénylpropionamide,
- le N-cyclopropyl-3-{1-[4-(2-méthoxyphényl)pipérazinyl]}-2-phénylpropanamide,
- la 1-{3-{1-[4-(2-méthoxyphényl)pipérazinyl]}-2-phénylpropionyl}pyrrolidine,
- le N-cyclooctyl-3-{1-[4-(2-méthoxyphényl)pipérazinyl]}-2-phénylpropionamide,
- le N-cyclododécyl-3-{1-[4-(2-méthoxyphényl)pipérazinyl]}-2-phénylpropionamide,
- l'amide de l'acide (S)-N-[2-[1-[4-(2-méthoxyphényl)-pipérazinyl]]-1-phényléthyl]cyclohexanecarboxylique,
- l'O-[2-[1-[4-(2-méthoxyphényl)pipérazinyl]]-1-phényléthyl]-N-cyclohexylcarbamate,
- l'O-[2-[1-[4-(2-méthoxyphényl)pipérazinyl]]-1-phényléthyl]-N-phénylcarbamate,
- le 2-{1-[4-(2-méthoxyphényl)pipérazinyl]}-1-phényléthylcyclohexanecarboxylate,
- le (S)-N-[2-[1-[4-(2-méthoxyphényl)pipérazinyl]]-1-phényléthyl]carbamate d'éthyle,
- le 3-[1-[4-(2-méthoxyphényl)pipérazinyl]]-2-phénylpropionate de méthyle,
- le N-(1-éthylpropyl)-3-[1-[4-(2-méthoxyphényl)pipérazinyl]]-2-phénylpropionamide,
- la 4-[3-[1-[4-(2-méthoxyphényl)pipérazinyl]]-2-phénylpropionyl]morpholine,
- la 1-{3-{1-[4-(2-méthoxyphényl)pipérazinyl]}-2-phénylpropionyl}azétidine,
- l'O-{2-[1-[4-(2-méthoxyphényl)pipérazinyl]]-1-phényléthyl}-N-(3-chlorophényl)carbamate,
- le 1-éthyl-3-{2-[1-[4-(2-méthoxyphényl)pipérazinyl]]-1-phényléthyl}carbonate,
- l'ester 2-[4-(2-méthoxyphényl)pipérazin-1-yl]-1-phényléthylique de l'acide 2,3-dihydrobenzo[1,4]dioxin-2-yl-carboxylique,
- la (S)-8-{2-[1-[4-(2-méthoxyphényl)pipérazinyl]]-1-phényléthyl]-8-azaspiro[4,5]décan-7,9-dione,
- la 2,3,4,5,6,7-hexahydro-1-{2-[1-(4-(2-méthoxyphényl)-pipérazinyl)méthyl]-3-phénylpropanoyl}-lH-azépine,
- le N-cycloheptyl-2-{1-[4-(2-méthoxyphényl)pipérazinyl]-méthyl}-3-phénylpropanamide,
- ou le N-cyclopropyl-2-{1-[4-(2-méthoxyphényl)-pipérazinyl]méthyl}-3-phénylpropanamide

ou un sel d'addition acide pharmaceutiquement acceptable de ceux-ci.

**9.** Procédé de préparation d'une composition pharmaceutique comprenant la mise en association d'un composé de formule (I) comme défini dans la revendication 1 ou d'un sel d'addition acide pharmaceutiquement acceptable de celui-ci avec un support pharmaceutiquement acceptable.

**10.** Procédé selon la revendication 9, dans lequel le composé de formule (I) ou son sel est préparé par le procédé selon l'une quelconque des revendications 1 à 8.

**11.** Procédé de préparation d'un acide de formule

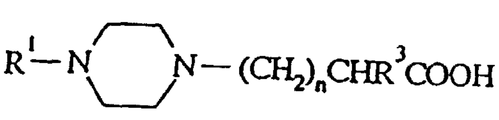

(où n, $R^1$ et $R^3$ sont comme définis dans la revendication 1) qui comprend

(a) la réaction d'un acide de formule

$CH_2{=}CR^3COOH$ (VIII)

et d'une pipérazine de formule

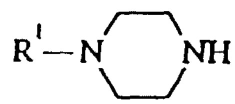

(IV)

au moyen d'une réaction de Michael, ou
(b) l'hydrolyse d'un nitrile de formule

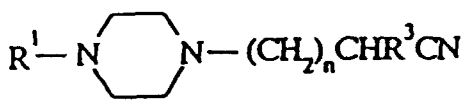

(IX)

**Revendications pour l'Etat contractant suivant : GR**

**1.** Procédé de préparation d'un composé de formule générale (I)

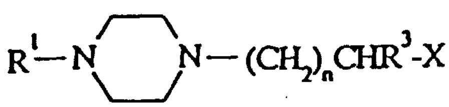

(I)

ou d'un sel d'addition acide pharmaceutiquement acceptable de celui-ci dans lequel

n est un des nombres entiers 1 ou 2,
$R^1$ est Ar ou Hét,
$R^3$ est Ar ou un radical Ar-alkyle($C_{1-6}$),
X est $-OCOR^{10}$, $-CO_2R^6$, $-CONR^5R^9$, $-OCO_2R^6$, $-NR^4COR^6$, $-OCONHR^{11}$, $-NHCO_2R^6$, $-NR^4CONHR^6$, $-CONHNHR^6$, $-CONHOR^6$,

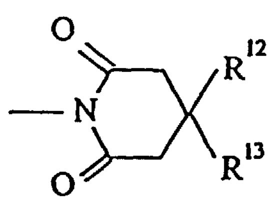

ou

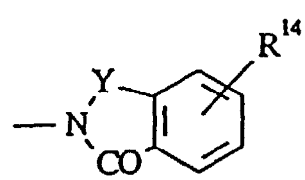

$R^4$ et $R^5$ sont chacun hydrogène ou alkyle en $C_{1-6}$,
$R^6$ est $-CHR^7R^8$, cycloalkyle de 3 à 12 atomes de carbone, ou Ar-alkyle($C_{1-6}$) (où $R^7$ et $R^8$ sont chacun hydrogène ou alkyle en $C_{1-6}$)
$R^9$ est hydrogène, un radical alkyle de 1 à 8 atomes de carbone autre qu'un radical d'alkyle tertiaire, cycloalkyle de 3 à 12 atomes de carbone, cycloalkyl-alkyle($C_{1-6}$), Ar-alkyle($C_{1-6}$) ou 8-azaspiro[4,5]déca-7,9-dione-8-yl-alkyle($C_{1-6}$) [avec la condition que quand $R^9$ est hydrogène, alkyle ou Ar-alkyle($C_{1-6}$), $R^5$ est autre qu'un radical

alkyle tertiaire], ou $R^5$ et $R^9$ conjointement avec l'atome d'azote auquel ils sont attachés représentent un cycle azétidino, pyrrolidino, pipéridino, hexahydroazépino, morpholino ou pipérazino qui peut être facultativement substitué par alkyle en $C_{1-6}$, Ar ou Ar-alkyle($C_{1-6}$),
$R^{10}$ est cycloalkyle de 3 à 12 atomes de carbone, ou 2,3-dihydro[1,4]benzodioxinyle facultativement substitué par alkyle en $C_{1-6}$, alkoxy en $C_{1-6}$ ou halogène,
$R^{11}$ est cycloalkyle de 3 à 12 atomes de carbone, Ar ou Ar-alkyle($C_{1-6}$),
$R^{12}$ et $R^{13}$ sont chacun alkyle en $C_{1-6}$ ou conjointement avec l'atome de carbone auquel ils sont attachés tous deux représentent cycloalkyle en $C_{4-6}$,
$R^{14}$ représente hydrogène, halogène, alkyle en $C_{1-6}$ ou alkoxy en $C_{1-6}$, et
Y est CO ou $SO_2$

avec les conditions supplémentaires que

(a) quand $R^1$ est phényle facultativement substitué par $CH_3$, $OCH_3$, Cl ou $CF_3$, $R^3$ est phényle facultativement substitué par alkyle en $C_{1-6}$, méthoxy ou halogène, et X est $-CONH_2$, $-CON(CH_3)_2$ ou $-COOC_2H_5$ alors n est 1, et (b) quand $R^1$ est 2-pyrimidyle, $R^3$ est phényle facultativement substitué à une ou deux positions du cycle avec alkyle en $C_{1-6}$, halogène, trifluorométhyle, cyano, nitro, alkyl($C_{1-6}$)amino, dialkyl($C_{1-6}$)amino, alkyloxy en $C_{1-6}$ et X est

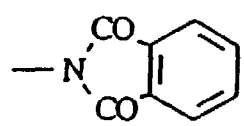

ou $-NR^4COR^6$ où $R^4$ est hydrogène ou alkyle en $C_{1-6}$ et $R^6$ est $-CHR^7R^8$ (où $R^7$ et $R^8$ sont chacun hydrogène ou alkyle en $C_{1-6}$ et $-CHR^7R^8$ contient un total de 1 à 6 atomes de carbone) alors n est 2;

et dans lequel le terme "Ar" désigne un radical aromatique ayant 6 à 12 atomes de carbone qui facultativement peut être substitué par un ou plusieurs substituants alkoxy en $C_{1-6}$, halogène, trifluorométhyle, nitro, carbalkoxy, carboxamido, cyano, amino, alkyl($C_{1-6}$)amino ou dialkyl($C_{1-6}$)amino et le terme "Hét" désigne un radical aromatique contenant un ou plusieurs atomes d'azote comme hétéroatomes et qui peut être substitué par un ou plusieurs substituants alkyle en $C_{1-6}$, alkoxy en $C_{1-6}$, halogène, trifluorométhyle, amino, alkyl($C_{1-6}$) amino ou dialkyl($C_{1-6}$) amino;
procédé qui comprend

(a) l'acylation d'une amine de formule

$$NHR^5R^9 \quad (II)$$

(où $R^5$ et $R^9$ sont comme définis ci-dessus) avec un acide de formule

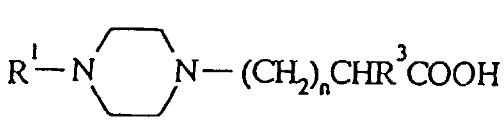

**(III)**

(où $R^1$ et $R^3$ sont comme définis ci-dessus) ou avec un dérivé d'acylation de celui-ci pour donner un composé de formule (I) où X représente $-CONR^5R^9$, ou
(b) l'acylation d'une alkylamine de pipérazine de formule

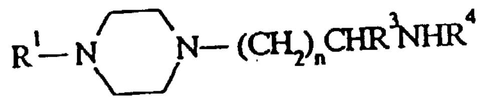

avec un acide de formule $R^6COOH$

ou avec un dérivé d'acylation de celui-ci pour donner un composé de formule (I) dans laquelle X est $-NR^4COR^6$, ou

(c) la réaction d'une amine de formule

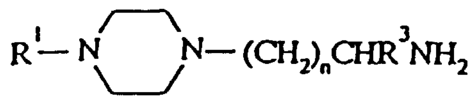

avec un anhydride de formule

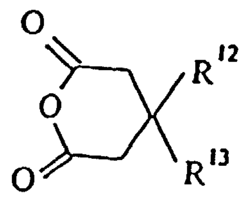

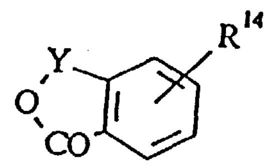

ou avec un acide de formule

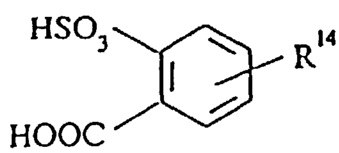

pour donner un composé de formule (I) dans lequel X est

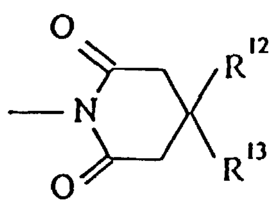

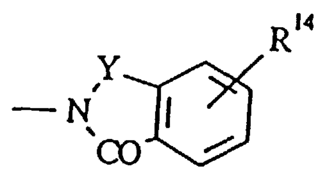

ou

ou (d) l'estérification d'un acide de formule (III), comme donné ci-dessus avec un alcool de formule $R^6OH$ pour donner un composé de formule (I) dans lequel X est $-CO_2R^6$, ou

(e) l'estérification d'un alcool de pipérazine de formule

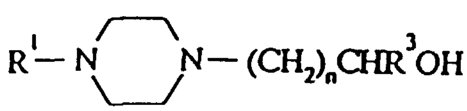

avec un acide de formule $R^{10}COOH$ pour donner un composé de formule (I) dans lequel X est $-OCOR^{10}$, ou

(f) la réaction d'un isocyanate avec un pipérazinyl-alcanol de formule

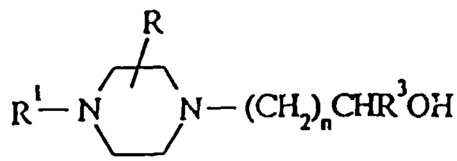

ou avec une pipérazinyl-alkylamine de formule

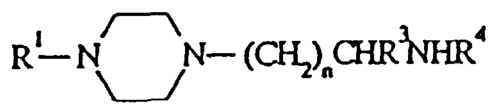

pour donner un composé de formule (I) dans lequel X est $-NR^4CONHR^6$ ou $-OCONHR^{11}$, ou
(g) la réaction d'un dérivé d'isocyanate de pipérazine de formule

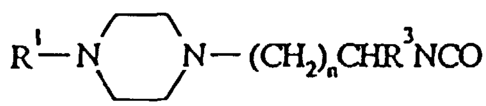

avec un alcool de formule $R^6OH$
pour donner un composé de formule (I) dans lequel X est $-NHCO_2R^6$, ou
(h) la réaction d'une amine de formule

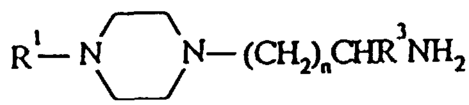

avec un composé de formule $R^6OCOHal$ (où Hal est halogène) pour donner un composé de formule (I) dans lequel X est $-NHCO_2R^6$, ou
(i) la réaction d'un acide de formule (III) comme donné ci-dessus, avec une hydroxylamine de formule $NH_2OR^6$ ou avec un hydrazide de formule $NH_2NHR^6$ pour donner un composé de formule (I) dans lequel X est respectivement $-CONHOR^6$ ou $-CONHNHR^6$, ou
(j) l'alkylation d'une pipérazine de formule

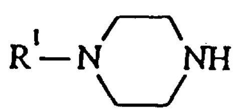

**(IV)**

avec un agent d'alkylation fournissant le radical $-(CH_2)_nCHR^3X$, ou
(k) la réaction d'un nitrile de formule (IX)

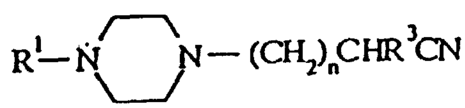

avec un alcool secondaire pour donner un composé de formule (I) dans lequel X est $-CONR^5R^9$ où $R^5$ est hydrogène et $R^9$ est un groupe alkyle secondaire (inférieur) ou en soumettant le nitrile à l'hydrolyse acide pour donner un composé de formule (I) dans lequel X est $-CONH_2$, ou

(l) la désulfuration d'un composé contenant du soufre de formule

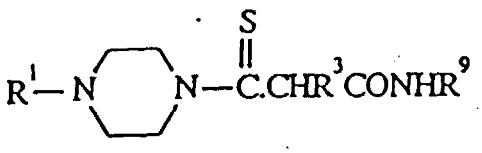

**(XI)**

pour donner un composé de formule (I) dans lequel X est $-CONHR^9$, ou
(m) la conversion d'une base libre de formule (I) en un sel pharmaceutiquement acceptable de celui-ci, ou
(n) la résolution d'un composé racémique de formule (I) pour donner un énantiomère optiquement actif.

**2.** Procédé selon la revendication 1, dans lequel, dans le produit, $R^1$ est un radical phényle facultativement substitué par un ou plusieurs substituants alkoxy en $C_{1-6}$, halogène, trifluorométhyle, nitro, carbalkoxy, carboxamido, cyano, amino, alkyl($C_{1-6}$)amino ou diaikyl($C_{1-6}$)amino.

**3.** Procédé selon la revendication 1 ou la revendication 2, dans lequel, dans le produit, $R^3$ est un radical phényle facultativement substitué par un ou plusieurs substituants alkoxy en $C_{1-6}$, halogène, trifluorométhyle, nitro, carbalkoxy, carboxamido, cyano, amino, alkyl($C_{1-6}$)amino ou dialkyl($C_{1-6}$)amino.

**4.** Procédé selon l'une quelconque des revendications 1 à 3, dans lequel, dans le produit, X représente $-CO_2R^6$ ou $-CONR^5R^9$ où $R^6$, $R^5$ et $R^9$ sont comme définis dans la revendication 1.

**5.** Procédé selon l'une quelconque des revendications 1 à 3 dans lequel, dans le produit, X est $-CONR^5R^9$ où $R^5$ et $R^9$ conjointement avec l'atome d'azote auquel ils sont liés représentent un cycle azétidino, pyrrolidino, pipéridino, hexahydroazépino, morpholino ou pipérazino.

**6.** Procédé selon la revendication 1 dans lequel le produit est la 1-{3-{1-[4-(2-méthoxyphényl)pipérazinyl]}-2-phényl-propionyl}pyrrolidine ou un sel d'addition acide pharmaceutiquement acceptable de celle-ci.

**7.** Procédé selon la revendication 1 dans lequel le produit est la 1-{3-{1-[4-(2-méthoxyphényl)pipérazinyl]}-2-phényl-propionyl}pipéridine ou un sel d'addition acide pharmaceutiquement acceptable de celle-ci.

**8.** Procédé selon la revendication 1 dans lequel le produit est

- le 3-{1-[4-(2-méthoxyphényl)pipérazinyl]}-2-phénylpropanoate de propyle,
- le 3-{1-[4-(2-méthoxyphényl)pipérazinyl]}-2-phényl-N-phénylpropanamide,
- le 3-{1-[4-(2-méthoxyphényl)pipérazinyl]}-2-phénylpropanamide,
- le N-méthyl-3-{1-[4-(2-méthoxyphényl)pipérazinyl]}-2-phénylpropanamide,
- le N-éthyl-3-{1-[4-(2-méthoxyphényl)pipérazinyl]}-2-phénylpropanamide,
- le N-propyl-3-{1-[4-(2-méthoxyphényl)pipérazinyl]}-2-phénylpropanamide,
- le N-butyl-3-{1-[4-(2-méthoxyphényl)pipérazinyl]}-2-phénylpropanamide,
- le N-isobutyl-3-{1-[4-(2-méthoxyphényl)pipérazinyl]}-2-phénylpropanamide,
- le N-cyclopropylméthyl-3-{1-[4-(2-méthoxyphényl)-pipérazinyl]}-2-phénylpropanamide,
- le N-néopentyl-3-{1-[4-(2-méthoxyphényl)pipérazinyl]}-2-phénylpropanamide,
- le N-hexyl-3-{1-[4-(2-méthoxyphényl)pipérazinyl]}-2-phénylpropanamide,
- le N-cyclohexylméthyl-3-{1-[4-(2-méthoxyphényl)-pipérazinyl]}-2-phénylpropanamide,
- la 8-{α-{1-[4-(2-méthoxyphényl)pipérazinyl]méthyl}-phénylacétamidoéthyl-8-azaspiso[4,5]déca-7,9-dione,
- le N,N-diméthyl-3-{1-[4-(2-méthoxyphényl)pipérazinyl]}-2-phénylpropanamide,
- le 3-{1-[4-(2-méthoxyphényl)pipérazinyl)}-2-phényl-N-(phénylméthyl)propanamide,
- le N-cyclohexyl-2-phényl-3-{1-[4-(2-pyrimidyl)-pipérazinyl]}propanamide,
- le 3,N-bis{1-(4-(2-méthoxyphényl)pipérazinyl]}-2-phénylpropanamide,
- le 3-{1-[4-(2-méthoxyphényl)pipérazinyl]}-2-phénylpropanoate de 2-méthylpropyle,
- le 3-{1-[4-(2-méthoxyphényl)pipérazinyl]}-2-phénylpropanoate d'éthyle,
- le 2-{1-[4-(2-méthoxyphényl)pipérazinyl]méthyl}-N-méthyl-3-phénylpropanamide,
- le 2-{1-[4-(2-méthoxyphényl)pipérazinyl]méthyl}-3-phénylpropanoate d'éthyle,

- le 3-{1-[4-(2-méthoxyphényl)pipérazinyl]}-N-(3,3-diméthyl)butyl)-2-phénylpropionamide,
- le N-cyclopropyl-3-{1-[4-(2-méthoxyphényl)pipérazinyl]}-2-phénylpropanamide,
- la 1-{3-{1-[4-(2-méthoxyphényl)pipérazinyl]}-2-phénylpropionyl}pyrrolidine,
- le N-cyclooctyl-3-{1-[4-(2-méthoxyphényl)pipérazinyl]}-2-phénylpropionamide,
- le N-cyclododécyl-3-{1-[4-(2-méthoxyphényl)pipérazinyl]}-2-phénylpropionamide,
- l'amide de l'acide (S)-N-[2-[1-[4-(2-méthoxyphényl)-pipérazinyl]]-1-phényléthyl]cyclohexanecarboxylique,
- l'O-[2-[1-[4-(2-méthoxyphényl)pipérazinyl]]-1-phényléthyl]-N-cyclohexylcarbamate,
- l'O-[2-[1-[4-(2-méthoxyphényl)pipérazinyl]]-1-phényléthyl]-N-phénylcarbamate,
- le 2-{1-[4-(2-méthoxyphényl)pipérazinyl]}-1-phényléthylcyclohexanecarboxylate,
- le (S)-N-[2-[1-[4-(2-méthoxyphényl)pipérazinyl]]-1-phényléthyl]carbamate d'éthyle,
- le 3-[1-[4-(2-méthoxyphényl)pipérazinyl]]-2-phénylpropionate de méthyle,
- le N-(1-éthylpropyl)-3-[1-[4-(2-méthoxyphényl)pipérazinyl]]-2-phénylpropionamide,
- la 4-[3-[1-[4-(2-méthoxyphényl)pipérazinyl]]-2-phénylpropionyl]morpholine,
- la 1-{3-{1-[4-(2-méthoxyphényl)pipérazinyl]}-2-phénylpropionyl}azétidine,
- l'O-{2-[1-[4-(2-méthoxyphényl)pipérazinyl]]-1-phényléthyl}-N-(3-chlorophényl)carbamate,
- le 1-éthyl-3-{2-[1-[4-(2-méthoxyphénol)pipérazinyl]]-1-phényléthyl}carbonate,
- l'ester 2-[4-(2-méthoxyphényl)pipérazin-l-yl]-1-phényléthylique de l'acide 2,3-dihydrobenzo[1,4]dioxin-2-yl-carboxylique,
- la (S)-8-{2-[1-[4-(2-méthoxyphényl)pipérazinyl]]-1-phényléthyl]-8-azaspiro[4,5]décan-7,9-dione,
- la 2,3,4,5,6,7-hexahydro-1-{2-[1-(4-(2-méthoxyphényl)-pipérazinyl)méthyl]-3-phénylpropanoyl}-lH-azépine,
- le N-cycloheptyl-2-{1-[4-(2-méthoxyphényl)pipérazinyl]-méthyl}-3-phénylpropanamide,
- ou le N-cyclopropyl-2-{1-[4-(2-méthoxyphényl)-pipérazinyl]méthyl}-3-phénylpropanamide

ou un sel d'addition acide pharmaceutiquement acceptable de ceux-ci.

**9.** Procédé de préparation d'une composition pharmaceutique comprenant la mise en association d'un composé de formule (I) comme défini dans la revendication 1 ou d'un sel d'addition acide pharmaceutiquement acceptable de celui-ci avec un support pharmaceutiquement acceptable.

**10.** Procédé selon la revendication 9, dans lequel le composé de formule (I) ou son sel est préparé par le procédé selon l'une quelconque des revendications 1 à 8.

**11.** Procédé de préparation d'un acide de formule

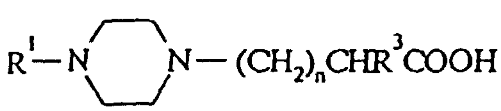

(où n, $R^1$ et $R^3$ sont comme définis dans la revendication 1) qui comprend

(a) la réaction d'un acide de formule

$$CH_2=CR^3COOH \qquad (VIII)$$

et d'une pipérazine de formule

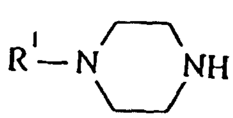

(IV)

au moyen d'une réaction de Michael, ou
(b) l'hydrolyse d'un nitrile de formule

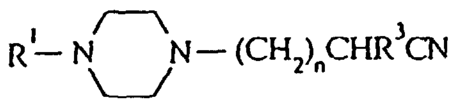

(IX)

**12.** Acide de formule

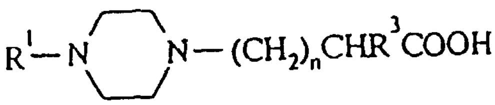

(où n, $R^1$ et $R^3$ sont comme définis dans la revendication 1).